# EUROPEAN PATENT APPLICATION

(11) **EP 1 006 190 A1**
(43) Date of publication of application: **07.06.2000**
(21) Application number: 98204018.0
(22) Date of filing: 02.12.1998
(51) Int. Cl.: C12N 15/53, C12N 15/54, C12N 15/60, C12N 15/11, C12N 15/82, C12N 9/10, C12N 9/88, C12N 9/04, C12P 7/62, C12Q 1/68, C07K 16/40, A01H 5/00, G01N 33/50

(54) **Fruit flavour related genes and use thereof**

(71) Applicant: CENTRUM VOOR PLANTENVEREDELINGS- EN REPRODUKTIEONDERZOEK, 6708 PB Wageningen (NL)
(72) Inventor: Verhoeven, Harrie Adrianus, 5694 LB Breugel (NL); van Tunen, Arjen Johannes, 6708 NA Wageningen (NL); Aharoni, Asaph, Tel-Aviv (IL); Lücker, Joost, 5991 DA Baarlo (NL); O'Connell, Ann Patricia, 2633 JH Nootdorp (NL)
(74) Representative: De Hoop, Eric

(57) **Abstract**

DNA sequences encoding enzymes involved in the biosynthetic pathway for aliphatic and/or aromatic ester production in fruit are disclosed. The enzymes have alcohol acyl transferase, alcohol dehydrogenase, pyruvate decarboxylase, thiolase and aminotransferase activities, respectively. The polypeptides having said activities are disclosed. Expression vectors comprising the DNA sequences may be used for regulating ester formation in fruit. Genetically modified plants and microorganisms may be used for producing esters.

## Description

The invention disclosed herein relates to strawberry (*fragaria ananassa*) and lemon (*citrus limon*) fruit derived genes and enzymes specifically involved in the formation of aliphatic and/ or aromatic esters and other aroma and flavour compounds in fruits. More specifically it relates to a process for improving natural volatile fruit flavours by the control of one or more than one gene implicated in that process.

### Background of the invention

Like most natural products the chemical composition of strawberry aroma and flavour is quite complex [Zabetakis and Holden, (1997); J. Sci. Food. Agric 74: 421-434]. Over 300 compounds have been identified which may contribute to flavour/aroma. Qualitatively, the major components of strawberry flavour and aroma may be grouped into several chemical classes including acids, aldehydes, ketones, alcohols, esters, and lactones. Other groups include sulphur compounds, acetals, furans, phenols and even traces of epoxides, and hydrocarbons. Compounds produced by these groups, whilst often present at low levels, may have a significant impact on the overall flavour of strawberry.

In general, fruit flavour compounds develop during ripening when the metabolism of the fruit changes to catabolism. Esters are proposed to be qualitatively and quantitatively the most important class of volatiles produced and are a key component of strawberry flavour. Seven volatile esters, ethyl hexanoate, methyl hexanoate, ethyl butanoate, methyl butanoate, hexyl acetate, ethyl propionate and 2-hexenyl-acetate were reported to contribute largely to the aroma associated with the fruit. However, more then a 100 types of esters have been reported during the years to be identified in strawberry volatile analysis [Zabetakis and Holden, (1997); J. Sci. Food. Agric 74: 421-434].

In lemon, the acetate esters derived from cyclic terpene alcohols are used frequently as flavour, fragrance, scent and aroma components. The lower fatty acid esters of acyclic terpene alcohols (geraniol, linalool, and citronellol) are the acetates (linalyl acetate, geranyl acetate) which are the main flavour components of lemon peel oils. These are used for obtaining citrus notes [Bauer, K and Garbe, D, (1985). Common fragrance and flavour materials. Preparation, properties and uses. VCH Publishers, Florida, USA]. Formates, propionates, butyrates occur less frequently. In addition to the cyclic and acyclic terpene esters, lemon is a rich source for the ester methyl anthranillate, which is a methyl ester of o-aminobenzoic acid. This compound is known to be a major constituent of *citrus limon* fruit and flowers and can also be found in the leaves.

Primary metabolites which include sugars, fatty acids, nucleotides and amino acids have been proposed to serve as precursors for the production of secondary metabolites such as volatile compounds. Sugars in the fruit in addition to acting as a carbon source, act as precursors for the flavour compound furanones [Zabetakis and Holden, (1996); Plant Cell Tissue and Organ Culture 45:25-29]. Amino acid metabolism leads to the formation of various aldehydes, acids, carbonyls, aliphatic and branched-chain alcohols, some of which serve as aroma and flavour components. The changes in amino acid content during maturation of fruits in relation to fruit flavour formation, have been studied in banana, tomato and strawberry. The amino acids valine, leucine and isoleucine have been proposed to serve as flavour precursors in banana fruit, and are metabolised to branched chain alcohols, 2-propanol, isoamyl alcohol, and 2-methylbutanol, respectively [Drawert and Berger, (1981); Bioflavour '81; Schreier, P., Ed.; de Gruyter: Berlin]. The conversion of alanine, leucine and valine to flavour compounds has also been demonstrated in tomato [Yu *et al*., (1968); Phytochemistry 6: 1457-1465]. In strawberry, alanine was proposed to be the main free amino acid metabolised to flavour compounds. This was based mainly on the dramatic decrease in its content just before the formation of volatile aroma and flavour compounds commenced in the fruit [Perez *et al*., (1992); J.Agric. Food Chem. 40: 2232-2235]. In addition feeding alanine to strawberry cultures resulted in the formation of several esters such as methyl and ethyl hexanoate which are important constituents of strawberry volatiles [Drawert and Berger, (1981); Bioflavour '81; Schreier, P., Ed.; de Gruyter: Berlin]. Like sugars and amino acids the fatty acids in strawberry play an important role in ester formation. In mango fruit there have been reports that fatty acids are actively metabolised during ripening. Changes in fatty acids and triglycerides have been associated with changes in aroma and flavour during mango ripening [Gholop AS and Bandyophaday C, J Am. Oil Chem. Soc.,52: 514-516, 1975]. Therefore fatty acid related enzymes may have a role in the production of flavour volatiles. Thiolase is the last enzyme in the β-oxidation of fatty acids. It catalyses the thiolytic cleavage involving a molecule of CoA. The products of this reaction are acetyl-CoA and acyl-CoA derivatives containing two carbon atoms less than the original acyl-CoA molecule that underwent oxidation. The acyl-CoA formed in the cleavage reaction may be utilised at the final stage of the biosynthetic pathway for ester formation in fruit. Therefore the profile of fatty acid and amino acid precursors found in each fruit, along with the specificity of the enzymes in the biosynthetic pathway/process leading to ester formation could have a key role in determining the types of esters formed.

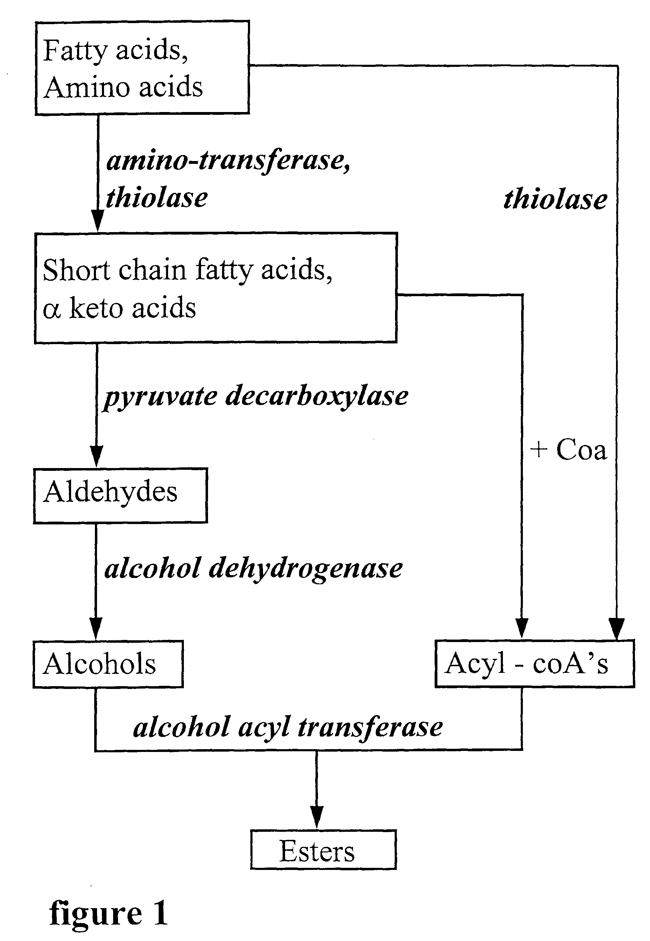

A proposed biosynthetic pathway for volatile ester formation in fruits is illustrated in Figure 1. In fruits the transamination and oxidative decarboxylation of amino acids provides the precursors for volatile aroma compounds such as aldehydes, acids, alcohols, esters and thiols. The transamination reaction is catalysed by aminotransferases. Aminotransferases or transaminases are enzymes promoting the first step in the catabolism of L-amino acids by removing the α-amino groups. As a result of this transamination reaction, the α-amino group is transferred to the α-carbon atom of α-ketoglutarate, leaving behind the α-keto acid analogue of the amino acid. Aminotransferases have been studied in various micro-organisms and some of them have been purified [Gelfand *et al*, (1997); J. Bacteriol. 130:429-440, Lee et al., (1985); J. Gen. Microbiol. 131: 459-467]. No strawberry fruit specific aminotransferase to date has been cloned or characterised.

Ketoacids produced by transamination can be enzymatically degraded to the corresponding aldehydes or carboxylic acids. The enzyme which can catalyse this reaction is pyruvate decarboxylase. An EST encoding part of pyruvate decarboxylase, the first to be characterised from a strawberry fruit has been mentioned in patent application WO 97/27295. The pyruvate decarboxylase enzyme provides precursors for the biosynthesis of volatile flavour compounds. Alcohol dehydrogenase enzymes have been implicated in the interconversion of the aldehyde and alcohol forms of flavour volatiles. The mechanism of ester formation has been shown to be a coenzyme A dependent reaction. Esterification is a result of transacylation from acyl-coenzyme A to an alcohol. The enzyme is therefore termed as alcohol acyl transferase (AAT) and plays a major role in the biosynthesis of volatile esters.

Ester formation by micro-organisms has been studied in most detail and has been cited in a number of publications [Harada et al., (1985); Plant Cell Physiol. 26(6): 1067-1074], Fujii *et al.,* (1996); Yeast 12:593-598, Fujii *et al.*, (1994); Applied and Enviro. Microbiology 8:2786-2792 ]. Alcohol acyl transferases (AAT's) have been identified from both fungi [Yoshihide *et al*., (1978); AGRIC. Biol. Chem. 42(2):269-274], and yeast [Fujii *et al*., (1994); Applied and Enviro. Microbiology 8:2786-2792 ] and have been the subject of patent applications (EP 0 574 941 A2). The AAT from the fungi *Neurospora* sp. which was purified to homogeneity acts on various acyl Coenzyme A containing more than a four carbon linear chain but not on acetyl coenzyme A as described for the yeast enzyme [Yamauchi *et al*., (1989); Agric. Biol. Chem. 53(6) 1551-1556]. In *Cladosporium cladosporioides* No. 9, AAT was partially purified and was described to form acetate esters like the yeast enzyme [Yamakawa *et al.*, (1978); Agric. Biol. Chem. 42(2):269-274]. So it is clear that the type of esters formed is dependant on the substrate specificity of the ester forming enzyme to alcohols, and the availability of acyl-CoAs. For example the yeast *Saccharomyces cerevisiae* (sake yeast) and brewer's yeast (bottom fermenting yeast) AAT's show high affinity to acetyl-CoA and the alcohols ethanol and isoamyl alcohol, giving rise to the formation of acetate esters. These esters play an important role in determining flavour characteristics of beer.

In plants ester formation has been studied both in fruit and flowers. Melon, banana and strawberry AAT's have been investigated using crude fruit extracts. The formation of esters from aldehydes that were incubated with whole strawberry fruit has been reported [Yamashita, *et al.,* (1989); Agr. Biol. Chem. 39(12) 2303-2307]. Analysis of the substrate specificity of the enzymes from the various sources, revealed differences in their affinity to acyl-CoAs and alcohols [Ueda *et al*., (1992); Nippon Shokukin Kogyo Gakkaishi 39(2): 183-187, Perez *et al*., (1996); J. AgricFood Chem. 44: 3286-3290, Harada et al., (1985); Plant Cell Physiol. 26(6): 1067-1074]. Maximum activity for the strawberry (AAT) was obtained using acetyl CoA and hexyl alcohol as substrates, and acetyl-CoA and butyl alcohol is the preferred substrate for the banana enzyme. A clear correlation could be observed between substrate preference and volatile esters present in both fruits. The purification method and some properties of the strawberry AATase enzyme have been reported. However, to date no sequence of the peptide or of the gene has been disclosed. In *Clarkia breweri* the ester benzylacetate is an important constituent of flower scent. The purification of the protein and the gene AAT encoding it has been reported. The flower AAT enzyme was not reported to have affinity to aliphatic alcohols. The enzyme has high affinity to the aromatic alcohols such as benzyl alcohol and cinnamyl alcohol [Dudareva *et al.*, (1998); *The Plant Journal* 14(3) 297-304]. Genes coding for flower aromatic acyl transferases, that acylate plant pigments, causing changes in colour tone have been the subject of a recent patent application [EP 0810287]. Recently, an aromatic amino transferase enzyme has been purified from lactic acid bacteria and was shown to initiate the conversion of amino acids to cheese flavour compounds [Yvon *et al*., (1997); Applied and Environmental Microbiology 414-419].

Although volatile esters are qualitatively and quantitatively one of the most important classes of volatile compounds in fruit flavour and aroma, there are very few reports concerning the biochemical aspects of ester formation in fruits. An understanding of the precursors and characterisation of enzymes involved in the pathways leading to the formation of flavours in fruit is essential for the production of natural flavours. Plant derived flavour components alone represent a world-wide market of 1.5 billion dollars. Presently, the main ways to produce plant flavour compounds is by the synthetic route. Synthetic organic chemicals constitute more than 80-90% (by weight and value) of the raw materials used in flavour and fragrance formulations. Problems often exist concerning production. Extraction from intact plants and conventional fermentation are currently providing alternative routes for the commercial production of flavour and aroma chemicals. However, the demand for natural flavours by the consumer has been steadily increasing, and often demand outstrips supply. In many cases sought after flavour compounds preclude isolation. Unlike other fruit flavours, no single chemical or class of chemicals in particular are associated with strawberry flavour. To date no strawberry fruit specific aminotransferase, pyruvate decarboxylase, thiolase, alcohol dehydrogenase, or acyl-transferase enzymes at the route of this invention, involved directly or in providing precursors for ester formation, hence flavour compounds, has been isolated and characterized. The genes and their peptides at the route of this invention are involved in the biosynthetic pathway for aliphatic and/or aromatic ester production in fruit and can therefore provide a novel method for the *in-vivo* and *in-vitro* biotechnology production of bio-flavours, natural flavour chemicals by recombinant means.

### Summary of invention

The object of the present invention is to disclose the genes/peptides involved in the process of volatile ester production, particularly aliphatic esters although not exclusively so, hence fruit flavour and aroma. This is the first time to our knowledge, that the genes/peptides sequences from an entire pathway to the production of volatile esters in fruit, in particularly strawberry, have been disclosed. The nucleotide and polypeptide sequences described in this application can serve as a tool for a vast number of applications related to ester formation and the biotechnological engineering of natural and artificial fruit flavours for the food industry. The invention is based on the identification of genes which encode proteins central to the pathway leading to volatile ester formation in fruit. DNA sequences which encode these proteins have been cloned and characterised. The nucleic acid/peptide sequences may be used in expression systems, for industrial application and/or to modify plants with the goal to produce natural and/or synthetic flavours.

### Definitions of Terms

'Nucleic acid' sequence as used herein refers to an oligonucleotide, nucleotide or polynucleotide, and fragments or portions thereof, and to DNA or RNA of genomic or synthetic origin which may be single- or double-stranded, and represents the sense or antisense strand. Similarly, 'amino acid sequence' as used herein refers to peptide or protein sequence.

The term 'agonist', as used herein, refers to a molecule which, when bound to a polypeptide causes a change in the polypeptide which modifies the activity of the polypeptide. Agonist may include proteins, nucleic acids, carbohydrates, or any other molecule which binds to the polypeptide.

The term 'antagonist' or 'inhibitor' as used herein, refer to a molecule which when bound to a polypeptide, blocks or modulates the biological or immunological activity of the polypeptide. Antagonists or inhibitors may include proteins, nucleic acids, carbohydrates, or any other molecules which bind to the polypeptide.

'Stringency' typically occurs in the range from about Tm-5°C (5°C below the Tm of the probe) to about 20°C to 25°C below Tm. As will be understood by those skilled in the art, a stringent hybridisation can be used to identify or detect identical polynucleotide sequences or to identify or detect similar or related polynucleotide sequences.

The term 'hybridisation' as used herein shall include 'any process' by which a strand of nucleic acid joins with a complementary strand through base pairing' (Coombs J, (1994) Directory of biotechnology, Stockton press, New York NY).

A 'deletion' is defined as a change in either nucleotide or amino acid sequence in which one or more nucleotides or amino acid residues, respectively, are absent.

An 'insertion' or 'addition' is that change in nucleotide or amino acid sequence which has resulted in the addition of one or more nucleotides or amino acid residues, respectively, as compared to the naturally occurring polypeptide(s).

A 'substitution' results from the replacement of one or more nucleotides or amino acids by different nucleotides or amino acids, respectively.

As used herein, the term 'substantially purified' refers to molecules, either nucleic or amino acid sequences, that are removed from their natural environment, isolated or separated, and that are at least 60% free, preferably 75% free, and most preferably 90% free from other components with which they are naturally associated. The definition 'substantially homologous' means that a particular subject sequence, for example a mutant sequence, varies from the reference sequence by one or more substitutions, deletions, or additions, the net effect of which does not result in an adverse functional dissimilarity between the reference and the subject sequence.

A 'variant' of a polypeptide as outlined in the first and second aspects of this invention is defined as an amino acid sequence that is different by one or more amino acid 'substitutions'. A variant may have 'conservative' changes, wherein a substituted amino acid has similar structural or chemical properties eg replacement of leucine with isoleucine. More rarely a variant may have 'non-conservative' changes, eg replacement of a glycine with a tryptophan. Similar minor variations may also include amino acid deletions or insertions, or both. Guidance in determining which and how many amino acid residues may be substituted, inserted or deleted, without abolishing biological or immunological activity may be found using computer programmes well known in the art, for example, DNAStar software.

The term 'biologically active' refers to a polypeptide as outlined in the first and second aspects of the invention, having structural, regulatory, or biochemical function of their naturally occurring counterparts. Likewise, 'immunologically active' defines the capability of the natural, recombinant or synthetic polypeptide, as outlined in the first and second aspects of the invention, or any oligopeptide thereof, to induce a specific immune response in an appropriate animal or cells and to bind with specific antibodies.

The term 'natural products' are those products that are obtained directly from plants and sometimes from animal sources by physical procedures. 'Nature identical compounds' are produced synthetically but are chemically identical to their 'natural counterparts'. Artificial flavour substances are compounds that have not been identified in plant or animal products for human consumption. 'Nature-identical' aroma substances are with few exceptions the only synthetic compounds used in flavours in addition to 'natural products'.

The definition 'host cell' refers to a cell in which an alien process is executed by bio-interaction, irrespective of the cell belongs to a unicellular, multicellular, a differentiated organism or to an artificial cell, cell culture or protoplast. The definition 'host cell' in the context of this invention is to encompass the definition 'plant cell'.

'Plant cell' by definition is meant by any self-propagating cell bounded by a semi permeable membrane and containing one or more plastids. Such a cell requires a cell wall if further propagation is required. 'Plant cell', as used herein, includes without limitation, seeds, suspension cultures, embryos, meristematic regions, callous tissues, protoplasts, leaves, roots, shoots, gametophytes, sporophytes, pollen and microspores.

With the definition 'transformed cell' or 'transgenic cell' is meant a cell (or ancestor of said cell) into which by means of recombinant DNA techniques, DNA encoding the target polypeptide can be introduced.

The definition 'micro-organisms' refers to microscopic organisms, such as Archaea, Bacteria, Cyanobacteria, Microalgae, Fungi, Yeast, Viruses, Protozoa, Rotifers, Nematodes, Micro-Crustaceans, Micro-Molluscs, Micro-Shellfish, Micro-insects etc.

The definition 'plant(s)' refers to eukaryotic, autotrophic organisms. They are characterised by direct usage of solar energy for their primary metabolism, their permanent cell wall and in case of multicellular individuals their open unlimited growth. In case of heterotrophic plants, the organisms are in an evolutionary context essentially derived from autotrophic plants in their structure and metabolism.

'Dicotyledons' (and all scientific equivalents referring to the same group of plants) form one of the two divisions of the flowering plants or angiospermae in which the embryo has two or more free or fused cotyledons.

'Monocotyledons' (and all scientific equivalents referring to the same group of plants) form one of the two divisions of the flowering plants or angiospermae in which the embryo has one cotyledon.

'Angiospermae' or flowering plants are seed plants characterised by flowers as specialised organs of plant reproduction and by carpels covering the ovaries. Gymnospermae are seed plants characterised by strobili as specialised organs for plant repoduction and by naked sporophylls bearing the male or female reproductive organs. 'Ornamental' plants are plants that are primarily in cultivation for their habitus, special shape, (flower, foliage or otherwise) colour or other characteristics which contribute to human well being indoor as cut flowers or pot plants or outdoors in the man made landscape. 'Vegetables' are plants that are purposely bred or selected for human consumption of foliage, tubers, stems, fruits, flowers of parts of them and that need an intensive cultivation regime. 'Arable crops' are purposely bred or selected for human objectivity's (ranging from direct or indirect consumption, feed or industrial applications such as fibres).

The definition 'process' is the development of, a method(ology) concerning, the progress of a series of activities in a certain context. 'Biological' process is a process based on metabolic activity in organisms, or essentially derived from that by biochemical, biophysical, physiological, ecological or genetic means. 'Industrial' process is an economically feasible process with high added value, aiming at bulk production.

The definition 'homology' refers to the basic similarity of a particular structure in different organisms, usually as result from the descent from a common ancestor.

The definition 'promoter' is intended as a nucleotide sequence sufficient to direct transcription. Also included are those promoter elements which are sufficient to render tissue-specific gene expression; such elements may be located in the 5' or 3' regions of the native gene.

The definition 'operably linked' is meant that a gene and a regulatory sequence(s) are connected in such a way as to permit gene expression when the appropriate molecules (for example, transcriptional activator proteins) are bound to the regulatory sequence(s).

The definition 'fruit' (botanically) is the ripened ovary of a plant and its contents. The definition 'fruit' (agronomically linguistically) is the ripened ovary and its contents together with any structure with which they are combined, as in case of strawberry or apple, the receptacle.

The definition 'climacteric' is pointing at the phase of increased respiration found at fruit ripening and at senescence. The definition 'non-climacteric' is pointing at no such phase being present.

The definition 'antisense' RNA is an RNA sequence which is complementary to a sequence of bases in the corresponding mRNA: complementary in the sense that each base (or majority of bases) in the antisense strand (read in the 5' to 3' sense) is capable of pairing with the corresponding base (G with C, A with U), in the mRNA sequence read in the 5' to 3' sense.

The definition 'sense' RNA is an RNA sequence which is substantially homologous to at least part of the corresponding mRNA sequence.

### Brief description of drawings

The present invention may be more fully understood by reference to the following description, when read together with the accompanying drawings:
**Figure 1.** The proposed biosynthetic pathway for the production of esters in fruits.
**Figure 2.** A GCMS chromatogram of a mature strawberry fruit of the cultivar *Elsanta*. The analysis was done using the headspace method. Esters are marked with dots.
**Figure 3**. The expression pattern of strawberry alcohol acyl transferase cDNA (SLE27, SEQ ID NO:1A) in various tissues of the cultivar *Elsanta* by Northern blot analysis. The Northern blot was hybridised with a full length cDNA fragment of SEQID:1A (SLE27).
**Figure 4.** The expression pattern of a lemon alcohol acyl transferase (CLF26, SEQ ID NO:2A) in various tissues of the lemon cultivar *Mayer* by Northern blot analysis. The Northern blot was hybridised with a full length cDNA fragment of SEQ ID 2A (CLP26).
**Figure 5.** Expression analysis in various tissues of the strawberry cultivar *Elsanta* by Northern blots analysis. The Northern blots where hybridised with full length cDNA fragments corresponding to (a) a thiolase SEQ ID 4A (SLG150) (b) a pyruvate decarboxylase SEQ ID 5A (SLH51) and (c) an alcohol dehydrogenase SEQ ID 6A (SLB39).
**Figure 6.** Strawberry genomic Southern analysis. 10 µg DNA was digested with restriction enzymes HindIII, EcoRI, XbaI and XhoI, separated on an agarose gel and blotted onto a nylon filter. The DNA was hybridised with a full length ³²P-labelled strawberry alcohol acyl transferase cDNA (SLE27) probe, corresponding to SEQ ID 1A and autoradiographed.
**Figure 7.** A scheme of the pRSET B (Invitrogen) vector used for the expression ofthe strawberry alcohol acyl transferase (SLE27, SEQ ID NO: 1A)) in *E.coli.*
**Figure 8.** Shows acetic acid butyl ester formation from acetyl-CoA and 1-butanol by (**A**) 25 µl of protein elute from *E. coli* expressing the strawberry alcohol acyl transferase (SLE27, SEQ ID NO:1A) (**B**) 25 µl of inactivated protein elute from *E. coli* expressing the strawberry alcohol acyl transferase (SLE27, SEQ ID NO:1A) (**C**) 25 µl of protein extract from *E. coli* expressing the empty vector cassette.
**Figure 9.** Western blot loaded with protein corresponding to four different treatments carried out on the pellet, supernatant and elute from a Ni-NTA column after expression of strawberry alcohol acyl transferase (SLE 27, SEQ ID NO:1A) in *E. coli.* As a control for the experiment the Green Fluorescent Protein (GFP) was partially purified in the same way and the elute from one treatment was loaded on the gel. The blot was hybridised with a commercial antibody (Clontech) raised against an epitope fused at the N- terminus of the recombinant protein.
**Figure 10.** Gene expression profiles of strawberry alcohol acyl transferase (SLE 27, SEQ ID NO: 1A)), alcohol dehydrogenase (SLF138, SEQ ID NO: 11A), pyruvate decarboxylase (SLH 51, SEQ ID NO: 5A), alcohol dehydrogenase (SLG16, SEQ ID NO:12A) and amino transferase (SLF96, SEQ ID NO: 3A). Expression ratios were monitered during fruit development and ripening in the red stage vs green fruit and red fruit v.s turning fruit.

### Detailed description of the invention

According to a first aspect the invention provides an insolated DNA sequence encoding
(a) a polypeptide having an amino acid sequence as shown in SEQ ID NO: 1B or SEQ ID NO: 2B, or
(b) a polypeptide having at least 30% homology with the amino acid sequence SEQ ID NO: 1B or at least 40% homology with the amino acid sequence SEQ ID NO: 2B, or
(c) a fragment of polypeptide (a) or (b),
   which polypeptide or fragment of polypeptide has alcohol acyl transferase activity and is involved in the biosynthetic pathway for aliphatic and /or aromatic ester production in fruit.

Preferably, the DNA sequence encodes a polypeptide having at least 50% homology, more preferably at least 70% homology, with any of the amino acid sequences SEQ ID NO: 1B or SEQ ID NO: 2B, or a fragment thereof.

With reference to the nucleic acid sequence the invention provides an isolated DNA sequence having a nucleic acid sequence
(a) as shown in SEQ ID NO: 1A or SEQ ID NO: 2A which encodes a polypeptide having alcohol acyl transferase activity and being involved in the biosynthetic pathway for aliphatic and/or aromatic ester production in fruit, or
(b) complementary to SEQ ID NO: 1A or SEQ ID NO: 2A, or
(c) which has at least 25% homology with any of the sequences of (a) or (b), or
(d) which is capable of hybridising under stringent conditions to any of the sequences (a)-(c).

Preferably the DNA sequence has a nucleic acid sequence
(c) which has at least 40% homology, more preferably at least 60% homology, with any of the sequences of (a) or (b), or
(d) which is capable of hybridising under stringent conditions to said sequence (c).

According to a second aspect the invention provides a purified and isolated polypeptide,
(a) having an amino acid sequence as shown in SEQ ID NO: 1B or SEQ ID NO: 2B, or
(b) having at least 30% homology with the amino acid sequence SEQ ID NO: 1B or at least 40% homology with the amino acid sequence SEQ ID NO: 2B, or
(c) being a fragment of polypeptide (a) or (b), which polypeptide or fragment of polypeptide has alcohol acyl transferase activity and is involved in the biosynthetic pathway for aliphatic and/or aromatic ester production in fruit.

Preferably, the polypeptide has at least 50% homology, more preferably at least 70% homology, with any of the amino acid sequences SEQ ID NO: 1B or SEQ ID NO: 2B, or a fragment thereof.

It may be advantageous to produce nucleotide sequences according to the first aspect of the invention as defined above or derivatives thereof possessing a substantially different codon usage. It is known by those skilled in the art that as a result of degeneracy of the genetic code, a multitude of gene sequences, some bearing minimal homology to the nucleotide sequences of any known and any naturally occurring genes may be produced. The invention contemplates each and every possible variation of the nucleotide sequences that could be made by selecting combinations based on possible codon choices. These combinations are made in accordance with the standard triplet genetic code as applied to the nucleotide sequence of naturally occurring gene sequences, and all such variations are to be considered as being specifically disclosed. In addition the nucleotide sequences of the invention may be used in molecular biology techniques that have not been developed, providing the new techniques rely on properties of nucleotide sequences that are currently known, including but are not limited to such properties such as the triplet genetic code and specific base pair interactions.

Altered nucleic acid sequences of this invention include deletions, insertions, substitutions of different nucleotides resulting in the polynucleotides that encode the same or are functionally equivalent. Deliberate amino acid substitution may be made on the basis of similarity in polarity, charge, solubility, hydrophobicity, and/or the amphipathetic nature of the residues as long as the biological activity of the polypeptide is retained. Included in the scope of the present invention are alleles of the polypeptides according to the second aspect of the invention as defined above. As used herein, an 'allele' or 'allelic sequence' is an alternative form of the polypeptides described above. Alleles result from a mutation, eg a change in the nucleic acid sequence, and generally produce altered mRNA or polypeptide whose structure or function may or may not be altered. Any given polypeptide may have none, or more allelic forms. Common allelic changes that give rise to alleles are generally ascribed to natural deletions, additions or substitutions of amino acids. Each of these types of changes may occur alone, or in combination with the others, one or more times in a given sequence.

It is envisaged that the polynucleotide sequences of the present invention can be used as probes for the isolation of similar sequences from the strawberry genome and lemon genome. By using as a probe a cDNA of the gene sequences of the invention it would be possible by those skilled in the art to obtain comparable gene sequences from various tissues, particularly fruit tissues, from climacteric and non climacteric plants. One aspect of the invention is to provide for hybridisation or PCR probes which are capable of detecting polynucleotide sequences, including genomic sequence(s), encoding the polypeptides of the invention, or closely related molecules. The specificity of the probe, whether it is made from a highly specific region, eg 10 unique nucleotides in the 5' regulatory region, or a less specific region, e.g. in the 3' region, and the stringency of the hybridisation or amplification (maximal, high, intermediate, low) will determine whether the probe identifies only naturally occurring sequence(s) encoding the polypeptide, allele's or related sequences.

Probes may also be used for the detection of related sequences and preferably contain at least 50% of any of the nucleotides from any one of the gene encoding sequences according to the present invention. For gene sequences of the present invention, the hybridisation probes may be derived from the nucleotide sequence, or from genomic sequence including promoter, enhancer element and introns. Hybridisation probes may be labelled by a variety of reporter groups, including radionuclides such as ³²P or ³⁵S, or enzymatic labels such as alkaline phosphatase coupled to the probe *via* avidin/biotin coupling systems, and the like.

Other means for producing specific hybridisation probes for encoding gene sequences of the invention include the cloning of nucleic acid sequences into suitable vectors for the production of mRNA probes. Such vector are known in the art and are commercially available and may be used to synthesise RNA probes *in-vitro* by means of the addition of the appropriate RNA polymerase as T7 or SP6 RNA polymerase and the appropriately radioactively labeled nucleotides.

According to a third aspect the invention provides a recombinant expression vector comprising a coding sequence which is operably linked to a promoter sequence capable of directing expression of said coding sequence in a host cell for said vector, and a transcription termination sequence, in which the coding sequence is a DNA sequence according to the invention.

In a further embodiment the invention provides a replicative cloning vector comprising an isolated DNA sequence of the invention and a replicon operative in a host cell for said vector.

Methods which are well known to those skilled in the art can be used to construct expression vectors containing the gene coding sequence of the invention, and appropriate transcriptional and translational controls. These methods include *in-vitro* recombinant techniques. Such techniques are described in Sambrook *et al*., 1989. Molecular cloning a laboratory manual, cold spring Harbour press, Plain view, NY and Ausubel FM *et al*., (1989) Current protocols in molecular biology, John Wiley and Sons, New York, NY.

In order to express a biologically active polypeptide, the nucleotide sequence encoding the polypeptide, or the functional equivalents thereof, fragments of the polypeptide, is inserted into the appropriate expression vector (i.e. a vector that contains the necessary elements for the transcription or translation of the inserted coding sequence). Specific initiation signals may also be required for efficient translation of the polypeptides of the invention. These signals include the ATG initiation codon and adjacent sequences. In cases where the polypeptides, their initiation codons and upstream sequences are inserted into the appropriate expression vector, no additional translational control systems including the ATG initiation codon must be provided. Furthermore, the initiation codon must be in the correct reading frame to ensure transcription of the entire insert. Exogenous transcriptional elements and initation codons can be of various origins, both natural and synthetic. The efficiency of expression may be enhanced by the inclusion of enhancers appropriate to the cell system in use (Scharf D et al (1994) Results Prob Cell Differ 20: 125-62; Bittner *et al*. (1987) *Methods in Enzymol* 153: 516-544).

In addition a host strain may be chosen for its ability to modulate the expression of the inserted sequences or to process the expressed protein in the desired fashion. Such modifications of the polypeptide include, but are not limited to, acylation, carboxylation, glycosylation, phosphorylation and lipidation. Post translation processing which cleaves a 'prepro' form of the protein may also be important for correct insertion, folding and/or function. Different host cells which have the correct cellular machinery and characteristic mechanisms for such post-translational activities maybe chosen to ensure correct modification and processing of the introduced, foreign protein.

The nucleotide sequences of the present invention can be engineered in order to alter the coding sequence for a variety of reasons, including but not limited to, alterations which modify the cloning, processing, and/or expression of the gene product. For example mutations may be introduced using techniques which are well known in the state of the art, e.g. site directed mutagenesis to insert new restriction sites, to alter glycosylation patterns, to change codon usage, to produce splice variants etc. Codons may be selected to increase the rate at which expression of the peptide occurs in a particular procaroytic or eukaroytic expression host in accordance with the frequency with which particular codons are utilised by the host (Murray E *et al*. (1989) *Nuc Acids Res* 17: 477-508). Other reasons for substantially altering the nucleotide sequence(s) of the invention and their derivatives, without altering the encoded amino acid sequences include the production of RNA transcripts having more desirable properties, such as a greater half life, than transcripts produced from naturally occurring sequences.

In developing the expression cassette, the various fragments comprising the regulatory regions and open reading frame may be subjected to different processing conditions, such as ligation, restriction enzyme digestion, resection, *in-vitro* mutagenesis, primer repair, use of linkers and adapters and the like. Thus, nucleotide transitions, transversions, insertions, deletions and the like, may be performed on the DNA which is employed in the regulatory regions and/or open reading frame. The expression cassette may be wholly or partially derived from natural sources endogenous to the host cell. Furthermore, the various DNA constructs (DNA sequences, vectors, plasmids, expression cassettes) of the invention are isolated and/or purified, or synthesised and thus are not naturally occurring. The invention further contemplates the use of yet undescribed biological and non biological based expression systems and novel host(s) systems that can be can be utilised to contain and express the gene coding sequence(s) of the invention.

'Antisense' or 'partial sense' or other techniques may also be used to reduce the expression of genes in the pathway leading to the production of flavour volatile esters. Full length 'sense' techniques may be designed to increase or reduce the expression of genes in the pathway leading to the production of flavour volatiles. The 'antisense' or 'partial sense' molecules may be designed to block translation of mRNA by preventing the transcript from binding to the ribosomes. Ribozymes are enzymatic RNA molecules capable of catalysing the specific cleavage of RNA. The mechanism of ribozyme action involves sequence-specific hybridisation of the ribozyme molecule to complementary target RNA, followed by endonucleolytic cleavage. Within the scope of the invention are engineered hammerhead motif ribozyme molecules that can specifically and efficiently catalyse endonucleolytic cleavage of gene sequences of the invention. Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for secondary structural features which may render the oligonucleotide inoperable. The suitability of candidate targets may also be evaluated by testing accessibility to hybridisation with complementary oligonucleotides using ribonuclease protection assays.

'Antisense' molecules and ribozymes of the invention may be prepared by any method known in the art for the synthesis of RNA molecules. These include techniques for chemically synthesizing oligonucleotides such as solid phase phosphoramidite chemical synthesis. Alternatively, RNA molecules may be generated by *in-vitro* and *in-vivo* transcription of DNA sequences of the invention. Such DNA sequences may be incorporated into a wide variety of vectors with suitable RNA polymerase promoters such as T7 and SP6. Alternatively, antisense cDNA constructs that synthesise antisense RNA constitutively or inducibly can be introduced into cell lines, cells or tissues. RNA molecules may be modified to increase intracellular stability and half-life. Possible modifications include, but are not limited to, the addition of flanking sequences, at the 5' and/or 3' end of the molecule or the use of phosphorothioate or 2' O-methyl rather than phosphodiesterase linkages within the backbone of the molecule.

A variety of vector/host expression systems can be utilised to contain and express the gene coding sequences of the invention. These include and are not limited to micro-organisms such as bacteria (e.g. *E coli*, B *subtilis*, Streptomyces, Pseudomonads) transformed with recombinant bacteriophage, plasmid or cosmid DNA expression systems, yeast (e.g S. *cerevisiae*, *Kluyveromyces lactis*, *Pichia pastoris*, *Hansenula polymorpha*, *Schizosacch*. *Pombe*, *Yarrowia*) transformed with yeast expression vectors; filamentous *fungi* (*aspergillus nidulans*, *aspergillus orizae*, *aspergillus niger*) transformed with filamentous fungi expression vectors, insect cell systems transfected with virus expression vectors (eg baculovirus, adenovirus, herpes or vaccinia viruses); plant cell systems transfected with virus expression vectors (e.g. cauliflower mosaic virus, CaMV, tobacco mosaic virus, TMV) or transformed with bacterial expression vectors (e.g Ti or Pbr322 plasmid); or mammalian cell systems (chinese hamster ovary (CHO), baby hamster kidney (BHK), Hybridoma's, including cell lines of mouse, monkey, human and the like.

In the case of plant expression vectors, the expression of a sequence (s) of the invention may be driven by a number of previously defined promoters, including inducible and developmentally regulated promoters. The invention further contemplates the use of the individual promoters of the polynucleotide sequence(s) of the present invention for this purpose. In particular an alcohol acyl transferase promoter(s) pertaining to SEQ ID IA and SEQ ID 2A or any promoters particularly responsive to ripening events may be used to drive the tissue specific expression of the target genes. In addition, viral promoters such as the 35S and the 19S promoters of CaMV (Brisson *et al*., 1984; Nature, 310: 511 -514) may be used alone or in combination with the omega leader sequence from TMV (Takamatsu *et al*. (1987); *EMBO J* 6:307-311). Promoters or enhancers derived from the genomes of plant cells, tissue specific promoters i.e fruit specific promoters, Fbp7 (Columbo *et al*. 1997; Plant Cell 9; 703-715), 2A11 promoter (Pear *et al*., 1989, *Plant Molecular Biology*, 13:639-651), small subunit of Rubisco (Corruzzi *et al*., 1984; *EMBO J* 3:16; Broglie *et al.*, 1984 Science 224:838-843) or timing specific promoters such as ripening specific promoters (the E8 promoter, Diekman and Fisher, 1988, *EMBO J*, 7:3315-3320) may be used. Suitable terminator sequences include that of the *agrobacterium tumefaciens* nopaline synthase gene (Nos 3' end), the tobacco ribulose bisphosphate carboxylase small subunit termination region; and other 3' regions known to those skilled in the art. These constructs can be introduced into plant cells by direct DNA transformation, or pathogen mediated transfection. For reviews of such techniques, see Hobbs S or Murry LE, in McGraw Hill yearbook of Science and technology (1992), Mc Graw Hill NY, pp 191-196 or Weissbach and Weissbach (1988) Methods for Plant Molecular Biology, Academic Press, New York, NY, pp421-463, Fillatti *et al*., Biotechnology, 5: 726-730. Manipulation of DNA sequences in plant cells may be carried out using the Cre/lox site specific recombination system as outlined in patent application WO91-09957.

According to a fourth aspect the invention provides a method for regulating aliphatic and/or aromatic ester formation in fruit, comprising inserting into the genome of a fruit-producing plant one or more copies of one or more DNA sequences of the invention.

The activity of genes according to the present invention involved in the biosynthetic pathway leading to volatile ester formation, hence flavour, may be either increased or reduced depending on the characteristics desired for the modified plant part. The gene sequence may be the in the same 'sense' or 'antisense' orientation as the endogenous target gene. Methods well known to those skilled in the art can be used to construct recombinant vectors which will express 'sense' or 'antisense' polynucleotides encoding the gene sequences of the present invention. Such technology is now well known in the art. In the case of the 'plant cell' officiating as 'host cell', the target 'plant cell' may be part of a whole plant or may be an isolated cell or part of a tissue which may be regenerated into a whole plant. The target plant may be selected from any monocotyledonous or dicotyledonous plant species. Suitable plants include any fruit bearing plant such as strawberry, citrus (lemon), banana, apple, pear, melon, sweet pepper, peach or mango. Other suitable plant hosts include vegetable, ornamental (to include sunflower) and arable crops (to include soybean, sunflower, corn, peanut, maize, wheat, cotton, safflower, and rapeseed). For any particular plant cell, the gene sequences, cDNA, genomic DNA, or synthetic polynucleotide, used in the transformation vector construct, may be derived from the same plant species, or may be derived from other plant species (as there will be sufficient homology to allow modification of related enzyme gene function). The procedure or method for preparing a transformant can be performed according to the conventional technique used in the fields of molecular biology, biotechnology and genetic engineering.

According to a fifth aspect the invention provides a plant and propagating material thereof which contains in its genome a DNA sequence of the invention or a vector as defined above.

In a preferred embodiment the invention provides a genetically modified strawberry or lemon plant and propagating material derived therefrom which has a genome comprising an expression vector for over-expression or down-regulation of an endogenous strawberry or lemon plant gene counterpart of any of the DNA sequences of the invention.

In mammalian cells a number of viral based systems may be utilised. In cases where the adenovirus is used as an expression vector, a gene coding sequence(s) of the invention may be ligated into the adenovirus transcription/translation complex consisting of the late promoter and tripartite leader sequence. Insertion in a non essential E1 or E3 region of the viral genome will result in a variable virus capable of expressing the gene sequence(s) in infected host cells (Logan and Shark (1984) *Proc Natl Acad. Sci* 81:3655-59) In addition transcription enhancers, such as the *Rous sarcoma* virus (RSV) enhancer, may be used to increase expression in mammalian host cells.

Furthermore insect cells such as silkworm cells or larvae themselves may be used as a host. In one such system, *Autographa californica* nuclear polyhedrosis virus (AcNPV) is used as a vector to express foreign genes in *Spodoptera frugiperda* cells or in *Trichoplusia larvae*. The gene coding sequence(s) of the invention may be cloned into the nonessential region of the virus, such as the polyhedrin gene, and placed under control of a polyhedrin promoter. Successful insertion of the sequence(s) will render the polyhedrin gene inactive and produce recombinant virus lacking coat protein coat. The recombinant viruses are then used to *infect S frugiperda* cells or *Trichoplusia larvae* in which the gene sequence or sequences are expressed (*Smith et al*. (1993) J Virol 46:584; Engelhard *et al.* (1994) *Proc. Natl acad Sci*, 91: 3224-7).

The control elements or regulatory sequences of the described systems vary in their strength and specificities and are those nontranslated regions of the vector, enhancers, promoters and 3' untranslated regions, which interact with host cellular proteins to carry out transcription and translation. Dependent on the vector system and host utilised, any number of suitable transcription and translation elements, including constitutive and inducible promoters, may be used. For example, when cloning in bacterial systems, inducible promoters such as the hybrid lacZ promoter of the Bluescript ® phagemid (Strategene, La Jolla) or pSPORT (Gibco BRL) and ptrp-lac hybrids and the like may be used. Other conventionally used promoters are trc and trc promoters. The baculovirus polyhedrin promoter may be used for insect cells. As a promoter for filamentous organisms, for example, amalyse, trp C, and the like may be used. As a promoter for yeast for example glyceraldehyde-3-phosphate dehydrogenase may be used. As a promoter for animal cell hosts, viral promoters such as the SV40 early promoter, SV40 late promoter and the like may be used. In the yeast *Saccharomyces cerevisiae*, a number of vectors containing constitutive or inducible promoters such as the alpha factor, alcohol oxidase and PGH may be used. For review, see Grant *et al*., 1987 Methods in Enzymology 153: 516-544.

For long term, high yield production of recombinant proteins, stable expression is preferred. For example cell lines which stably express the polypeptides of the invention may be transformed using expression vectors which contain viral origins of replication or endogenous expression elements and a selectable marker gene. Following the introduction of the vector, cells may be allowed to grow for one to two days in an enriched media before they are switched to selective media. The purpose of the selectable marker is to confer resistance to selection and its presence allows growth and recovery of cells which successfully express the introduced sequences. Resistant clumps of stable transformed cells can be proliferated using tissue culture techniques appropriate to the cell type.

Although the presence/absence of the marker gene expression suggests that the gene of interest is also present, its presence and expression must be confirmed. For example, if the gene sequence(s) of the invention are inserted within a marker gene sequence, recombinant cells can be identified by the absence of gene function. Alternatively a marker gene can be placed in tandem with the gene sequence of interest, under the control of a single promoter. Expression of the marker gene in response to induction or selection usually indicates expression of the tandem gene as well.

Any number of selection systems may be used to recover the transformed cell lines. Anti-metabolite, antibiotic or herbicide resistance can be used as the basis of selection; for example, dhfr which confers resistance to methotrextate (Wiger M. *et al*. (1980) *Proc Natl Acad* Sci 77: 3567-70; npt, which confers resistance to the aminoglycosides neomycin and G-418 (Colbere-Garapin F. *et al.* (1981) *J Mol Biol* 150: 1-14) and als and pat, which confer resistance to chlorsulfuron and phosphinotricin acyl transferase, respectively (Murry supra). Additional selectable genes have been transcribed, for example, trpB, which allows cells utilise indole in place of tryptophan, or His D, which allows cells to utilise histinol in place of histidine (Hartman SC and RC Mulligan (1988) *Proc Natl Acad Sci* 85: 8047-51). Alternatively one could use visible markers such as anthocyanidins, β-glucuronidase and its substrates, GUS, GFP and variants, and luciferase and its substrate, luciferin, which are widely used to identify transformants, but also to quantify the amount of stable protein expression attributable to a specific vector system (Rhodes CA *et al*. (1995) *Methods Mol Biol* 55: 121-131.

Host cells transformed with a nucleotide sequence(s) of the invention, may be cultured under conditions suitable for expression and recovery of the encoded protein from cell culture. The protein produced by recombinant cells may be secreted or contained intracellularly depending on the sequence and/or the vector used. As will be understood by those skilled in the art, expression vectors containing polynucleotides sequences of the invention, can be designed with signal sequences for direct secretion of the protein product, through a prokaroytic or eukaryotic cell membrane. Other recombinant constructions may join the gene sequences of the invention, to a nucleotide sequence encoding a polypeptide domain, which will facilitate purification of soluble proteins (Kroll DJ *et al*. (1993). *DNA Cell Biol* 12: 441-53).

According to a sixth aspect the invention provides a method for producing aromatic and/or aliphatic esters in microorganisms, plant cells or plants comprising inserting into the genome of the microorganism or plant one or more copies of DNA sequences of the invention, and feeding an alcohol and an acyl-CoA to the microorganism or plant (cell).

Alternatively, host cells which contain the gene sequences or polypeptides thereof according to the invention may be identified by a variety of procedures known to those skilled in the art. These procedures include, but are not limited to DNA-DNA, DNA-RNA hybridisation, amplification using probes (portions or fragments of polynucleotides), protein bioassay or immunoassay techniques which include membrane, solution or chip based technologies for the detection and /or quantification of the nucleic acid or protein.

Nucleic acid amplification based assays involve the use of oligonucleotides or oligomers based on the gene sequences of the invention to detect transformants containing DNA or RNA encoding the polypeptides. As used herein 'oligonucleotides' or 'oligomers' refer to nucleic acid sequence of at least 10 nucleotides and as many as about 60 nucleotides, preferably about 15-30 nucleotides, and more preferably about 20-25 nucleotides which can be used as a probe or amplimer. Polynucleotide sequences according to the invention encoding alcohol acyl transferase polypeptides may be used to detect volatile ester (aliphatic and aromatic) formation in fruits. PCR as described in US patent Nos 4,683,195 and 4,965,188, provides additional uses for oligonucleotides based on the alcohol acyl transferase sequence. Such oligomers are chemically synthesised, but they may be generated enzymatically or produced from a recombinant source. Oligomers generally comprise two nucleotide sequences, one with sense orientation (5'-3') and one with antisense (3'-5'), employed under less stringent conditions for detection and /or quantitation of closely related DNA or RNA sequences. For example, polynucleotide sequences encoding the alcohol acyl transferase protein may be used in the hybridisation or PCR assay of plant tissues, more specifically fruit tissues, to detect alcohol acyl transferase protein presence. The form of such quantitative methods may include, Southern or Northern analysis, dot/slot blot or other membrane based technologies; PCR technologies such as DNA Chip, Taqman and ELISA Technologies. All of these technologies are well known in the art and are the basis of many commercially owned diagnostic kits. The polynucleotide sequences according to the present invention herein provide the basis for assays that detect signal transduction events associated with volatile ester production, aliphatic and aromatic, hence flavour formation.

A variety of protocols for detecting and measuring expression of the polypeptides of the invention, using either polyclonal or monoclonal antibodies specific for the protein are well known in the art. Examples include enzyme-linked immunosorbent assay (ELISA), radioimmunoassay (RIA) and fluorescent activated cell sorting (FACS). These methods are described, among other places, in Hampton R *et al.* (1990), Serological methods, a laboratory manual, APS Press, St Paul MN) and Maddox DE *et al*. (1993, *J Exp Med* 158:1211).

A variety of labels and conjugation techniques are known by those skilled in the art and can be used in various nucleic acid and amino acid assays. Means for producing labelled hybridisation or PCR probes for detecting sequences related to polynucleotides as outlined in the first and second aspects of the invention, include oligolabeling, nick translation, end labelling of PCR amplification using a labelled nucleotide. Alternatively, the gene sequence or sequences of the invention, or any portion of it, may be cloned into a vector for the production of the mRNA probe. Such vectors are known in the art, are commercially available, and may be used to synthesise RNA probes *in-vitro* by addition of an appropriate RNA polymerase, such as T7, T3, or SP6 and labelled nucleotides. A number of companies such as Pharmacia Biotech (Piscataway NJ), promega (Madisson WI), and US Biochemical Corp (Cleveland OH) supply commercial kits and protocols for these procedures. Suitable reporter molecules or labels include fluorescent, chemiluminescent, or chromogenic agents as well as substrates, cofactors, inhibitors, magnetic particles and the like.

According to another aspect of the invention, natural, modified or recombinant polynucleotide sequences according to the invention may be ligated to a heterologous sequence to encode a fusion protein. A fusion protein may be engineered with one or more additional domains added to facilitate protein purification (i.e. a cleavage site located between the sequence of the polypeptide and the heterologous protein sequence) so that the polypeptide of the invention may be purified away from the heterologous moiety. The invention further contemplates the creation of fusion proteins, chosen from the group of sequences of the invention, degenerate variants thereof, functional equivalents thereof, pertaining to 'fruit' of strawberry and lemon, or gene sequences with substantial homology to the sequences of the invention from other 'fruit' tissues concerned with the pathway for the formation of aliphatic or aromatic esters, hence flavour. Such purification facilitating domains include, but are not limited to, metal chelating peptides such as histidine-tryptophan modules that allow purification on immobilised metals, protein A domains that allow purification on immobilised immunoglobin, and the domain utilised in the FLAGS extension/affinity purification system (Immunex Corp. Seattle, WA). The introduction of a cleavable linker such as factor XA, thrombin or enterokinase (Invitrogen, San Diego CA) between the purification domain and the polypeptide is useful to facilitate purification. One such expression vector provides for the expression of a fusion protein comprising gene sequence(s) which encode polypeptides of the invention, and contains nucleic acid encoding six histidine residues followed by thioredoxin and the enterokinase cleavage site. The histidine residues facilitate purification on IMIAC (immobilised metal ion affinity chromatography as described in Porath *et al*., (1992) Protein Expression and Purification 3:263-281) while the enterokinase cleavage site provides a means for purifying the polypeptide from the fusion protein.

According to a seventh aspect the invention provides an isolated DNA sequence encoding
(a) a polypetide having an amino acid sequence as shown in SEQ ID NO: 3B, or
(b) a polypeptide having at least 80% homology with the amino acid sequence SEQ ID NO: 3B, or
(c) a fragment of polypeptide (a) or (b),
   which polypeptide or fragment of polypeptide has aminotransferase activity and is involved in the biosynthetic pathway for aliphatic and/or aromatic ester production in fruit.

In particular the invention provides an isolated DNA sequence having a nucleic acid sequence
(a) as shown in SEQ ID NO: 3A which encodes a polypeptide having aminotransferase activity and being involved in the biosynthetic pathway for aliphatic and/or aromatic ester production in fruit, or
(b) complementary to SEQ ID NO: 3A, or
(c) which has at least 70 % homology with any of the sequences of (a) or (b), or
(d) which is capable of hybridising under stringent conditions to any of the sequences (a) - (c).

Further the invention provides a purified and isolated polypeptide,
(a) having an amino acid sequence as shown in SEQ ID NO: 3B, or
(b) having at least 80% homology with the amino acid sequence SEQ ID NO: 3B, or
(c) being a fragment of polypeptide (a) or (b), which polypeptide or fragment of polypeptide has aminotransferase activity and is involved in the biosynthetic pathway for aliphatic and/or aromatic ester production in fruit.

According to an eighth aspect the invention provides an isolated DNA sequence encoding
(a) a polypeptide having an amino acid sequence as shown in SEQ ID NO: 4B, or
(b) a polypeptide having at least 90% homology with the amino acid sequence SEQ ID NO: 4B, or
(c) a fragment of polypeptide (a) or (b),
   which polypeptide or fragment of polypeptide has thiolase activity and is involved in the biosynthetic pathway for aliphatic and/or aromatic ester production in fruit.

In particular the invention provies an isolated DNA sequence having a nucleic acid sequence
(a) as shown in SEQ ID NO: 4A which encodes a polypeptide having thiolase activity and being involved in the biosynthetic pathway for aliphatic and/or aromatic ester production in fruit, or
(b) complementary to SEQ ID NO: 4A, or
(c) which has at least 75 % homology with any of the sequences of (a) or (b), or
(d) which is capable of hybridising under stringent conditions to any of the sequences (a) - (c).

Further the invention provides a purified and isolated polypeptide,
(a) having an amino acid sequence as shown in SEQ ID NO: 4B, or
(b) having at least 90% homology with the amino acid sequence SEQ ID NO: 4B, or
(c) being a fragment of polypeptide (a) or (b), which polypeptide or fragment of polypeptide has thiolase activity and is involved in the biosynthetic pathway for aliphatic and/or aromatic ester production in fruit.

According to a ninth aspect the invention provides an isolated DNA sequence encoding
(a) a polypetide having an amino acid sequence as shown in SEQ ID NO: 5B, or
(b) a polypeptide having at least 90% homology with the amino acid sequence SEQ ID NO: 5B, or
(c) a fragment of polypeptide (a) or (b),
   which polypeptide or fragment of polypeptide has pyruvate decarboxylase activity and is involved in the biosynthetic pathway for aliphatic and/or aromatic ester production in fruit.

In particular the invention provides an isolated DNA sequence having a nucleic acid sequence
(a) as shown in SEQ ID NO: 5A which encodes a polypeptide having pyruvate decarboxylase activity and being involved in the biosynthetic pathway for aliphatic and/or aromatic ester production in fruit, or
(b) complementary to SEQ ID NO: 5A, or
(c) which has at least 75 % homology with any of the sequences of (a) or (b), or
(d) which is capable of hybridising under stringent conditions to any of the sequences (a) - (c).

Further the invention provides a purified and isolated polypeptide,
(a) having an amino acid sequence as shown in SEQ ID NO: 5B, or
(b) having at least 90% homology with the amino acid sequence SEQ ID NO: 5B, or
(c) being a fragment of polypeptide (a) or (b), which polypeptide or fragment of polypeptide has pyruvate decarboxylase activity and is involved in the biosynthetic pathway for aliphatic and/or aromatic ester production in fruit.

According to a tenth aspect the invention provides an isolated DNA sequence encoding
(a) a polypetide having an amino acid sequence as shown in SEQ ID NO: 6B, or
(b) a polypeptide having at least 75% homology with the amino acid sequence SEQ ID NO: 6B, or
(c) a fragment of polypeptide (a) or (b),
   which polypeptide or fragment of polypeptide has alcohol dehydrogenase activity and is involved in the biosynthetic pathway for aliphatic and/or aromatic ester production in fruit.

In particular the invention provides an isolated DNA sequence having a nucleic acid sequence
(a) as shown in SEQ ID NO: 6A which encodes a polypeptide having alcohol dehydrogenase activity and being involved in the biosynthetic pathway for aliphatic and/or aromatic ester production in fruit, or
(b) complementary to SEQ ID NO: 6A, or
(c) which has at least 65 % homology with any of the sequences of (a) or (b), or
(d) which is capable of hybridising under stringent conditions to any of the sequences (a) - (c).

Further the invention provides a purified and isolated polypeptide,
(a) having an amino acid sequence as shown in SEQ ID NO: 6B, or
(b) having at least 75% homology with the amino acid sequence SEQ ID NO: 6B, or
(c) being a fragment of polypeptide (a) or (b), which polypeptide or fragment of polypeptide has alcohol dehydrogenase activity and is involved in the biosynthetic pathway for aliphatic and/or aromatic ester production in fruit.

According to an eleventh aspect the invention provides an isolated DNA sequence encoding
(a) a polypeptide having an amino acid sequence selected from the group consisting of sequences SEQ ID NO: 7B, 8B, 9B and 10B, or
(b) a polypeptide having at least
   i) 55% homology with the amino acid sequence of a 326 aa fragment from the C terminal end of the coding sequence of SEQ ID NO: 7B, or
   ii) 75% homology with the amino acid sequence of a 278 aa fragment from the C terminal end of the coding sequence of of SEQ ID NO: 8B, or
   iii) 85% homology with the amino acid sequence of a 284 aa fragment from the C terminal end of the coding sequence of SEQ ID NO: 9B, or
   iv) 80% homology with the amino acid sequence of a 188 aa fragment from the C terminal end of the coding sequence of SEQ ID NO: 10B, or
(c) a fragment of polypeptide (a) or (b),
   which polypeptide or fragment of polypeptide has alcohol dehydrogenase activity and is involved in the biosynthetic pathway for aliphatic and/or aromatic ester production in fruit.

In particular the invention provides an isolated DNA sequence having a nucleic acid sequence
(a) selected from the group consisting of sequences SEQ ID NO: 7A, 8A, 9A and 10A, which encodes a polypeptide having alcohol dehydrogenase activity and being involved in the biosynthetic pathway for aliphatic and/or aromatic ester production in fruit, or
(b) complementary to SEQ ID NO: 7A, 8A, 9A or 10A, or
(c) which has at least 55% homology, preferably at least 65% homology, with any of the sequences of (a) or (b), or
(d) which is capable of hybridising under stringent conditions to any of the sequences (a) - (c).

Further the invention provides a purified and isolated polypeptide,
(a) having an amino acid sequence selected from the group consisting of sequences SEQ ID NO: 7B, 8B, 9B and 10B, or
(b) having at least
   i) 55% homology with the amino acid sequence of a 326 aa fragment from the C terminal end of the coding sequence of of SEQ ID NO: 7B
   ii) 75% homology with the amino acid sequence of a 278 aa fragment from the C terminal end of the coding sequence of SEQ ID NO: 8B, or
   iii) 65% homology with the amino acid sequence of a 284 aa fragment from the C terminal end of the coding sequence of SEQ ID NO: 9B, or
   iv) 80% homology with the amino acid sequence of a 188 aa fragment from the C terminal end of the coding sequence of SEQ ID NO: 10B, or
(c) being a fragment of polypeptide (a) or (b), which polypeptide or fragment of polypeptide has alcohol dehydrogenase activity and is involved in the biosynthetic pathway for aliphatic and/or aromatic ester production in fruit.

According to a twelfth aspect the invention provides an isolated DNA sequence encoding
(a) a polypeptide having an amino acid sequence as shown in SEQ ID NO: 11B, or
(b) a polypeptide having at least 75% homology with the amino acid sequence of a 181 aa fragment from the C terminal end of the coding sequence of SEQ ID NO: 11B, or
(c) a fragment of polypeptide (a) or (b),
   which polypeptide or fragment of polypeptide has alcohol dehydrogenase activity and is involved in the biosynthetic pathway for aliphatic and/or aromatic ester production in fruit.

In particular the invention provides an isolated DNA sequence having a nucleic acid sequence
(a) as shown in SEQ ID NO: 11A which encodes a polypeptide having alcohol dehydrogenase activity and being involved in the biosynthetic pathway for aliphatic and/or aromatic ester production in fruit, or
(b) complementary to SEQ ID NO: 11A, or
(c) which has at least 48 % homology with any of the sequences of (a) or (b), or
(d) which is capable of hybridising under stringent conditions to any of the sequences (a) - (c).

Further the invention provides a purified and isolated polypeptide,
(a) having an amino acid sequence as shown in SEQ ID NO: 11B, or
(b) having at least 75% homology with the amino acid sequence of a 181 aa fragment from the 3' end of SEQ ID NO: 11B, or
(c) being a fragment of polypeptide (a) or (b), which polypeptide or fragment of polypeptide has alcohol dehydrogenase activity and is involved in the biosynthetic pathway for aliphatic and/or aromatic ester production in fruit.

According to a thirteenth aspect the invention provides an isolated DNA sequence encoding
(a) a polypeptide having an amino acid sequence selected from the group consisting of sequences SEQ ID NO: 12B and 13B, or
(b) a polypeptide having at least
   i) 55% homology with the amino acid sequence of a 176 aa fragment from the C terminal end of the coding sequence of SEQ ID NO: 12B, or
   ii) 35% homology with the amino acid sequence of a 284 aa fragment from the C terminal end of the coding sequence of SEQ ID NO: 13B, or
(c) a fragment of polypeptide (a) or (b),
   which polypeptide or fragment of polypeptide has alcohol dehydrogenase activity and is involved in the biosynthetic pathway for aliphatic and/or aromatic ester production in fruit.

In particular the invention provides an isolated DNA sequence having a nucleic acid sequence
(a) selected from the group consisting of sequences SEQ ID NO: 12A and 13A, which encodes a polypeptide having alcohol dehydrogenase activity and being involved in the biosynthetic pathway for aliphatic and/or aromatic ester production in fruit, or
(b) complementary to SEQ ID NO: 12A or 13A, or
(c) which has at least 20% homology, preferably at least 30% homology, with any of the sequences of (a) or (b), or
(d) which is capable of hybridising under stringent conditions to any of the sequences (a) - (c).

Further the invention provides a purified and isolated polypeptide,
(a) having an amino acid sequence selected from the group consisting of sequences SEQ ID NO: 12B and 13B, or
(b) having at least
   i) 55% homology with the amino acid sequence of a 176 aa fragment from the C terminal end of the coding sequence of SEQ ID NO: 12B, or
   ii) 35% homology with the amino acid sequence of a 284 aa fragment from the C terminal end of the coding sequence of SEQ ID NO: 13B, or
(c) being a fragment of polypeptide (a) or (b), which polypeptide or fragment of polypeptide has alcohol dehydrogenase activity and is involved in the biosynthetic pathway for aliphatic and/or aromatic ester production in fruit.

According to a fourteenth aspect the invention further provides a method for regulating aliphatic and/or aromatic ester formation in fruit comprising inserting into the genome of a fruit-producing plant one or more copies of one or more DNA sequences as defined above encoding various enzymes involved in the biosynthetic pathway for aliphatic and/or aromatic ester formation in fruit.

The levels of protein may be increased for example by the incorporation of additional genes. The additional genes maybe designed to give either the same or different spatial or temporal patterns of expression in the fruit as the target gene.

Further the invention provides a genetically modified strawberry or lemon plant and propagating material derived therefrom which has a genome comprising an expression vector for overexpression or downregulation of an endogenous strawberry or lemon plant gene counterpart of any of the DNA sequences as defined above encoding various enzymes involved in the biosynthetic pathway for aliphatic and/or aromatic ester formation in fruit.

Further the invention provides a method for producing aromatic and/or aliphatic esters in microorganisms, plant cells or plants comprising inserting into the genome of the microorganism or plant one or more copies of DNA sequences of this invention encoding a polypeptide having alcohol acyl transferase activity, and one or more copies of DNA sequences of this invention encoding a polypeptide having alcohol dehydrogenase activity, and feeding aldehydes and acyl-CoA to the microorganism or plant (cell).

In a further embodiment the above method further comprises inserting one or more copies of DNA sequences of this invention encoding a polypeptide having pyruvate decarboxylase activity, and feeding alpha-keto acids and acyl-CoA to the microorganism or plant (cell).

In a still further embodiment the above method further comprises inserting one or more copies of DNA sequences of this invention encoding a polypeptide having aminotransferase activity, and feeding amino acids and acyl-CoA to the microorganism or plant (cell).

In a still further embodiment the above method further comprises inserting one or more copies of DNA sequences of this invention encoding a polypeptide having thiolase activity, and feeding amino acids and fatty acids to the microorganism or plant (cell).

Strawberry and citrus are among the most popular fruits for natural flavour ingredients because of their flavour, fragrance, aroma and scent. Examples of commercially important aliphatic esters are ethyl acetate (used in artificial fruit and brandy flavours), ethyl butyrate (the primary constituent of apple aromas), isoamyl acetate (the main component of banana aroma) and ethyl butyrate (has a fruity odour, reminiscent of pineapple). Flavour usage of these esters is among the highest of all natural flavour ingredients. The invention further contemplates the use of the polynucleotide sequences according to the present invention, for the industrial production of 'fruit' flavours which are natural to match the odour fidelity of the natural fruit. As the chemical composition of flavour is quite complex, both the type and ratio of compounds present being the key determinant of flavour quality, this present invention further contemplates the production of novel flavours by the use of the polynucleotide sequences according to the present invention, alone or in combination, to provide novel avenues for natural flavour production in the future.

The invention further contemplates the generation of strawberry and lemon plants which amongst other phenotypic modifications may have one of the following characteristics:
(a) reduced/enhanced flavour from the reduced/enhanced production of volatile esters,
(b) increased disease resistance due to the enhanced production of volatile esters,
(c) modified plant-insect interactions, to include increased insect resistance.

In a further aspect, the gene sequences of the present invention are used for the manipulation of flavour in other 'fruits' and or/ industrial processes. The genes/ peptides of the present invention, in particular SEQID 1A (strawberry SLE27) and SEQ ID 1B (lemon CLF26) may be used in the following industrial applications; with particular reference to the manipulation of fruit derived products, however, not exclusively limited to these:
a) in the processed food industry as food additives to enhance the flavour of syrups, ice-creams, frozen desserts, yoghurts, confectionery, and like products,
b) flavouring agents for oral medications and vitamins,
c) providing additional flavour/aroma in beverages, including alcoholic beverages,
d) enhancing or reducing fruit flavour/aroma/fragrance/scent,
e) enhancing the flavour/aroma of 'natural products (eg flower scent however, including but not exclusively limited to flower scent),
f) enhancing the flavour/aroma of 'synthetic or artificial products',
g) as an alternative to the industrial synthesis of nature identical flavour/aroma substances,
h) for the production of novel combinations of artificial flavour substances,
i) as antibacterial or as anti-fungal agents,
j) fragrance /perfumes in the cosmetics, creams, sun-protectant products, hair conditioners, lengthening agent and fixative in perfumes, suspension aid for aluminium salts in anti-perspirants pharmaceuticals, cleaning products, personal care products and animal care products,
k) disinfectant additives,
l) as degreasing solvents for electronics (etc.),
m) insect pheromones,
n) dye carrier, solvents, insect repellent, miticide, scabicide, plasticiser, deodorants.

An example as way of illustration of the industrial importance of volatile esters (aromatic and aliphatic) is the ester methyl anthranillate. Methyl anthranillate, a methyl ester of o-aminobenzoic acid, apart from being a very important perfumary, aroma, scent ester (it imparts a peculiar aroma known as "foxy" to grapes of the North american species *Vitis Labrusca*), the same compound is known to serve as a pheromone in various insects. Results of investigations aimed at testing the attractiveness of methyl anthranilate for the the soybean beetle *Anomala rufocurea*, Motschulsky suggested the potential use of methyl anthranilate as a lure for mass trapping, as well as for monitoring (Imai *et al*., Applied Entomology and Zoology, 32: 1, 45 - 48, 1997). In addition methyl anthrenilate acts as a bird repellent. According to the invention we propose the use of the SEQ1D: 2A for the production of methyl anthranilate, for a number of applied applications.

The present invention also relates to antibodies which specifically bind to the polypeptides as claimed in the invention, compositions comprising substantially purified polypeptides, fragments thereof, agonists or alternatively antagonists, and methods of producing polypeptides. In bacterial/prokaroyte systems a number of expression vectors may be selected depending on the used of the polypeptide(s) according to the invention. For example, when large quantities of the polypeptide are needed for the induction of the antibody, vectors which drive high levels of expression of fusion proteins that are readily purified are desirable. Such vectors include, but are not limited to the multi-functional *E.coli* cloning and expression vectors, such as Bluescript® (Stratagene), in which the gene sequence(s) according to the invention, can be ligated into the vector in frame with sequences for the amino-terminal Met and the subsequent 7 residues of β-galactosidase so that the hybrid protein is produced; pPIN vectors (Van Heekle and Schuster (1989) *J Biol chem* 264: 503-5509); and the like. pGEX vectors (Promega, Madison WI) may also be used to express foreign polypeptides as fusion proteins with glutathione-S-transferase (GST). In general such fusion proteins are soluble and can be easily purified from lysed cells by absorption to gluthionine-agarose beads followed by elution in the presence of free gluthionine. Proteins made in such systems are designed to include heparin or factor XA protease cleavage sites so that the cloned polypeptide of interest can be released from the GST moiety at will.

In addition to recombinant production the amino acid sequences of the polypeptides, or any parts thereof, may be altered by direct synthesis or by genetic means known by those skilled in the art, and/or combined using chemical methods with sequences from other proteins, or any part thereof, to produce a variant polypeptide. Fragments of the polypeptides according to the invention may be produced by direct peptide synthesis using solid phase techniques (c.f. Stewert *et al.* (1969) Solid Phase Peptide Synthesis, WH Freeman Co, San Fancisco, Merrifield J (1963) *J Am Them Soc* 85:2149-2154). *In vitro* protein synthesis may be performed using manual techniques or by automation. Automated synthesis may be achieved, for example, using Applied Biosystems 431A Peptide Synthesizers (Perkin Elmer, Forster City CA) in accordance with the instructions provided by the manufacturer. Various fragments of each of the polypepides may be chemically synthesised separately and combined using chemical methods to produce full length molecules.

The invention further contemplates a diagnostic assay using antibodies raised to each of the polypeptides of the present invention, with or without modification. For example, although not exclusively limited to, alcohol acyl transferase antibodies raised to the polypeptides as outlined in the 6-8th embodiments are useful for the detection of flavour forming esters in various fruits. Diagnostic assays for alcohol acyl transferase include methods utilising the antibody and a label to detect the alcohol acyl transferase protein in various fruit extracts. Frequently the polypeptides and antibodies are labelled by joining them, either covalently or non covalently, with a reporter molecule. A variety of reporter molecules are known and several of them have been previously described (cf. reporter genes).

A variety of protocols for measuring the polypeptide, using either polyclonal or monoclonal antibodies specific for the respective protein are known in the art. Polynucleotide sequence(s) of the invention, SEQ ID 1A strawberry (SLE27), SEQ ID 2A, lemon alcohol acyl transferase proteins (CLF26), or any part thereof, may be used for diagnostic purposes, to detect and quantify gene expression in various tissues (eg leaves, stems and in particular fruits (climacteric and non climacteric)), hence volatile ester (aliphatic and aromatic) formation, hence flavour at various stages of plant maturation. The diagnostic assay is useful to distinguish between absence, presence and excess expression of the alcohol acyl transferase proteins and to monitor expression levels during development.

The following examples are offered by way of illustration and not by limitation.

### Example 1: Method for the detection of volatiles

The composition of the headspace of the strawberries was analysed using Solid Phase Micro-extraction for trapping and concentrating the emitted volatiles. The fibres used were 100 µm Polydimethyl siloxane (PDMS) supplied by Supelco. Analysis was performed on a Fisons GC 8000 system, coupled to the Fisons MD800 mass spectrometer. The gas chromatograph was temperature programmed, with a plateau at 80 °C for 2 min., followed by a ramp of 8°C/min to 250 °C for 5 min. The injector port was operated at 250°C for thermal desorption during 60 sec. of the SPME fibers, using the deactivated standard 0.7 ml split/splitless injection port liner with zero split. The column used was a HP-5 with a length of 50 m and internal diameter of 0.32 mm and a film thickness of 1.03 µm, and operated with helium at a pressure of 38kPa. Eluted components were identified by their mass spectrum, using the Fisons Masslab software and the NIST library. The identity of the components was verified where possible by using known substances as reference (most of them obtained from Aldrich).

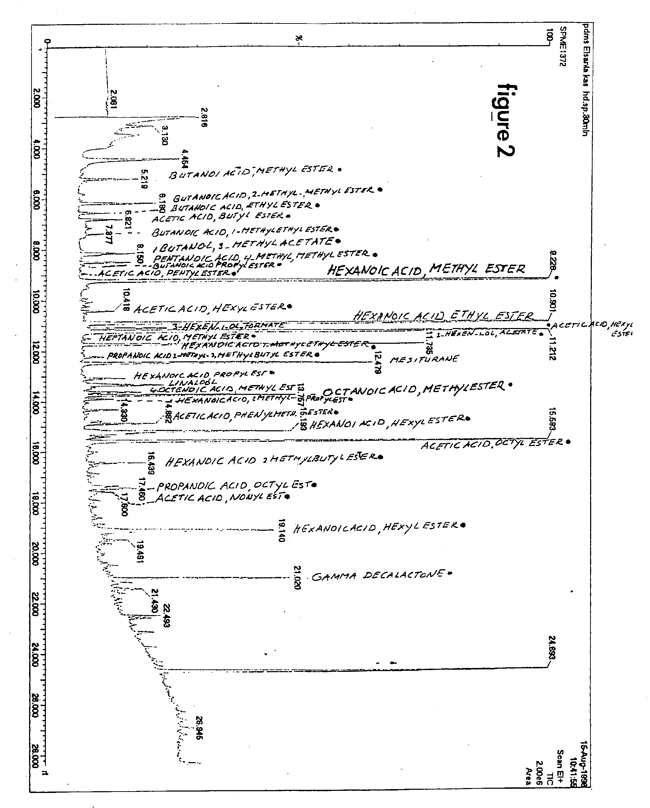

Headspace analyses were performed both on the plant and in the laboratory using detached fruits. The fruits were put onto new clean aluminium foil and covered with a glass funnel closed with aluminium foil. The SPME fiber was inserted in the funnel through the foil closing, and the fiber was exposed to the air in the funnel during 30 min. Figure 2 shows a typical chromatogram obtained after headspace measurement of red ripe fruit of strawberry grown in the greenhouse. The importance of esters to the overall volatile emission from the ripe strawberry fruit can be clearly observed.

### Example 2: Construction of a strawberry red fruit stage and lemon young fruit peel cDNA library, mass excision and random sequencing

### 2.1 Messenger RNA isolation and cDNA libraries construction

Total RNA was isolated from strawberry fruit red stage of development using the method described by Manning K. [Analytical Biochemistry 195, 45-50 (1991)]. The cultivar used was *Fragaria X ananassa Duch. cv. Elsanta*. Plant material for the isolation of RNA from lemon (citrus Limon cv. *Meyer*) was the green peel of a small fruit (1.2 x 2 cm) of the cultivar. Isolation of RNA from lemon was done using the same method as described for strawberry. The cDNA libraries for both strawberry and lemon where produced as a custom service by (Stratagene) in the lambda zap vector. Messenger RNA was isolated from total RNA using the polyA+ isolation kit (Pharmacia).

### 2.2 Mass excision and random sequencing

The ExAssist™/SOLR™ system (Stratagene) was used for mass excision of the pBluescript SK(-) phagemid. The excision was done according to the manufacturer's instructions using 20 × 10³ pfu from the non amplified library for each excision. High quality plasmid DNA from randomly picked colonies was extracted using the QIAGEN BioROBOT 9600. Colonies were grown overnight in 3ml Luria Broth medium (10g/l tryptone, 5 g/liter yeast extract, 5 g/liter NaCl) supplemented with 100 mg/litre Ampicillin, centrifuged at 3000 RPM for 10 min. and the pellet was used directly for plasmid DNA isolation by the robot. Each DNA isolation round consisted of 96 cultures.

Insert size was estimated by agarose gel electrophoresis after restriction enzyme digestion of the pBlue-Script (SK-) vector with EcoRI and XhoI. Inserts with length above 500 bp were used for sequencing. Plasmid DNA from the selected samples were used for polymerase chain reaction (PCR) sequencing reactions using the ABI PRISM™ Dye Terminator Cycle Sequencing Ready Reaction Kit and the MJ Research PTC-200 DNA Engine™ thermal cycler. The T3 and T7 universal primers were used for sequencing from the 5' and 3'ends respectively. PCR program was according to the Dye Terminator manufacture's protocol (ABI PRISM). The ABI 373, 370A and 310 sequencers (Applied Bio-systems) were used. Sequences were edited manually to remove vector and non reliable sequences and submitted to the Blast homology search program using BlastX and BlastN (Altschul et al. *J. Mol. Biol.* 215, 403 - 410, 1990) provided by the National Centre for Biotechnological Information on the world wide web. The strawberry and lemon sequencing data received from the Blast search was transferred using a self designed macro program to a database made using the software Access (Microsoft). Sequences with a BlastX score above 80 were considered as having a significant homology (Pearson, *Curr*. *Opinion. Struct. Biol*, 1, 321 - 326, 1991) and they were used for data interpretation in the Access and Excel programmes (Microsoft). The cDNA clones: SLE27 (SEQ ID NO: 1A), CLF26 (SEQ ID NO: 2A), SLF96 (SEQ ID NO: 3A), SLG150 (SEQ ID NO: 4A), SLH51 (SEQ ID NO: 5A), SLB39 (SEQ ID NO: 6A), SLF193 (SEQ ID NO: 7A), SLF122 (SEQ ID NO: 8A), SLD194 (SEQ ID NO: 9A), SLF17 (SEQ ID NO: 10A), SLF138 (SEQ ID NO: 11A), SLG16 (SEQ ID NO: 12A), SLG144 (SEQ ID NO:13A) were selected for further analysis.

### Example 3: Characterisation of selected cDNAs

### 3.1 Sequencing the entire length cDNAs

Plasmid DNA from the selected cDNAs was used for PCR sequencing reactions using the ABI PRISM™ Dye Terminator Cycle Sequencing Ready Reaction Kit and the MJ Research PTC-200 DNA Engine™ thermal cycler. Each individual selected cDNA was sequenced completely on both strands. DNA and protein alignment 'contigs' were performed using the GeneWorks software (IntelliGenetics, Oxford).

### 3.2. RNA isolation from different tissues and organs of strawberry and lemon.

RNA isolation from both strawberry and lemon tissues was done as described for the preparation of total RNA for the construction of the cDNA libraries (Manning K. *Analytical Biochemistry* 195, 45-50 (1991)]. For both species green house grown plant material was used. From strawberry the different tissues/organs used for RNA isolations were; leaf, root, petiole, flower, green fruit, white fruit, turning fruit, red fruit, red fruit without seeds, seeds and overripe fruit. For lemon the different tissues/organs used were; root, leaf, flower bud, albedo, green fruit peel (1.2 x 2 cm), green fruit peel (2 x 3 cm), green fruit peel (3.5 x 6 cm), green fruit peel (6 x 8 cm), yellow fruit peel (2 weeks fruit detached).

### 3.3 Expression analysis using Northern blots

Ten µg of each RNA sample was denatured by glyoxal (1.5M) prior to electrophoresis on a 1.4% agarose gel in 15mM sodium phosphate buffer pH = 6.5 (without Ethidium bromide). Equal loading of RNA was verified by visualising equal amount of RNA from each sample on a 1% TAE buffer [1x : 0.04M Tris-acetate / 0.001M ethylenediaminetetra-acetate (EDTA)] agarose gel prior to loading. Blotting was done using Hybond N⁺membrane (Amersham) in 25 mM Sodium phosphate buffer ph = 6.5 overnight.

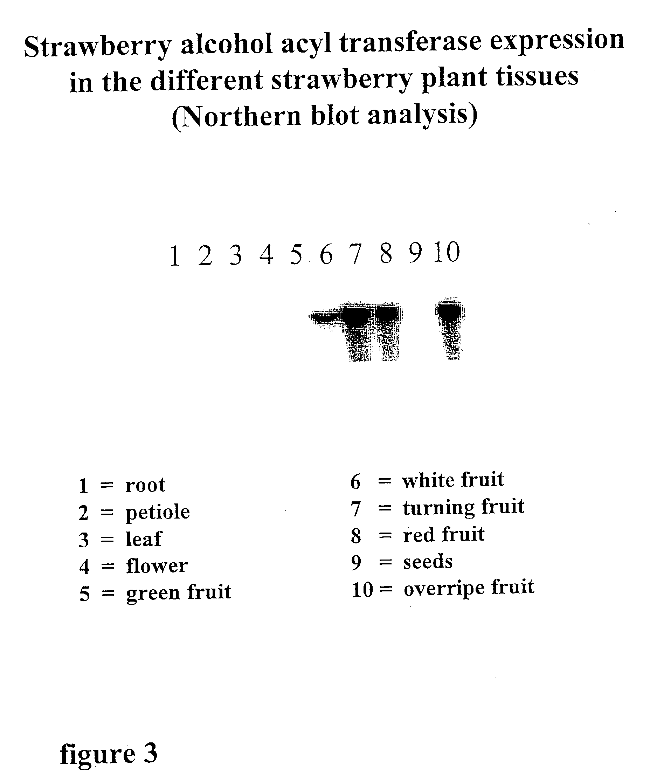

After fixation (2h 800c) blots were hybridised as described by Angenent *et al.* 1992 (Plant Cell 4, 983-993). The hybridisation probes were made by random labelling oligonucleotide priming (Feinberg and Vogelstein, *Anal. Biochem*. 137, 266-267, 1984) of the entire cDNAs. The hybridised membranes were autoradiographed at -70°C with intensifying screens. In Figure 3 the expression pattern of strawberry alcohol acyl transferase (SLE27, SEQ ID NO: 1A) is demonstrated by a Northern blot loaded with total RNA extracted from 10 different plant tissues and/or organs from strawberry. The expression pattern of strawberry alcohol acyl transferase is in perfect match with the accumulation of volatile esters in the strawberry plant. The gene SLE27, (SEQ ID NO: 1A) is expressed at it lowest level in the white fruit, gradually increasing as fruit develops reaching its highest levels in the turning stage and the red stage when the volatile ester compounds can be detected at their maximum. In other strawberry plant tissues/organs which do not produce those volatile fruity esters the expression of the gene can not be detected even when the film is exposed to a longer period after hybridisation.

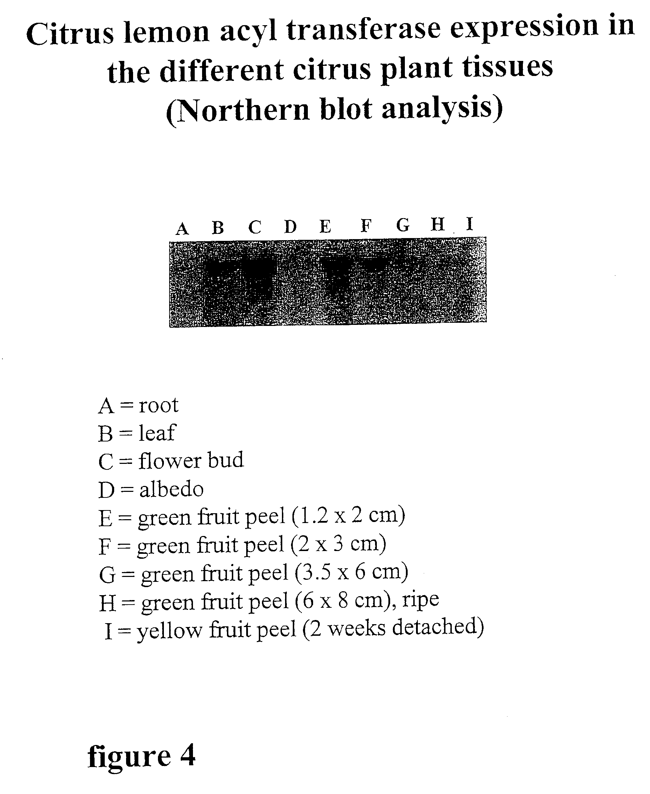

The expression of the lemon alcohol acyl transferase (CLF26 SEQ ID NO:2A) seems to be correlated with the accumulation of volatile monoterpene compounds in the lemon tree tissues/organs (Figure 4). These compounds can be converted by this enzyme to their ester derivatives as a result of alcohol acyl transferase enzyme activity. The gene is more highly expressed in the flower bud. Analysis by Gas chromatograph (GCMS) shows high concentrations of terpene compounds produced in the flower bud such as limonene. Expression analysis of montoterpene cyclases (genes directly responsible for the formation of monoterpenes) isolated from lemon thus shows a similar pattern of expression to the lemon alcohol acyl transferase (CLF26, SEQ ID NO:2A). Expression of the gene was also strongly detected in the early stages of fruit development and in the flower bud whilst no expression could be detected in the root and albedo tissues. This data provides evidence for a link between the presence of terpene compounds mainly monoterpenes and the lemon alcohol acyl transferase (CLF26, SEQ ID NO:2A).

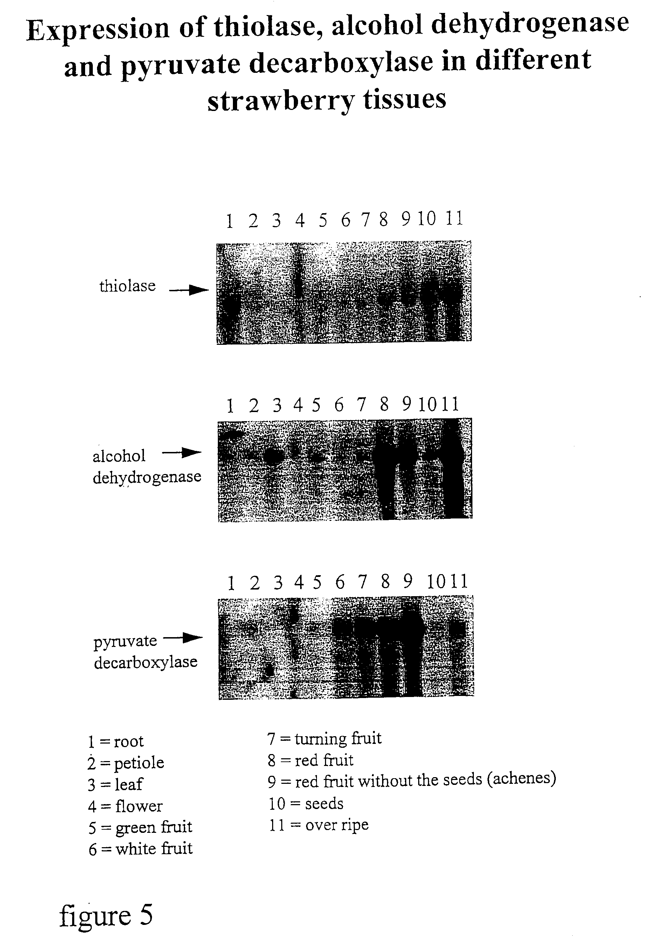

The expression of other three genes part of the metabolic pathway described in this invention for the formation of volatile ester compounds, in particular aliphatic esters in fruits is shown in Figure 5. Thiolase (SLG150, SEQ ID NO: 4A) alcohol dehydrogenase (SLB39, SEQ ID NO: 6A) and pyruvate decarboxylase (SLH51, SEQ ID NO: 5A) gene expression was monitored at different organs or tissues of the strawberry plant. The data shown provides evidence that thiolase (SLG150, SEQ ID NO: 4A), alcohol dehydrogenase (SLB39, SEQ ID NO: 6A) and pyruvate decarboxylase (SLH51, SEQ ID NO: 5A) are involved in ripening processes such as the production of strawberry flavour and aroma compounds. Clones SLF193 (SEQ ID NO: 7A), SLF122 (SEQ ID NO: 8A), SLD194 (SEQ ID NO: 9A), SLF17 (SEQ ID NO: 10A), SLF138 (SEQ ID NO: 11A), SLG16 (SEQ ID NO: 12A), SLG144 (SEQ ID NO:13A) and SLF96 (SEQ ID NO: 3A) were also tested for the expression and they were all appearing to have upregulated expression in the ripening fruit (data not shown).

### 3.4 Co-ordinate expression of genes in the biosynthetic pathway leading to aromatic and aliphatic ester formation

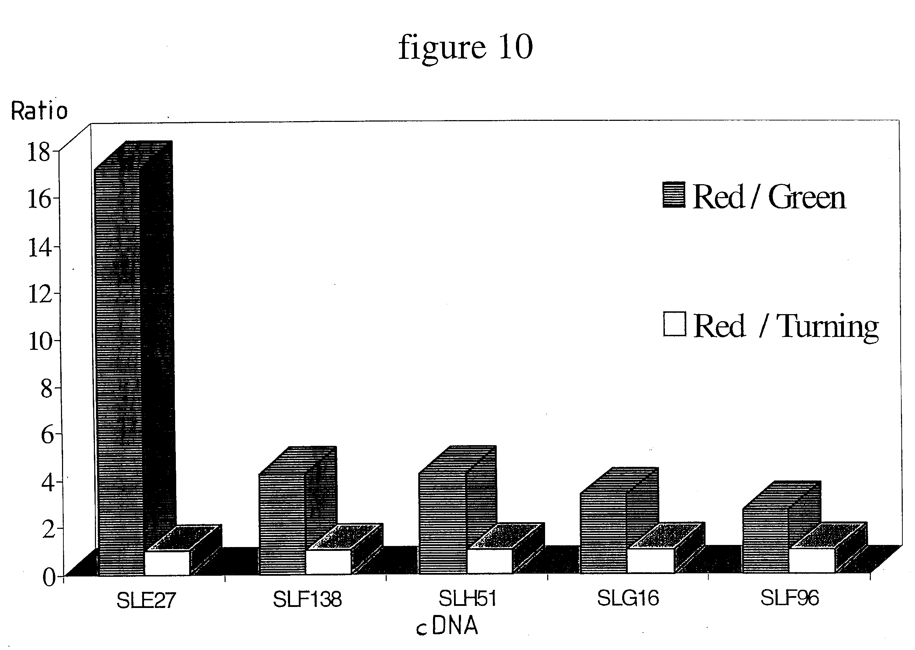

The co-ordinate expression pattern of genes of the biosynthetic pathway leading to aromatic and aliphatic ester formation was simultaneously monitored using arrays of cDNAs[Ermolaeva *et al.*, 1998, *Nature Genetics*, Vol, (20), 19-23]. The method allows one to simultaneous quantitatively and statistically analyse the expression profiles of genes pertaining to a particular metabolic pathway, such as pathway leading to aliphatic and aromatic ester formation, flavour, fragrance, aroma and scent. The experimental and hybridisation conditions followed were according to Schena *et al*., (PNAS, 93: 10614 - 10619). The expression levels derived from four images were statistically analysed with an analysis of variance modelling the factors dye, origin, and spot levels on a logarithmic scale giving the significant ratios of expression between the spots of origin (eg. green versus red). As way of illustration the expression profiles of SEQ ID 1A, SEQ ID 3A, SEQ ID 5A, SEQ ID 11A, SEQ ID 12A, analysed in the green versus red and turning versus red stages of fruit development are outlined in Figure 10. The co-ordinated expression pattern of the genes during strawberry fruit development is in perfect match with the accumulation of volatile ester compounds aromatic and aliphatic during strawberry fruit development. In addition alcohol acyl transferase enzyme activity was detected by Perez *et al*., (J. Agric. Food Chem. 1996, 44, 3286-3290) to be elevated dramatically between the white and the turning stages of strawberry fruit development. This correlates well with a dramatic increase in expression of the genes of the biosynthetic pathway as shown in Figure 10. A supporting detail is the fact that volatile ester compounds can be detected in their maximum when the fruit is completely red and this is probably a result of gene activation at earlier stages between the white and turning stage of development.

### 3.5 DNA isolation from strawberry leaf tissue.

Genomic DNA was isolated from green house grown strawberry plants (cv. *Elsanta*). Young folded leaves kept 2 days in water in the dark were used for the isolation. 1 gram of leaf material was ground in liquid nitrogen and immediately added to 7 ml DNA extraction buffer (100 mM Tris-Cl, 1.4 M NaCl, 20 mM EDTA, 2% CTAB, 0.2% β- mercaptoethanol). After incubation for 30 min. at 600C one volume of chloroform:isoamyl alcohol (24:1) was added to the tubes. The samples were then centrifuged for 10 min. at 4000 rpm and the aqueous phases were transferred to 4.5 ml isopropanol and incubated for 30 min. at -20°C. Nucleic acids were spun down at 4000 rpm for 12 min. washed with 70% ethanol and air dried. Pellets were dissolved in 3 ml TE and 0.25 volumes of NaCl (5M) and 0.1 volume absolute ethanol were added. Samples were centrifuged for 10 min at 4000 rpm and supernatants were transferred to 4.5 ml isopropanol for precipitation. Pellets were washed with 70% ethanol dried and dissolved in 0.5 ml TE buffer (10mM Tris-Cl, 1mM EDTA pH = 8.0). RNA was selectively precipitated by the addition of 0.6 volumes of 8M lithium chloride. The RNA was pelleted by centrifugation 10 min. 4000 rpm and the DNA was precipitated again with the addition of 0.6 volume isopropanol. Pellets were washed with 70 % ethanol, dried and dissolved in 0.5 ml sterile water. The amount of DNA was determined by comparing to known amounts of phage lambda DNA.

### 3.6 southern blot analysis for strawberry alcohol acyl transferase (SLE27, SEQ ID NO:1A)

Aliquots of 2 µg strawberry genomic DNA were digested for 16h with the restriction enzymes: HindIII, XbaI, XhoI and EcoRI and separated on a 0.7% TAE (0.04M Tris-acetate pH = 8.0 and 1mM EDTA pH = 8.0) agarose gel. The DNA was then denatured in 0.4M NaOH for 30 min. and then transferred to a Hybond N+ membrane in 0.4M NaOH. Blot hybridisation and probe preparation was done as described previously for Northern blots. After hybridisation the blot was washed first under low stringency conditions (52°C with 2 times half an hour 2xSSC/0.1% SDS) and after observation of the results by autoradiography of the film they were washed further under stringent conditions (65°C with 2 times half an hour 0.1xSSC/0.1% SDS) and autoradiographed again.

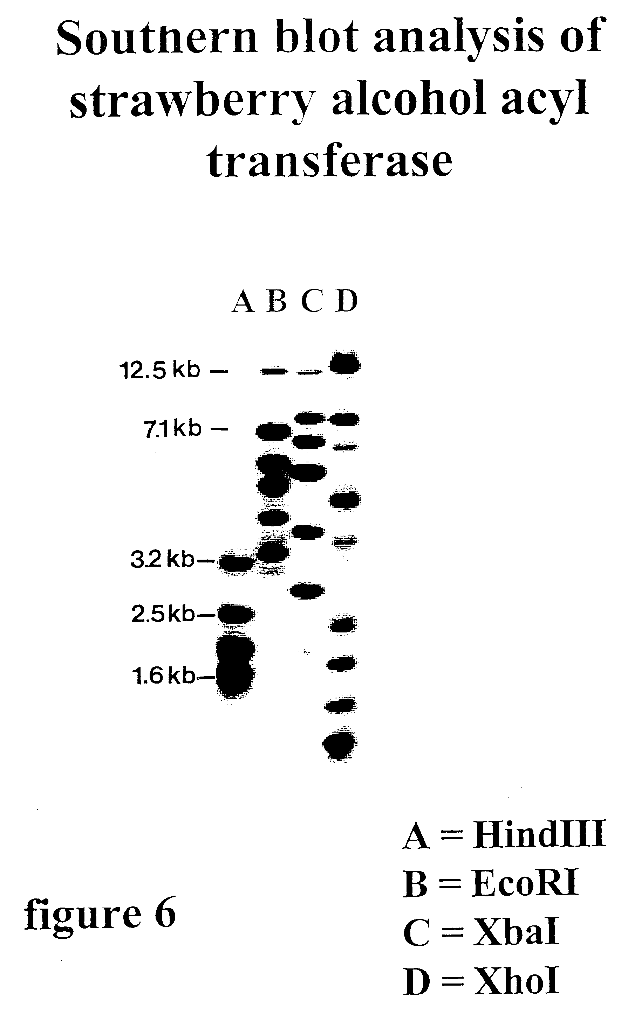

In order to investigate whether the different esters formed in the fruit are a result of an activity of one single enzyme or a multigene family, a Southern blot was performed using strawberry genomic DNA and the full length SLE27 cDNA (SEQ ID NO:1A) which served as a probe (Figure 6). Since the cultivar used for the investigation is an octaploid the interpretation of the results is more difficult than that for a diploid plant. However, the results obtained suggest the possible existence of another homolog of strawberry alcohol acyl transferase. This additional copy might arise from one of the parental lines with a similar function to the one isolated, or may represent another homolog with different function or substrate specificity to SLE27 (SEQ ID NO: 1A).

### Example 4

### Strawberry alcohol acyl transferase (SLE27, SEQ ID No:1B) protein analysis

### 4.1 Cloning to the expression vector pRSET B

The *E.coli* expression vector pRSETB (Invitrogen), was used for expression studies of strawberry alcohol acyl transferase (SLE27, SEQ ID NO: 1A). This pRSETB vector contains the T7 promoter which can be induced by isopropyl-beta-D-thiogalactopyranoside (IPTG) and therefore by inserting the desired gene downstream of this promoter, the gene can be expressed in *E. coli*. In addition, DNA inserts were positioned downstream and in frame with a sequence that encodes an N-terminal fusion peptide. This sequence includes (in 5' to 3' order from the N-terminal to C-terminal), an ATG translation initiation codon, a series of six Histidine residues that function as a metal binding domain in the translated protein, the Anti-Xpress epitope, and the enterokinase cleavage recognition sequence.

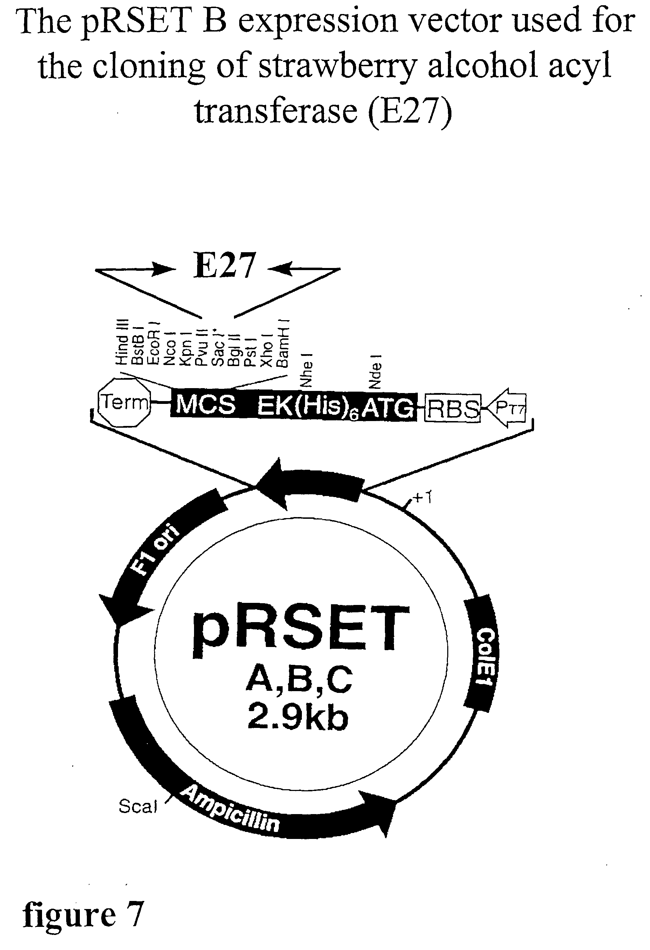

The original pRSETB was primarily used for the insertion of the gene encoding the Green Fluorescent Protein (GFP). The GFP gene was fused to the pRSETB vector using the BamHI and HindIII restriction sites located at the multiple cloning site (MCS) as can be seen in Figure 7. This construct for the expression of GFP served as control for the experiments together with the empty pRSETB vector.

Cloning the GFP gene to the pRSETB vector inserted an additional SalI restriction site at the 3' of the GFP gene and together with the BamHI site located at the 5' of the GFP gene served as sites for the cloning of the strawberry alcohol acyl transferase gene (SLE27, SEQ ID NO:1A). The BamHI and SalI sites were introduced to the 5' and 3' respectively of the strawberry alcohol acyl transferase coding sequence by the use of PCR. The 452 amino acid open reading frame of the SLE27 (SEQ ID NO:1A) clone was amplified with the pfu DNA polymerase (Stratagene) and primers AAP165 (5'-CGGATCCGGAGAAAATTGAGGTCAG) and AAP166 (5'-CGTCGACCATTGCACGAGCCACATAATC) according to the manufacturers instructions. The PCR product was cloned into PCR-script vector (Stratagene), cut out with BamHI and SalI and further inserted (as a translation fusion) into the corresponding restriction sites in the pRSETB vector.

### 4.2 Bacterial expression and partial purification using the His tag columns.

The pRSET B vector harbouring the strawberry alcohol acyl transferase (SLE27, SEQ ID NO: 1A) was used to transform *E. coli* strain BL21 Gold DE3 (Stratagene) as described by the manufacturer. For bacterial expression typically 1 ml of overnight liquid culture grown at 37°C in Luria Broth (LB) medium (10g/l .tryptone, 5 g/liter yeast extract, 5 g/liter NaCl) supplemented with 100 mg/liter Ampicillin was diluted 50 times in the same medium and grown until the OD₆₀₀ reached 0.4 (usually at 37°C except when different temperatures for growth during induction were examined). At this stage Isopropylthio-beta-D-galactoside (IPTG) was added to a final concentration of 1mM in order to induce expression . After one and a half hour of induction the cells were harvested by centrifugation at 4000 x g for 15 min. Pellet and a sample from the supernatant were kept for SDS gel analysis. The cells were further processed as described by the Ni-NTA Spin Columns manufacturers (QIAGEN) for protein purification under native conditions.

### 4.3. Recombinant protein detection using Western blot

The supernatant (S), pellet (P) and elute from the Ni-NTA columns (E) samples were diluted with 2x SDS-sample buffer [(standard Laemmli, pH 6.8 Tris /glycine/SDS/glycerol + 50mM Dithiothreitol(DTT)]. The samples were heated for 5-10 min at 95°C, followed by a short spin of 30 seconds, full speed. From the samples 10µl was loaded on 10% SDS-gel (mini-gel, 1mm, 7x9cm). The gels were run with standard buffer (25mM Tris 192mM, glycine 0.1% SDS, pH 8.3) at 100-200V, 50-30mA, ca 3hr with 16°C water cooling. After complete run, gels were used for Coomassie Brilliant Blue (CBB) stain or Western blot. For CBB staining, the gel was fixed during 1hour in 40% Methanol, 10% Acetic Acid in water. Then incubated overnight with CBB staining solution (0.05% CBB-250 in 40% Methanol, 10% Acetic acid in water) and destained by repeated washing in 25% Methanol, 10% Acetic acid in water. Western blot was onto Polyvinyl di fluoride (PVDF), (Immobilon P, Amersham) pre-wetted with Methanol. Transfer buffer was Towbin buffer plus 0.1% SDS (25mM Tris, 192mM Glycine, 20% 0.1% SDS, pH 8.3). Transfer was submarine (Novex blot unit, plate electrodes, 3cm distance) at 200mA overnight plus 2hr. at 400mA, cooled in ice-water with cooling block. Transfer was checked by using pre-stained markers as a reference.

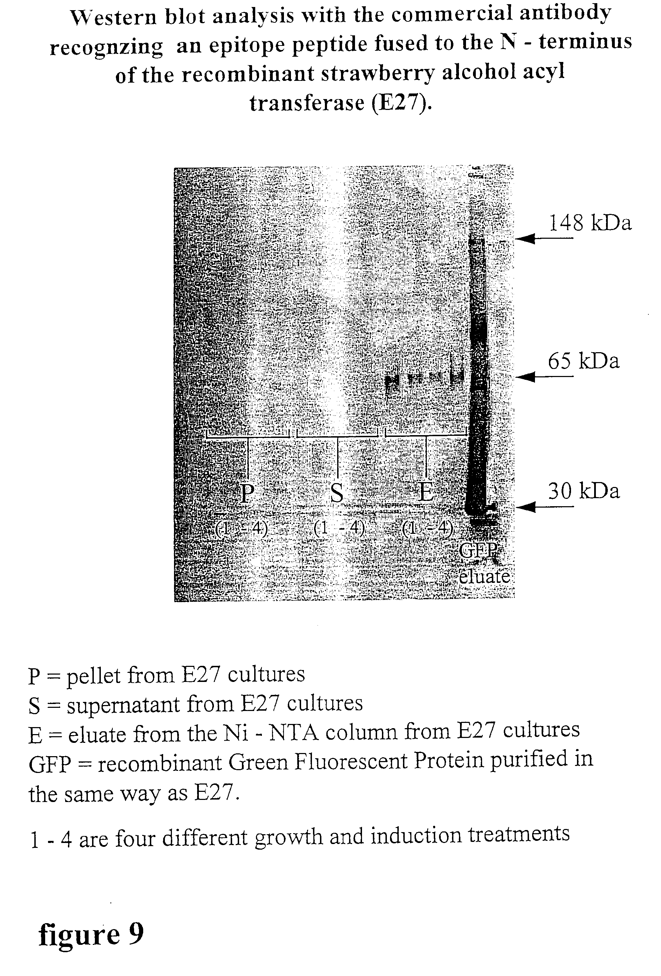

After transfer the blot was washed in Tris buffer saline TBS (20mM Tris-HCl pH7.5 / 150mM NaCl)- Tween20 (0.02%), blocked with 2% fat free milk in TBS-Tween20, then washed with TBS-Tween 20. Then first Antibody was anti-Xpress (Invitrogen) 1/1000 in 2% BSA TBS-Tween for 3hr. incubate at room temprature, then 4x washed with TBS-Tween 20. Second Antibody is GAR-AP (Boehringer) 1/3000 in 2% BSA TBS-Tw (TBS + 0.05% Tween - 20) for 1-2hr. incubation at room temprature, then 4x washed with TBS-Tween 20. Final wash was in water, then in Alkaline phosphatase-buffer (100mM Tris pH9.2, 1mM MgCl₂). The staining reaction was with nitroblue tetrazolium/5-bromo-4-chloro-3-indolphosphate/alkaline phosphatase-buffer (45µl nitroblue tetrazolium 30mg/ml in 70% dimethylformamide, 33µl 5-bromo-4-chloro-3-indol phosphate 15mg/ml in dimethylformamide in 10ml of alkaline phosphatase buffer). The staining reaction was stopped by rinsing with water. Western blotting was used in order to determine the amount of the recombinant SLE27 (SEQ ID NO: 1B) protein in the elute after the Ni-NTA column purification compared to the pellet and supernatant before loading on the column. Figure 9 shows a western analysis of the pellet supernatant and elute after partial purification on the Ni-NTA column. 4 samples were loaded from each pellet, supernatant and elute originating from 4 different growth and induction of the *E. coli* cells. A commercial antibody recognising an epitope peptide fused to the N-terminus of the SLE27 recombinant protein showed relatively high expression of SLE27 (SEQ ID NO:1B) in the elute from the Ni-NTA column. The estimated size of the partial purified recombinant SLE27 (SEQ ID NO:1B)protein seems to be approximately 60kDa (minus the fusion peptide at the N-terminus. As a control for the experiment served the Green Fluorescent Protein (GFP) partially purified in the same method as done for SLE27 (SEQ ID NO:1B). No band was detectable at the level of the SLE27 (SEQ ID NO:1B) recombinant protein in the GFP lane.

### 4.4 Method for establishing alcohol acyl transferase activity of the strawberry SLE27 (SEQ ID NO:1B)-encoded protein.

The alcohol acyl transferase activity of the strawberry cDNA SLE27 (SEQ ID NO:1A) encoded protein was established by analysing the volatile esters produced from acyl-CoA and different alcohols. In a 1.8 ml glass vial with a small magnetic stirring bar, 325 µl of buffer (50 mM Tris/HCl pH 8.0, containing 1 mM dithiothreitol) , 25 µl of protein extract and 25 µl of acyl-CoA (4 mM in buffer) were subsequently pipetted. The enzyme reaction was started by the addition of 25 µl of alcohol solution (160 mM in buffer). The vial was quickly closed with a snap cap and placed in a waterbath (at 35°C) with a stirring device. After 15 min enzyme reaction with continuous stirring, the vial was opened, 0.29 g of solid CaCl₂ was added (final concentration 5M) and the vial was quickly closed again with an open top crimp cap. The high CaCl₂ concentration was used to stop further enzymatic reactions and to drive the volatiles into the headspace. After addition of CaCl₂, the mixture was incubated again at 35°C while stirring. The volatile components released into the headspace were trapped using solid phase micro-extraction (SPME; Supelco Inc., Bellefonte, PA, USA), exposing the fused silica fiber coated with 100 1m polydimethylsiloxane (PDMS; Supelco Inc.) to the headspace for 15 min. The presence of esters in the alcohol stock solutions and the spontaneous formation of esters were checked by adding 25 µl of buffer instead of protein extract into the reaction vial.

The volatiles trapped by SPME were separated and identified by gas chromatography (GC 8000 Series; Interscience B.V., Breda, The Netherlands) coupled to mass spectrometry (MD-800, Fisons Instruments, Interscience) as described by *Verhoeven et al*. (1997) [*Chromatographia* Vol. 46, No 1,2]. Compounds were thermally desorbed (2500C) from the SPME-fiber for 1 min. Separation of compounds was on a capillary HP-5 column (50 m x 0.32 mm, film thickness 1.05 µm; Hewlett Packard) with He (37 kPa) as a carrier gas. The oven temperature was programmed at 2 min at 80°C, then an increase of 8°C min⁻¹ to 250°C, and finally 5 min at 250°C. Mass spectra of eluting compounds were generated at 70ev each second. The MD 800 Masslab software (Interscience) was used to record mass spectra and to calculate peak areas. Volatile compounds were identified by screening the NIST library for comparable mass spectra and by comparison with authentic reference compounds.

### 4.5.Substrates used by the strawberry alcohol acyl transferase-encoded enzyme (SLE 27: SEQ ID NO: 1B)

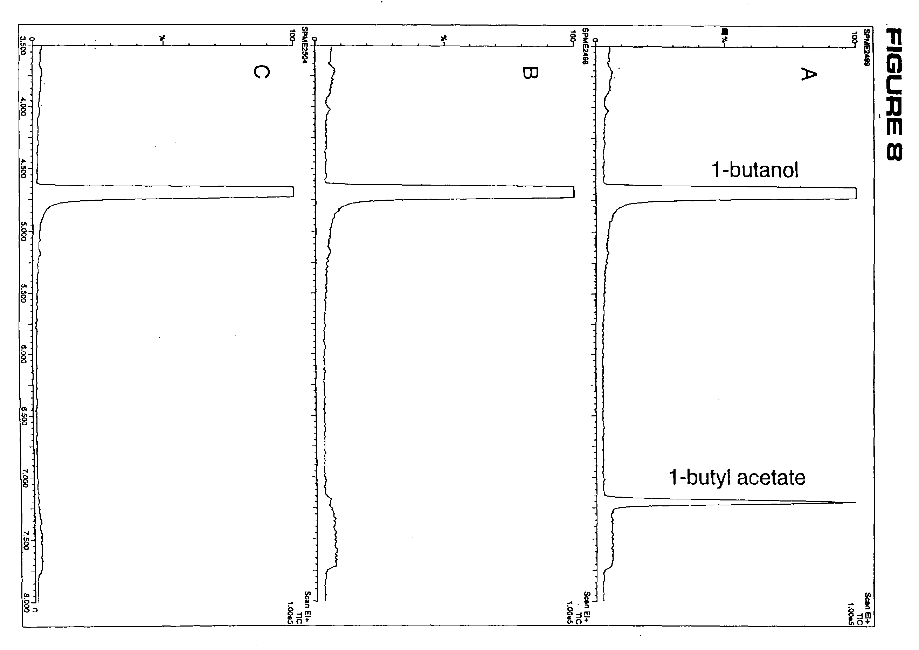

The histidine-released protein elute from *E. coli* expressing the strawberry alcohol acyl transferase gene (SLE27, SEQ ID NO:1A) was incubated with both acetyl-CoA and 1-butanol. The chromatograms obtained by SPME of volatiles released into the headspace indicated a clear peak of acetic acid 1- butyl ester (Figure 8A). This ester was not detected when the enzyme was inactivated by freezing and subsequent thawing of the elute (Fig 8B). Moreover, when the SLE27 (SEQ ID NO:1B)-encoded protein elute in the assay was replaced by an equal amount of histidine-released elute from lysate of *E. coli* expressing only the histidine-tag (i.e. the same vector without the SLE27 (SEQ ID NO:1A gene), the ester could not be detected either (Fig 8C). To investigate which substrates can be used by the SLE27(SEQ ID NO:1B)-encoded protein to produce volatile esters with acetyl-CoA, the alcohol species in the assay was varied. Table 1 shows all substrates tested and the species that can be used by the SLE27-encoded enzyme (SEQ ID NO:1B). Clearly, this enzyme can use both aliphatic and aromatic alcohols.

**Table 1.** An illustration of some of the different ester products formed by the SEQ ID: 1A, the strawberry alcohol acyl transferase (SLE27) as detected by GCMS analysis, after heterologous expression in *E.coli.*

**Table 1**

| **Acyl CoAs** | **Alcohol** | **Ester formed** |
|---|---|---|
| acetyl CoA | 1 - propanol | 1 - propyl acetate |
| acetyl CoA | 1 - butanol | 1 - butyl acetate |
| acetyl CoA | 2 - butanol | 2 - butyl acetate |
| acetyl CoA | furfuryl alcohol | furfuryl alcohol acetate |
| acetyl CoA | iso propanol | isopropylacetate |
| acetyl CoA | 1 - hexanol | hexyl acetate |
| acetyl CoA | 1 - octanol | n-octyl acetate |
| acetyl CoA | phenylethyl alcohol | acetic acid phenylethyl ester |
| acetyl CoA | benzyl alcohol | acetic acid phenylmethyl ester |
| butyl CoA | 1 - propanol | butanoic acid propyl ester |
| hexyl CoA | 1 - propanol | hexanoic acid propyl ester |

Sequence listing
INFORMATION FOR SEQ ID NO: 1A (SLE27)

SEQUENCE CHARACTERISTICS:
LENGTH: 1638
TYPE: cDNA
STRANDNESS: Single
TOPOLOGY: Linear
OTHER INFORMATION: STRAWBERRY ALCOHOL ACYL TRANSFERASE
SEQUENCE DISCRIPTION FOR SEQ ID NO: 1A
ACCTACTTTGCCAAAATGGAGAAAATTGAGGTCAGTATAAATTCCAAACA-
CACCATCAAACCATCAACTTCCTCTACACCACTTCAGCCTTACAAGCTTACC-
CTCCTGGACCAGCTCACTCCTCCGGCGTATGTCCCCATCGTGTTCTTCTACC-
CCATTACTGACCATGACTTCAATCTTCCTCAAACCCTAGCTGACTTAAGA-
CAAGCCCTTTCGGAGACTCTCACTTTGTACTATCCACTCTCTGGAAGGGT-
CAAAAACAACCTATACATCGATGATTTTGAAGAAGGTGTCCCATACCTT-
GAGGCTCGAGTGAATTGTGACATGACTGATTTTCTAAGGCTTCGGAAA-
ATCGAGTGCCTTAATGAGTTTGTTCCAATAAAACCATTTAGTATGGAAGCA-
ATATCTGATGAGCGTTACCCCTTGCTTGGAGTTCAAGTCAACGTTTTCGAT-
TCTGGAATAGCAATCGGTGTCTCCGTCTCTCACAAGCTCATCGATGGAG-
GAACGGCAGACTGTTTTCTCAAGTCCTGGGGTGCTGTTTTTCGAGGGT-
GTCGTGAAAATATCATACATCCTAGTCTCTCTGAAGCAGCATTGCTTTTCC-
CACCGAGAGATGACTTGCCTGAAAAGTATGTCGATCAGATGGAAGCGTTAT-
GGTTTGCCGGAAAAAAAGTTGCTACAAGGAGATTTGTATTTGGTGTGAAAG-
CCATATCTTCAATTCAAGATGAAGCGAAGAGCGAGTCCGTGCCCAAGCCAT-
CACGAGTTCATGCCGTCACTGGTTTTCTCTGGAAACATCTAATCGCTGCTTC-
TCGGGCACTAACATCAGGTACTACTTCAACAAGACTTTCTATAGCGGCC-

CAGGCAGTGAACTTAAGAACACGGATGAACATGGAGACAGTGTTGGATA-
ATGCCACTGGAAACTTGTTCTGGTGGGCACAGGCCATACTAGAGCTAAGT-
CATACAACACCAGAGATCAGTGATCTTAAGCTGTGTGACTTGGTTAACTTG-
CTCAATGGATCTGTCAAACAATGTAACGGTGATTACTTTGAGACTTTCAAG-
GGTAAAGAGGGATATGGAAGAATGTGCGAGTATCTAGATTTTCAGAGGAC-
TATGAGTTCTATGGAACCAGCACCGGATATTTATTTATTCTCGAGCTGGAC-
TAATTTTTTCAACCCACTTGATTTTGGATGGGGGAGGACATCATGGATTG-
GAGTTGCAGGAAAAATTGAATCTGCAAGTTGCAAGTTCATAATATTAGTTC-
CAACACAATGCGGTTCTGGAATTGAAGCGTGGGTGAATCTAGAAGAAGA-
GAAAATGGCTATGCTAGAACAAGATCCCCATTTTCTAGCGTTAGCATCTC-
CAAAGACCTTAATTTAAAGATATTGATTAAGAAAGATTATGTGGCTCGTG-
CAATGTTTCGATTTTGCAGTGAATAAGGTTTAAATTAGTTCACCAGCCAAT-
CAATAAAATGCAAGTATGATAGACTTTGTCTACGTATGTTATCCGAATGT-
GTTTCCATATGCTTGTAACCAATATAGCTCTTTATTGTAACAAATGCTCTAT-
TAAGCTTCTAGCTATAAAGTTATTTATCTATTAAAAATAAAACTATGGAAGT-
TTTACCAAAAAAAAAAAAAA

INFORMATION FOR SEQ ID NO: 1B (SLE27)
SEQUENCE CHARACTERISTICS:
LENGTH: 452
TYPE: Peptide
STRANDNESS: Single
TOPOLOGY: Linear
OTHER INFORMATION: STRAWBERRY ALCOHOL ACYL TRANSFERASE

SEQUENCE DISCRIPTION FOR SEQ ID NO: 1B

MEKIEVSINSKHTIKPSTSSTPLQPYKLTLLDQLTPPAYVPIVFFYPITDHDFNL-

PQTLADLRQALSETLTLYYPLSGRVKNNLYIDDFEEGVPYLEARVNCDMTDFL-

RLRKIECLNEFVPIKPFSMEAISDERYPLLGVQVNVFDSGIAIGVSVSHKLIDGG-

TADCFLKSWGAVFRGCRENIIHPSLSEAALLFPPRDDLPEKYVDQMEALWFAG-

KKVATRRFVFGVKAISSIQDEAKSESVPKPSRVHAVTGFLWKHLIAASRALTS-

GTTSTRLSIAAQAVNLRTRMNMETVLDNATGNLFWWAQAILELSHTTPEIS-

DLKLCDLVNLLNGSVKQCNGDYFETFKGKEGYGRMCEYLDFQRTMSS-

MEPAPDIYLFSSWTNFFNPLDFGWGRTSWIGVAGKIESASCKFIILVPTQCGS-

GIEAWVNLEEEKMAMLEQDPHFLALASPKTLI

INFORMATION FOR SEQ ID NO: 2A (CLF26)

SEQUENCE CHARACTERISTICS:

LENGTH: 1613

TYPE: cDNA

STRANDNESS: Single

TOPOLOGY: Linear

OTHER INFORMATION: CITRUS LIMON ALCOHOL ACYL TRANSFERASE

SEQUENCE DISCRIPTION FOR SEQ ID NO: 2A

CTTATTTAAAAGTTCATCAACAAATTGTTCTACCACTTACCATTTCTCATAG-

CTCTGCAAGTTCGGATTTGACTCTTTCTCTTTTCCTCATTCCGGCCGGTGTT-

GATAGTTACATTTTGGCACAATGAAAATTCACGTTAAGGAGTCAACAATTA-

TACGCCCTGCTCAAGAAACACCCAAGCATCGCCTACAAATATCCGACCTA-

GACATGATTGTGCCATCCAATTACGTTCCCAGTGTGTATTTCTATCGGCG-

GTCCAGTGACTGCACCGATTTTTTGAAGTTGGTTTGCTGAAGAAGGCTCT-

GAGCGAAGTTCTTGTGCCGTTTTACCCCGTTGCCGGAAGGTTGCAGAAG-

GATGAAAATCGCAAAATTGAGATTCTATGTAACGGAGAGGGAGTTTTGTT-

TCTGGAGGCCGAAACAAGTTGTGGTATTGATGATTTCGGTGACTTCTCA-

CAAGGCTCGAAACTCCTGACGCTTGTTCCAACTGTTGGTGATACAAAGGA-

TATATCCTCCCATCCACTCTTGATGGCACAGGTAACTTATTTCAAATGTG-

GAGGCGTTTGTGTTGGAACTAGAGTGAATCATACACTGGTAGATGGAGCTT-

CAGCGTACCATATCATCAACTCATGGGCGGAGACGACGCGTGGCGTTCC-

TATTAGCACTCAACCATTCTATGATCGGACCATACTGAGTGTTGGGGTTC-

CAACTTCTCCCAAATTCCATCACATTGAATATGACCCGCCTCCTTCCAT-

GAACGCTCCTCCTACCCAAAATCCTGAAATCATTTCTACCGCAATCCTTAAC-
CTATCACTTGATCAAATCCACACCCTCAAAGAGAAATCTAAGACAGATCAT-
GAACCCAACGTCAAGTATAGTAGGATGGCGATCCTAGCAGCACATATCTGG-
CGTAGCATGTGTAAAGCGCGGATTATCTGATGATCAAGTTAGCAAGTTA-
CACTTTCCTACAGACGGACGACAGAGATTGAATCCACCACTCCCGCCTGGA-
TATTTTGGAAATGTAATTTTCACCACGTCGTTGACGGCTTCATCGGGTGA-
TATCCTAAGTGAACCATTGAATCATACTGTTGAAAGAATTCAAAAAGCAT-
TAAAGCGGATGGACGATGAGTATTTGAAATCAGCACTTGCTTACCTAAAG-
CAACAGCCTGATTTAAATGCTCTACGGAAAGGAGGCCACATTTACAAGTGC-
CCTAACCTCAATATCGTCAATTTGGCAAATATGCCAATGTATGTTGCGAATT-
TTGGATGGGGCCAGCCGATATTTGCGAGGATCGTTAACACATATTATGAAG-
GGATAGCACATATTTATCCAAGTCCGAGCAATGATGGGACCTTGTCAGTG-
GTTATAAACTCGGTAGCCGATCACATGCAGCTGTTCAAGAAGTTCTTT-
TACGAGATCTTTGATTAAGGTATGAAAGACCTAGGTATTTTATATTTTCTA-
GAAATGTCACTTTTTTTTTTTTTTTTTTTGGGGGCGCAAATGTTGTCTTAC-
TTGGAATTTTATATATTTTAATCCATGTTTTATGGAAGGCAGTGGTGTTG-
CAAAAAAAAAAAAAAAAAAAAAAAAAAAAAAAAAAAAAAAAAAAAAAAAA

INFORMATION FOR SEQ ID NO: 2B (CLF26)

SEQUENCE CHARACTERISTICS:

LENGTH: 434

TYPE: Peptide

STRANDNESS: Single

TOPOLOGY: Linear

OTHER INFORMATION: CITRUS LIMON ALCOHOL ACYL TRANSFERASE
SEQUENCE DISCRIPTION FOR SEQ ID NO: 2B
MKIHVKESTIIRPAQETPKHRLQISDLDMIVPSNYVPSVYFYRRSSDCTDF-
FEVGLLKKALSEVLVPFYPVAGRLQKDENRKIEILCNGEGVLFLEAETSCGIDD-
FGDFSQGSKLLTLVPTVGDTKDISSHPLLMAQVTYFKCGGVCVGTRVNHTL-
VDGASAYHIINSWAETTRGVPISTQPFYDRTILSVGVPTSPKFHHIEYDPPPSM-
NAPPTQNPEIISTAILNLSLDQIHTLKEKSKTDHEPNVKYSRMAILAAHIWRSM-
CKARGLSDDQVSKLHFPTDGRQRLNPPLPPGYFGNVIFTTSLTASSGDILSEPLN-
HTVERIQKALKRMDDEYLKSALAYLKQQPDLNALRKGGHIYKCPNLNIVN-
LANMPMYVANFGWGQPIFARIVNTYYEGIAHIYPSPSNDGTLSVVINSVADHM-
QLFKKFFYEIFD.

INFORMATION FOR SEQ ID NO: 3A (SLF96)

SEQUENCE CHARACTERISTICS:

LENGTH: 1586

TYPE: cDNA

STRANDNESS: Single

TOPOLOGY: Linear

OTHER INFORMATION: STRAWBERRY AMINOTRANSFERASE

SEQUENCES DISCRIPTION FOR SEQ ID NO:3A

AAACCGTCGGCGTCTGTAAATGCGTCGCCGCTCCGGAGAAGACAGAGTA-

CAAGACTCAGGTGAATCGCAATGCCAACATGGCCAAGCTTCAAGCCGGT-

TATCTTTTTCCAGAGATTGCGAGGAGGAATGCGCACTTGCAGAAGCAC-

CCTGATGCGAAGATAATTCCACTTGGAATTGGTGATACTACCGAGCCAAT-

TCCAGAATATATAACCTCTGCAATGGCAAAGAGAGCACTTGCCATGTCCAC-

CCTAGAGGGTTACAGTGGTTATGGACCTGAACAAGGTGAAAAGCCACTGA-

GAGTTGCAATTGCTAAAACGTTTTATGGCGACCTTGGCATAGAGGAAGAT-

GACATATTTGTTTCTGATGGGGCAAAATGTGACATATCCCGCCTTCAGGT-

TCTTTTTGGGGCGGATAAAACAATAGCAGTGCAAGATCCATCGTATCCGGC-

TTATGTAGACTCAAGTGTTATTATGGGCCAGACAGGACAGTATCAGAAATC-

TGTTCAGAAGTTTGGAAACATCGAGTACATGAGGTGTACTCCCGATAATG-

GATTTTTTCCTGATCTGTCCTCTACTAAGCGAACAGATATCATATTTTTCT-

GTTCACCAAACAATCCTACTGGTTCTGCTGCAACAAGGGAGCAACTGACA-

CAACTTGTAAAGTTTGCCAAGGATAATGGTTCAATCATAGTCTATGATTCT-

GCATATGCCATGTATATGTCAGATGATAATCCACGCTCCATCTTTGAAATCC-
CTGGAGCTAAAGATGTTGCACTTGAGACATCATCATTTAGTAAGTATGCCG-
GATTCACTGGAGTTCGTTTGGGGTGGACTGTGGTTCCAAAGCAGTTGCAG-
TATTCAGATGGTTTTCAAGTTGCCAAGGATTTCAACCGCATTGTTTGTACTT-
GCTTCAATGGTGCATCCACTATTATCCAAGCTGGTGGTCTGGCTTGCCTT-
CAACCAAAGGGTGTTAAGGCTATGCACGGTGTGATAAATTTCTACAAA-
GAAAATACTAAGATCATAATGGAGACGTTTAACTCTCTTGGCTTTAACGTG-
TATGGAGGGACAAACGCTCCATATGTGTGGGTCCACTTCCCTGGACAAAGC-
TCCTGGGATGTGTTTGCTGAGATCCTTGAGAAGACTCATGTGGTAACCA-
CACCTGGAAGTGGCTTTGGACCTGGTGGTGAAGGTTTCATCAGGGTAAGTG-
CCTTTGGACACAGGAAAAATATATTAGAAGCATGTAAAAGATTCAAGCAAT-
TATACAAGTGAGGACTGCGGATCTGAATTGTAGACCAGTTTCTACTGCATG-
CTAGTTGAACCTATTTGCCTCCCATTTCCGTTCTATGCTAAATATTTTAG-
CACGTTCCAATTCCGTATTCAGTTTGTCGGCTTTAGTTTATGAATTATGGA-
GATTTTAGCTATTGTAAAAATGATTCGATCAGCCTTGTTTTCATGTGTTA-
CACTTAATTGTTGTAACATTTGTGAGGATCAGAAGCTTTGATTCTGTTTGC-
TAGAATAGTATAATTTTACCTAAATAAAGTGGTTGATCTTTCTTGGCCTG-
CAAAAAAAAAAAAAAAAAAAAA

INFORMATION FOR SEQ ID NO: 3B (SLF96)

SEQUENCE CHARACTERISTICS:

LENGTH: 397

TYPE: Peptide

STRANDNESS: Single

TOPOLOGY: Linear

OTHER INFORMATION: STRAWBERRY AMINOTRANSFERASE

SEQUENCES DISCRIPTION FOR SEQ ID NO:3B

MAKLQAGYLFPEIARRRNAHLQKHPDAKIIPLGIGDTTEPIPEYITSAMAKRA-

LAMSTLEGYSGYGPEQGEKPLRVAIAKTFYGDLGIEEDDIFVSDGAKCDISR-

LQVLFGADKTIAVQDPSYPAYVDSSVIMGQTGQYQKSVQKFGNIEYMRCTPD-

NGFFPDLSSTKRTDIIFFCSPNNPTGSAATREQLTQLVKFAKDNGSIIVYDSAYA-

MYMSDDNPRSIFEIPGAKDVALETSSFSKYAGFTGVRLGWTVVPKQLQYSDG-

FQVAKDFNRIVCTCFNGASTIIQAGGLACLQPKGVKAMHGVINFYKENTKI-

IMETFNSLGFNVYGGTNAPYVWVHFPGQSSWDVFAEILEKTHVVTTPGSGFGP-

GGEGFIRVSAFGHRKNILEACKRFKQLYK

INFORMATION FOR SEQ ID NO: 4A (SLG150)

SEQUENCE CHARACTERISTICS:

LENGTH: 1775

TYPE: cDNA

STRANDNESS: Single

TOPOLOGY: Linear

OTHER INFORMATION: STRAWBERRY THIOLASE

SEQUENCE DISCRIPTION FOR SEQ ID NO: 4A

CGCTCCTTTGATTTCCTTGTTTCAATTATCAAGAGTATGGAGAAAGCGAT-

CAACAGGCAGAAGGTTCTCCTCGACCATCTCCGACCTTCTTCTTCTTC-

CGACGACTCTTCTCTCTCCGCGTCGGTATGTGCGGCTGGGGATAGCGCTG-

CGTATGCTAGGAATCATGTCTTTGGGGACGATGTCGTCATCGTTGCAGCTT-

TTCGCACTCCACTCTGCAAGGCTAAGCGTGGCGGCTTCAAGTATACTTATG-

CTGATGATCTCCTCGCACCTGTCCTCAAGGCCGTGGTTGAGAAAACCAATC-

TCAATCCCAAGGAAGTCGGGGATATTGTTGTCGGTACCGTCTTGGCCCCAG-

GATCTCAGAGAGCTAGCGAATGCAGGATGGCTGCTTTCTATGCTGGCTTCC-

CTGAGACTGTGCCGGTTAGAACTGTGAACAGACAATGTTCGTCTGGCCTC-

CAAGCAGTTGCTGATGTTGCTGCTGCCATTAGAGCAGGGTTTTATGATATT-

GGCATTGGTGCTGGTTTGGAATCCATGACTGCAAACCCAATGGCATGG-

GAAGGGGATGTTAATCCTAAAGTAAAGATCTTTGAACAAGCCCAGAATTGC-

CTTCTTCCTATGGGAGTCACCTCAGAAAATGTTGCTCATCGTTTTGGTGTTT-

CAAGACAGGAGCAAGATCAGGCTGCAGTTGACTCTCATAGAAAGGCAGCT-

GCTGCTGCTGCTGCTGGTAGATTTAAAGATGAAATCATCCCTGTGGCAAC-

CAAGATTGTTGATCCAAAATCTGGTGATGAGAAACCTGTTACAATCTCT-
GTTGATGATGGGATTCGAAACACAACATTGGCGGACCTAGCAAAGCT-
GAAGCCTGTGTTTAAGAAAGATGGGACCACCACTGCTGGTAATTCTAGT-
CAAGTTAGTGATGGTGCTGGAGCTGTTCTCTTGATGAAGAGAAGTGTTGC-
CGACCAAAAAGGATTGCCGATTCTTGGTGTATTCAGGAATTTTGTTGCT-
GTTGGTGTGGATCCTGCCATCATGGGTGTTGGCCCAGCTGCTGCAATTC-
CAGTTGCAGTTAAGGCAGCTGGTTTAGAGCTTGATGATATTGACCTTTTT-
GAGATAAATGAGGCTTTTGCATCCCAATTTGTGTATTGCCGTAACAAGCTG-
GGACTTGATCCAGAAAAAATCAATGTTAACGGAGGTGCAATGGCCATCGGC-
CATCCACTTGGTGCAACAGGTGCCCGGTGTGTTGCCACTCTTTTGCATGA-
GATGAAGCGTCGTGGTAAAGACTGCCGCTATGGAGTGATCTCAATGTG-
CATAGGCACAGGGATGGGTGCAGCCGCTGTTTTTGAAAGAGGAGACCG-
GACCGATGAACTCTGCAATGCTCGCAAGGTTGAATCACTCAACTTCTTATC-
CAAGGATGTTCGGTAGTAGAGAATGGTTAGTGACAGGAGCTATTCCAATCA-
ATAATGTTTGGTGGAGTCTGAAAATCATAGTAAAGCACTGGAATAACGTTG-
CTAAGTTTTTCGTTGGGTACTACCTTGTTTATTGGGATGGAATACACATG-
TAGTTGGTTTGTTCTCCCAGACCTCCCACTTGTTGGCATATTCATTTTTGTC-
CAACCTAAAAAGTTCCATTTTATAGGACTTCATCTCAATAACATTGGGTTTG-
CGCCACTAAAGCAGTGCCTAAAACTGTAATTGGGTAATTTTGGTATACCT-
GTTTGCTACTTTTCTTTTCTAAGTTAATCAAGCCCTGCCCACCTCATA-
TAAAAAAAAAAAAAAAAAAAAA

INFORMATION FOR SEQ ID NO: 4B (SLG150)

SEQUENCE CHARACTERISTICS:

LENGTH: 458

TYPE: Peptide

STRANDNESS: Single

TOPOLOGY: Linear

OTHER INFORMATION: STRAWBERRY THIOLASE

SEQUENCE DISCRIPTION FOR SEQ ID NO: 4B

MEKAINRQKVLLDHLRPSSSSDDSSLSASVCAAGDSAAYARNHVFGDDVVI-

VAAFRTPLCKAKRGGFKYTYADDLLAPVLKAVVEKTNLNPKEVGDIVVGTV-

LAPGSQRASECRMAAFYAGFPETVPVRTVNRQCSSGLQAVADVAAAIRAGFY-

DIGIGAGLESMTANPMAWEGDVNPKVKIFEQAQNCLLPMGVTSENVAHRFGV-

SRQEQDQAAVDSHRKAAAAAAAGRFKDEIIPVATKIVDPKSGDEKPVTISVD-

DGIRNTTLADLAKLKPVFKKDGTTTAGNSSQVSDGAGAVLLMKRSVADQKGL-

PILGVFRNFVAVGVDPAIMGVGPAAAIPVAVKAAGLELDDIDLFEINEA-

FASQFVYCRNKLGLDPEKINVNGGAMAIGHPLGATGARCVATLLHEMKRRGK-

DCRYGVISMCIGTGMGAAAVFERGDRTDELCNARKVESLNFLSKDVR

INFORMATION FOR SEQ ID NO: 5A (SLH51)

SEQUENCE CHARACTERISTICS:

LENGTH: 2141

TYPE: cDNA

STRANDNESS: Single

TOPOLOGY: Linear

OTHER INFORMATION: STRAWBERRY PYRUVATE DECARBOXYLASE

SEQUENCE DISCRIPTION FOR SEQ ID NO: 5A

ATTTTCACTCAGAGTCTCAATCTTTCATCACAAAAATTCCCATTTGATCA-

CAAAAAAGTTTCAACCTTTAAACCTCCATGGACACCAAGATTGGCTC-

CATCGACGTCTGCAAAACCGAGAACCACGACGTCGGTTGTTTACCAAACAG-

CGCCACCTCCACCGTTCAAAACTCAGTCCCTTCCACCTCCCTCAGCTCCGC-

CGACGCCACCCTCGGCCGCCACCTGGCACGCCGCCTCGTTCAAATCGGCGT-

CACCGACGTCTTCACCGTCCCCGGCGACTTCAACTTGACCCTTCTTGACCAC-

CTCATCGCCGAGCCCGGCCTCACCAACATTGGCTGCTGCAACGAGCT-

CAACGCCGGGTACGCCGCCGACGGCTACGCGCGGTCGCGTGGCGTCGGCG-

CGTGCGTGGTGACTTTCACTGTTGGTGGACTGAGTGTGCTGAACGCGATCG-

CCGGCGCGTATAGTGAGAATTTGCCGGTGATTTGTATTGTTGGTGGGCC-

CAACTCTAACGATTATGGGACTAACCGGATTCTTCACCATACTATTGGGTT-

GCCGGACTTCAGTCAAGAGCTCCGGTGCTTTCAGACCGTGACTTGCTTT-

CAGGCTGTGGTGAATAATCTGGAGGATGCACATGAGATGATTGATACTGCA-

ATTTCGACTGCGTTGAAAGAAAGCAAGCCTGTGTATATCAGCATTGGCTG-

CAACTTGGCTGGGATTCCTCATCCTACTTTCAGCCGTGAACCTGTTCCATTT-

TCATTGTCTCCAAAATTGAGCAATAAGTGGGGATTAGAGGCTGCAGTG-
GAGGCTGCTGCAGAGTTCTTGAACAAGGCAGTGAAGCCAGTTATGGTGGG-
CGGGCCCAAACTGCGCTCTGCACATGCTGGTGATGCCTTTGTTGAACTGGC-
TGATGCTTCTGGATTTGCTCTGGCTGTGATGCCATCAGCAAAGGGGCAAGT-
GCCAGAGCACCACCCCCATTTCATCGGAACGTACTGGGGTGCTGTGAGCAC-
TGCCTTTTGTGCTGAGATTGTGGAGTCTGCAGATGCATACTTGTTTGCTG-
GGCCGATTTTCAATGACTACAGCTCAGTTGGGTACTCGCTCCTTCTCAA-
GAAAGAGAAGGCGATCATTGTGCAGCCAGATCGTGTGACGATAGGGAAT-
GGCCCTACATTTGGTTGTGTTCTCATGAAGGATTTCCTCTTAGGCCTAG-
CAAAGAAGCTGAAGCATAACAACACTGCTCATGAGAACTACCGCAGGATCT-
TTGTGCCTGATGGCCACCCTCTGAAGGCTGCACCCAAAGAACCTTTGAGG-
GTTAATGTTCTGTTCAAACACATTCAGAATATGCTGTCAGCTGAAACCGCT-
GTGATTGCTGAGACAGGGGACTCATGGTTTAACTGTCAGAAGCTGAAATTG-
CCACCCGGCTGCGGGTATGAGTTCCAAATGCAATATGGATCAATTGGTTG-
GTCAGTTGGAGCAACTCTTGGGTATGCTCAGGCTGTACCTGAGAAGCGAGT-
GATTTCTTTCATTGGTGATGGGAGCTTCCAGGTGACTGCTCAAGATGTGTC-
CACAATGATTCGAAATGGACAGAGAACCATTATTTCCTGATAAACAATG-
GTGGATACACCATTGAAGTGGAAATCCATGATGGACCATACAATGTGAT-
CAAGAACTGGAACTACACTGGACTGGTTGATGCAATCCACAATGGG-
GAAGGCAAGTGCTGGACAACCAAGGTGCGTTGCGAAGAGGAGCTGATT-
GAAGCAATAGAGACTGCAAATGGACCCAAGAAGGATAGCTTCTGCTTCATT-
GAGGTGATTGTTCACAAGGATGATACCAGCAAAGAGTTGCTTGAGTGGGG-
GTCTAGGGTTTCTGCTGCCAACAGCCGCCCACCTAATCCTCAGTAAAACTC-
TCCTGTGTCATATGAAGGCCTTCATTCACATTCACAGATTTAGATCAAGC-

CAAGCTCTTGTGCAAATTTTCCTTATGTTTTTCCTGTCAACTGGAGAATGGT-

GTCTGTCAAGTTTTTTTACACTACAGTGATTTCTGGTTTGTCTGTATATT-

TCCTTCTGAATATTAGTATCTTCTGATTTTTCAATTGATCAAATTCTGT-

GATCCTAAATGGTTTGTGGAAAAAAAAAAAAAAAAAAA

INFORMATION FOR SEQ ID NO: 5B (SLH51)

SEQUENCE CHARACTERISTICS:

LENGTH: 605

TYPE: Peptide

STRANDNESS: Single

TOPOLOGY: Linear

OTHER INFORMATION: STRAWBERRY PYRUVATE DECARBOXYLASE

SEQUENCE DISCRIPTION FOR SEQ ID NO: 5B

MDTKIGSIDVCKTENHDVGCLPNSATSTVQNSVPSTSLSSADATLGRHLARRL-

VQIGVTDVFTVPGDFNLTLLDHLIAEPGLTNIGCCNELNAGYAADGYARSR-

GVGACVVTFTVGGLSVLNAIAGAYSENLPVICIVGGPNSNDYGTNRILHHTI-

GLPDFSQELRCFQTVTCFQAVVNNLEDAHEMIDTAISTALKESKPVYISIGCN-

LAGIPHPTFSREPVPFSLSPKLSNKWGLEAAVEAAAEFLNKAVKPVMVGGPKL-

RSAHAGDAFVELADASGFALAVMPSAKGQVPEHHPHFIGTYWGAVSTAF-

CAEIVESADAYLFAGPIFNDYSSVGYSLLLKKEKAIIVQPDRVTIGNGPTFGCV-

LMKDFLLGLAKKLKHNNTAHENYRRIFVPDGHPLKAAPKEPLRVNVLFK-

HIQNMLSAETAVIAETGDSWFNCQKLKLPPGCGYEFQMQYGSIGWSVGATL-

GYAQAVPEKRVISFIGDGSFQVTAQDVSTMIRNGQRTIIFLINNGGYTIEVEIH-

DGPYNVIKNWNYTGLVDAIHNGEGKCWTTKVRCEEELIEAIETANGPKKDSF-
CFIEVIVHKDDTSKELLEWGSRVSAANSRPPNPQ

INFORMATION FOR SEQ ID NO: 6A (SLB39)

SEQUENCE CHARACTERISTICS:

LENGTH:1415

TYPE: cDNA

STRANDNESS: Single

TOPOLOGY: Linear

OTHER INFORMATION: STRAWBERRY ALCOHOL DEHYDROGENASE

SEQUENCE DISCRIPTION FOR SEQ ID NO: 6A

TAATCTAGCTTCTGCACCAAAACTATCAGATAATTAAGAATCTGCCACAGA-

GAAAATGGTGATGTCTATCGAGCAGGAACACCCCAAGAAGGCATCTGGAT-

GGGCTGCAAGAGATTCATCTGGTGTTCTCTCTCCCTTCAGTTTCTCCAGAAG-

GGAAACCGGAGAGAAAGACGTGACGTTCAAAGTGATGTACTGTGGGATTT-

GCCATTCGGACCTTCACATGGTCAAGAATGAATGGGGCTTCTCTACCTATC-

CTCTGGTTCCAGGGCATGAGATTGTTGGTGAAGTGACGGAAGTAGGAAG-

CAATGTACAAAAATTCAAAGTTGGAGACAGAGTCGGTGTTGGATGCATTGT-

GGGATCTTGCCGATCTTGTGAAAATTGTACCGACCACCTTGAGAACTACTG-

CCCCAAACAGATACTCACTTACGGTGCCAAGTACTACGACGGAACCACCAC-

CTATGGCGGTTACTCTGACATTATGGTGGCCGATGAACACTTCATAGTACG-

CATCCCAGACAACTTGCCTCTTGATGGTGCTGCGCCGCTCCTATGTGCCGG-

GATTACAACCTACAGCCCCCTGAGATATTTCGGACTTGACAAGCCCGGCAT-

GCATGTAGGTGTGGTCGGCCTAGGCGGTTTAGGCCACGTCGCCGTGAAGT-

TTGCCAAGGCTATGGGAGTGAAGGTTACAGTGATTAGTACATCCCCTAA-

GAAAGAGGAGGAAGCTCGTAAACACCTAGGAGCTGACTCGTTTTTGGTTAG-
CCGTGACCAAGATCAAATGCAGGCTGCCATTGGTACCATGGATGGGAT-
CATTGACACGGTTTCTGCACAACATCCTCTCCTGCCTTTGATTGGTTTGTT-
GAAGTCTCATGGAAAGCTTGTTATGGTTGGTGCACCAGAGAAGCCTCTT-
GAACTGCCAGTTTTTCCTTTACTCATGGGAAGAAAGATGGTAGCTGGTAG-
CGGCATTGGGGGTATGAAGGAGACACAAGAGATGATAGATTTTGCAGC-
CAAGCACAACATTACAGCAGACATCGAAGTCATACCAATCGACTACTTG-
TAACACTGCTATGGAGCGTCTAGTCAAAGCAGATGTCAGATACCGTTTTGT-
CATCGACATTGGAAACACACTGAAGGCTAGCTCTTAAATTCTGCAATCCA-
GACTGGATCAATGAAGAAACAAGAACAGAAACGGAGACTGATTTAGTGT-
CATACTCGGTGTTGGTTTTCCTTGTAGCATTTTTGTTGTCTGCTACATGA-
ATAATGATCACATGAACAACTGCCTTCTGTGATGATTTGATAATAAAAGA-
ATACATGAACAATGATACTGCCTTCTTTTGTAATGTTTTTTACTATATAAT-
CATTTCAAATTATTTTGCTA-
TATCTCTAAAAAAAAAAAAAAAAAAAAAAAAAAAAAAA

INFORMATION FOR SEQ ID NO: 6B (SLB39)
SEQUENCE CHARACTERISTICS:
LENGTH: 333
TYPE: Peptide
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 6B

MVMSIEQEHPKKASGWAARDSSGVLSPFSFSRRETGEKDVTFKVMYCGICHS-

DLHMVKNEWGFSTYPLVPGHEIVGEVTEVGSNVQKFKVGDRVGVGCIVGSCR-

SCENCTDHLENYCPKQILTYGAKYYDGTTTYGGYSDIMVADEHFIVRIPDNL-

PLDGAAPLLCAGITTYSPLRYFGLDKPGMHVGVVGLGGLGHVAVKFAKAM-

GVKVTVISTSPKKEEEARKHLGADSFLVSRDQDQMQAAIGTMDGIIDTVSAQH-

PLLPLIGLLKSHGKLVMVGAPEKPLELPVFPLLMGRKMVAGSGIGGM-

KETQEMIDFAAKHNITADIEVIPIDYL

INFORMATION FOR SEQ ID NO: 7A (SLF193)

SEQUENCE CHARACTERISTICS:

LENGTH: 1227

TYPE: partial cDNA

STRANDNESS: Single

TOPOLOGY: Linear

OTHER INFORMATION: STRAWBERRY ALCOHOL DEHYDROGENASE

SEQUENCE DISCRIPTION FOR SEQ ID NO: 7A

GGAAACAGGAGCAACGGACGTAAGATTCAAAGTGTTGTACTGTGGAGTAT-

GCCATTCGGACATACACATGGCCAAAAATGATTGGGGGACTTCTACCTATC-

CTATTGTACCTGGGCATGAACTTGTTGGTGTAGTAACAGAAGTAGGATG-

CAAAGTAAAGAAATTCAAAAGTTGGAGACAAGGTCGGTGTTGGTTGCATG-

GTCGACTCAGACCAACTTGCGAAAATTGTATCCATCACCTAGAAAATTACT-

GTCCGAATCTGATACAAACCTACGGTTCTAAATACTACGACGGAACCATGA-

CATACGGAGGTTACTCGAACAACATGGTGACTGATGAGCACTTCATTGT-

TCGGATCCCGGACAACTTACCTCTTGATGGCGCTGCTCCGCTTCTATGTGC-

CGGGATTACAACTTACAGCCCATGGAGATATTATGGACTTGACAAACCCGG-

TATGCATCTTGGTGTTGAATGGCCTAGGCGGTTTAGGTCACGTCCGCCGT-

TAAATTTGCCAGGGCTTTGGGGCTCAAGGTTACAGTCATTAGTACCTCCCC-

TAATTAAAGAAGGAGGCAGCTATGGAACATCTCCCGCGCTGATGCATTCCC-

TGCTTAGAACTGACCAAGATCAGATGGAGGCTGCCATGAGCACAATGGAT-

GGTATCATTGACACAGTTCCTGCAGTTCGACCTCTAGAGCCTTTGATTT-

CATTGTTGAAGACTAATGGAAAAGTTGTTACCGTTGGTATAGCAGTGCAGC-

CACTCGATCTCCCAGTTTTCCCTTTGATAATAGGAAGGAAGATGGTAGCTG-
GTAGTGCCATTGGAGGTATGAAAGAGACGCAAGAGATGATTGATTTTGCTG-
CTGAACATAACATAACAGCTGACATCGAGGTCATCCCGATTGATTACCT-
GAACACCGCAATGGAACGCGTTGTCAAAAAAGATGTCAGGTTTCGATTTGT-
CATCGACGTTGAGAACACATTGTAAGTCCGCCTAAGTTTTTCATTCAATTCT-
GTTAATAAGACTATGCATTAATATATGACTGACTCTCCATAGGATGGAGT-
TATCAGTCTTCAAATTTCTAGACATATTTTGTGATCAAATAAATGGAATGGC-
TTTGTTTTCCTTTTCCACTAAGATTAGATTTCAGTTGTATTGTTTTTAAAGA-
GATTGATGTTTTTATTAATTGTAACAGTGTTATCAGTCTAATCAT-
TAAAAAAAAAAAAAAAAAA

INFORMATION FOR SEQ ID NO: 7B (SLF193)
SEQUENCE CHARACTERISTICS:
LENGTH: 326
TYPE: partial peptide
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 7B

ETGATDVRFKVLYCGVCHSDIHMAKNDWGTSTYPIVPGHELVGVVTEVG-
CKVKKFKSWRQGRCWLHGRLRPTCENCIHHLENYCPNLIQTYGSKYYDGTM-
TYGGYSNNMVTDEHFIVRIPDNLPLDGAAPLLCAGITTYSPWRYYGLDKPGM-
HLGVEWPRRFRSRPPLNLPGLWGSRLQSLVPPLIKEGGSYGTSPALMHSLLRT-

DQDQMEAAMSTMDGIIDTVPAVRPLEPLISLLKTNGKVVTVGIAVQPLDLPVF-
PLIIGRKMVAGSAIGGMKETQEMIDFAAEHNITADIEVIPIDYLNTAMERVVKK-
DVRFRFVIDVENTL
INFORMATION FOR SEQ ID NO: 8A (SLF122)
SEQUENCE CHARACTERISTICS:
LENGTH: 1064
TYPE: partial cDNA
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 8A
GCAAAGTGCAAAAATTTAAAGTTGGAGACAAAGTTGGTGTTGGGTGCTTGG-
TAGGCTCATGCAAAACTTGCGACAGCTGTGCTAACGATTTGGAGAACTACT-
GCCCCAAACAGATACAGACTTACGGCGCCAAGTACCTTGACGGAACAAC-
CACATACGGCGGTTACTCTGACATCATGGTGGCGGATGAGGCCTTTGTA-
ATCCGTATTCCGGACAACCTGCCTCTTGAGGGTGCTGCTCCTCTCCTATGTG-
CCGGAATCACAACTTACAGTCCCCTGAGGTATTTCGGACTTGACAAACC-
CGGCATGCATGTCGGGGTGGTTGGCCTTGGCGGTTTAGGCCATGTCGCGGT-
GAAGTTTGCCAAGGCTTTGGGGGTTAATGTCACAGTGATCAGTACCTCCGC-
TAATAAGAAAGATGAAGCTATTAAACACCTTGGTGCTGATTCTTTCTTGGT-
CAGTCGTGACCAAGATCAGATGCAGGCTGCCATGGGAACATTGGACGGTAT-
CATCGACACAGTTTCCGCAGTCCACCCCCTCCCACCTTTGATTAGTTTATT-
GAAGGCTAATGGAAAGCTTGTTATGGTTGGAGCACCAGAGAAGCCACTT-
GAGCTACCAGTTTTTCTTAATAATGGAAGGAAGACTTTAGCCGGTAG-

TAATATCGGAGGTATCAAGGAGACACAAGAGATGATAGATTTGGCAGC-
CAAACACAACATAACGGCCGACATCGAGATTATCCCCATCGACTATTTGAA-
CACTGCTATGGAGCGTCTTGCTAAAGGGGATGTTAGATACCGTTTTGT-
CATCGACATCGGAAACACATTGAAGCCGGCCATTTAAATTTGCATTTCGAT-
CAGAAACTGAATCAAGCGAGGTCGAGAGGCCTACGTAACAATGCAAACAT-
GTGCTAGCTTGTTCTTGGAGTAGTCTTTAGCTTTTCTCTGATGTATTCCATC-
TGTTTTGTTCATGTCCCATCTTATTATGAGAAAAATGTGGGTACCGTGGA-
TATTGAATAAATGAAGAGCTACTGGAACGATGGTTTCA-
CAAAAAAAAAAAAAAAAAA

INFORMATION FOR SEQ ID NO: 8B (SLF122)
SEQUENCE CHARACTERISTICS:
LENGTH: 278
TYPE: partial peptide
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 8B

KVQKFKVGDKVGVGCLVGSCKTCDSCANDLENYCPKQIQTYGAKYLDGTT-
TYGGYSDIMVADEAFVIRIPDNLPLEGAAPLLCAGITTYSPLRYFGLDKPGM-
HVGVVGLGGLGHVAVKFAKALGVNVTVISTSANKKDEAIKHLGADSFLVSRD-
QDQMQAAMGTLDGIIDTVSAVHPLPPLISLLKANGKLVMVGAPEKPLELPVF-

SLIMGRKTLAGSNIGGIKETQEMIDLAAKHNITADIEIIPIDYLNTAMERLAKGD-
VRYRFVIDIGNTLKPAI

INFORMATION FOR SEQ ID NO: 9A (SLD194)
SEQUENCE CHARACTERISTICS:
LENGTH: 1228
TYPE: partial cDNA
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 9A

GCAAGAGATTCATCTGGTGTCCTCTCTCCCTTCAATTTCTCCAGAAGG-
GAAACCGGAGAGAAAGACGTTATGTTCAAAGTGTTGTACTGTGGAATTTGC-
CATTCGGACCTTCACATGGTCAAGAATGAATGGGGCTTCTCTACCTATCCT-
TTGGTCCCGGGGCATGAGATTGTTGGTGAAGTTACGGAAGTAGGGAG-
CAAAGTACAAAAATTTAAAGTTGGAGACAGAGTCGGTGTTGGATGCGTTGT-
GGGATCTTGCCGATCTTGTGAAAATTGTACCGACCACCTTGAGAACTACTG-
CCCCAAACAGATACTCACTTACGGTGCCAAGTACTACGACGGAACCACCAC-
CTATGGCGGTTACTCTGACATTATGGTGGCCGACGAACACTTCATAGTACG-
CATCCCAGACAACTTGCCTCTTGATGGCGCTGCGCCGCTCCTATGTGCCGG-
GATTACAACCTACAGCCCCCTGAGATATTTCGGACTTGACAAGCCCGGCAT-
GCATGTAGGTGTGGTCGGCCTAGGCGGTTTAGGCCACGTCGCCGTGAAGT-
TTGCCAAGGCTATGGGAGTGAAGGTTACAGTGATCAGTACGTCCCCTAA-

GAAAGAGGAGGAAGCTCTTAAACACCTAGGAGCTGACTCGTTTTTCGTTAG-
CCGTGACCAAGATCAAATGCAGGCTGCCATTGGTACCATGGATGGGAT-
CATTGACACAGTTTCTGCACAACATCCTCTCCTGCCTTTGATTGGTTTGTT-
GAAGTCTCATGGAAAGCTTGTTATGGTTGGTGCACCAGAGAAGCCTCTT-
GAACTTCCAGTTTTTCCTTTACTCATGGGAAGAAAGATGGGTAGCTGG-
TAACCGGCATTTGGGGGTATGAAGGAGACACAAGAGATGATAGATTTTGC-
TGCCAGGCACAACATAACAGCAGACATCGAAGTCATACAATCGACTACT-
TAAACACTGCTATGGAGCGTTTAGTCAAAGCAGATGTCAGATACCGTTTT-
GTCATCGACATTGGAAACACACTGAAGGCTAGCACTTAAATTCTGCAATC-
CAGACTGTATCAATGAAGAAACAAGAACAGAAACTGAGATTGATTTGGTGT-
CATACTCCGCCTATGGTTTTCCTTACAGCATTTTTGTTGTTTGCTACATGA-
ATAACGATCACATGAACTGTGATGATTTGATAATAAAAGAATACATAAA-
CAAAAAAAACAAAAAAAAAAAAAAAAA

INFORMATION FOR SEQ ID NO: 9B (SLD194)
SEQUENCE CHARACTERISTICS:
LENGTH: 284
TYPE: partial Peptide
STRANDNESS: Single
TOPOLOGY: Linear
OTHER INFORMATION:   STRAWBERRY ALCOHOL DEHYDROGENASE
SEQUENCE DISCRIPTION FOR SEQ ID NO: 9B
ARDSSGVLSPFNFSRRETGEKDVMFKVLYCGICHSDLHMVKNEWGFSTYPL-
VPGHEIVGEVTEVGSKVQKFKVGDRVGVGCVVGSCRSCENCTDHLENYCP-

KQILTYGAKYYDGTTTYGGYSDIMVADEHFIVRIPDNLPLDGAAPLLCAGIT-
TYSPLRYFGLDKPGMHVGVVGLGGLGHVAVKFAKAMGVKVTVISTSPK-
KEEEALKHLGADSFFVSRDQDQMQAAIGTMDGIIDTVSAQHPLLPLIGLLKSH-
GKLVMVGAPEKPLELPVFPLLMGRKMGSW

INFORMATION FOR SEQ ID NO: 10A (SLF17)
SEQUENCE CHARACTERISTICS:
LENGTH: 852
TYPE: partial cDNA
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 10A

GTCCCCTGAGGTATTTCGGACTTGACAAACCCGGCATGCATGTCGGGGTG-
GTTGGCCTTGGCGGTTTAGGCCATGTCGCGGTGAAGTTTGCCAAGGCTTTG-
GGGGTTGAGGTCACAGTGATCAGTACCTCCGCTAATAAGAAAGATGAAGC-
TATTAAACACCTTGGTGCTGATTCTTTCTTGGTCAGTCGTGACCAAGATCA-
GATGCAGGCTGCCATGGGAACATTGGACGGTATCATCGACACAGTTTCTG-
CAGTCCACCCCCTCCCACCTTTGATTAGTTTATTGAAGGCTAATGGAAAGC-
TTGTTATGGTTGGAGCACCAGAGAAGCCACTTGAGCTACCAGTTTTTCTT-
TAATAATGGGAAGGAAGACTTTAGCCGGTAGTAATATCGGAGGTATCAAG-
GAGACACAAGAGATGATAGATTTGGCAGCTAAACACAACATAACGGCCGA-
CATCGAGGTCATCCCCATCGATTATTTGAACACTGCAATGGAGCGTCTTGC-

TAAAGGGGATGTTAGATACCGGTTTGTCATCGACATCGGAAACACATT-

GAAGCCGGCCACTTAAATTTGCATTTCGATCAGAAACTGAATCAAGCGAT-

GTCGAGAGGCCTACGTAACAATGTAAACATGTGCTAGCTTGTTCTTGTAG-

TAGTCTTTAGCATTTCTCTGATGTACTCCTTCTGTTTTGTTCATGTTCCATCT-

TATAATAAGATTCTTATTATGAAAAAAATATGGTACCGTGGATATTGA-

ATAAATGAAGAACTACTGGAACAATGGTTTCACAAATTATTTGTGGTGC-

TAAAAAAAAAAAAAAAAAAAAAAAAAAAAAAAAAAAAAAAAAAAAAAAA

INFORMATION FOR SEQ ID NO: 10B (SLF17)

SEQUENCE CHARACTERISTICS:

LENGTH: 188

TYPE: partial peptide

STRANDNESS: Single

TOPOLOGY: Linear

OTHER INFORMATION: STRAWBERRY ALCOHOL DEHYDROGENASE

SEQUENCE DISCRIPTION FOR SEQ ID NO: 10B

PLRYFGLDKPGMHVGVVGLGGLGHVAVKFAKALGVEVTVISTSANKKDEAIK-

HLGADSFLVSRDQDQMQAAMGTLDGIIDTVSAVHPLPPLISLLKANGKLVM-

VGAPEKPLELPVFSLIMGRKTLAGSNIGGIKETQEMIDLAAKHNITA-

DIEVIPIDYLNTAMERLAKGDVRYRFVIDIGNTLKPAT

INFORMATION FOR SEQ ID NO: 11A (SLF138)

SEQUENCE CHARACTERISTICS:

LENGTH: 664
TYPE: partial cDNA
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 11A
AGTTTGGTCTTGATGTGGGTGGATTAAGGGGAGGGATATTGGGACTTG-
GAGGTGTTGGACACATGGGGGTGAAGATAGCAAAGGCTATGGGACAC-
CATATCACCGTGATAAGCTCTTCTGATAAGAAGAAAAAGAGGCCTTGGAG-
CATATTGGTGCTGATGAGTACTTGGTGAGCTCTGATGCCACCCAAATGCAA-
GAGGCTATGGACTCACTGGATTACATTATTGACACCATTCCAGTGTTCCAC-
CCTCTTGAGCCTTACCTCTCTTTGTTGAAGCTTGATGGGAAGTTGATCTT-
GATGGGTGTTATCAACACCCCATTGCAATTTGTCTCTCCATTGGTCATGCTT-
GGGGAGGAAGACGATCACCGGGAGCTTTGTGGGGAGCATGAAGGAGATG-
GAGGAGATGCTCGAGTTCTGCAAAGAGAAAGAGCTGAAACGATGATT-
GAAGTGGTGAAGATGGACTACATCAACGAAGCTTTCGAAAGGTTGGAGAA-
GAACGACGTTAGGTACAGGTTCGTTGTGGATTGTTGCCGGCAGCAATCTT-
GATCAATAAGAAAGAAAGAAGGCATCATCGAGTGTTGTCCTATTTT-
TATCGAGTACTCTGTCTCATCTTATCTTAAACAATATAAATAAACAAA-
GAAAAAAAAAAAAAAAAAAAA

INFORMATION FOR SEQ ID NO:11B (SLF138)
SEQUENCE CHARACTERISTICS:
LENGTH: 181

TYPE: partial Peptide

STRANDNESS: Single

TOPOLOGY: Linear

OTHER INFORMATION: STRAWBERRY ALCOHOL DEHYDROGENASE

SEQUENCE DISCRIPTION FOR SEQ ID NO: 11B

FGLDVGGLRGGILGLGGVGHMGVKIAKAMGHHITVISSSDKKKKEALEHI-

GADEYLVSSDATQMQEAMDSLDYIIDTIPVFHPLEPYLSLLKLDGKLILM-

GVINTPLQFVSPLVMLGEEDDHRELCGEHEGDGGDARVLQRERAETMIEVVK-

MDYINEAFERLEKNDVRYRFVVDCCRQQS

INFORMATION FOR SEQ ID NO: 12A (SLG16)
SEQUENCE CHARACTERISTICS:
LENGTH: 694
TYPE: partial cDNA
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 12A
GTGCATTGCTATGCCTATGAAGGCAAGATGCAAGAACATCTGCAATTATGT-
GAAGACGAGTTTAAAAAGATAATGAAGATAAATTTCATGTCTGCATGGTT-
TCTGGTAAATGCCGTTGGCAGAAGAATGCGAGATCATAAATCAGGAGGTTC-
CATCATATTGTTGACCTCGATTGTTGGAGCTGAAAGAGGGCTTTATACAG-
GAGCTGTTGCCTATGGTGCATGTTCGGCAGCACTGCAGCAGTTAGTAAG-
GTCGTCGGCATTGGAGATTGGAAAATACCAGATCAGGGTTAATGCAATCG-
CACGTGGTTTGCATTTGGAAGATGAGTTTCCTAAGTCTGTGGGAATAGAGA-
GAGCAAAGAAGCTGGTGAATGATGCAGTTCCGCTGGAGAGATGGCTTGAT-
GTTAAAAATGATGTGGCTTCAAGTGTCATATATTTGGTCAGTGATGGTT-
CAAGGTACATGACGGGCACAACTATATTTGTTGATGGGGCACAGTCTC-
TCGTGAGGCCTCGAATGCGTTCTTATATGTGATTCTTGCTCCTATTATATCC-
TCCTAGCCATTATTAGCTACTTAGGTTTGTTCATACTTCATAGGTGAACT-
CATTAGCTATTCTTACATTTGTTCCTTATGAATAAAGAAGTCAAGATT-
CAAAAAAAAAAAAAAAAAAAAAAAAAAAAAAAAAAAAAAAAAAA

INFORMATION FOR SEQ ID NO: 12B (SLG 16)

SEQUENCE CHARACTERISTICS:

LENGTH: 176

TYPE: partial Peptide

STRANDNESS: Single

TOPOLOGY: Linear

OTHER INFORMATION: STRAWBERRY ALCOHOL DEHYDROGENASE

SEQUENCE DISCRIPTION FOR SEQ ID NO: 12B

VHCYAYEGKMQEHLQLCEDEFKKIMKINFMSAWFLVNAVGRRMRDHKSGG-

SIILLTSIVGAERGLYTGAVAYGACSAALQQLVRSSALEIGKYQIRVNAIARGL-

HLEDEFPKSVGIERAKKLVNDAVPLERWLDVKNDVASSVIYLVSDGSRYMT-

GTTIFVDGAQSLVRPRMRSYM

INFORMATION FOR SEQ ID NO: 13A (SLG144)

SEQUENCE CHARACTERISTICS:

LENGTH: 1010

TYPE: partial cDNA

STRANDNESS: Single

TOPOLOGY: Linear

OTHER INFORMATION: STRAWBERRY ALCOHOL DEHYDROGENASE

SEQUENCE DISCRIPTION FOR SEQ ID NO: 13A

GGAAACTACCATCAATTTTGGGTCTAAGAAGATTGCAGTTGTTACTGGAGC-

CAACAAAGGGATTGGACTTGAGATTAGCAAGCAATTAGCTGCTAAAG-

GAGTTGGGGTGGTATTAACAGCAAGAGATGTGAAGAGAGGAACAGAAGCT-

GCTGAAAATCTTAAGGCTTCTGGGTTCTCTGATGTGGTATTTCATCAGCTA-

GATGTAACAGAGCCGACTACTATTGGTTCTTTGGCAAACTTTCTTGAAACG-

CAATTTGGAAAGCTTGACATATTGGTTAACAATGCAGGAGTCGTTGGATCT-

GTATACCTCACAGCCGACTATGATCCAGTGCAAACATACGAGACAGCGAG-

GGATTGTTTGAAAACAAACTATTATGGGCTCAAGCAAGTCACAGAAGCAC-

TTGTTCCGCTGCTTCAAAAATCTGAAGCTGCAAGGATAGTCAATGTCTCTTC-

CGGATTAGGACAGCTAAGAAATATTGGAAATGAGAAGGCCAAGAAGGAGC-

TAGGAGATGCAGATAACCTCAACGAGGAGAAAGTGGACAAGCTAGTTGAG-

GAATTTCTGGAGGATGTGAAACAGGATTCGATAGAATCCAAAGGCTGGCC-

TCTAAGTATATCTGCCTACATTGTCTCAAAAGCAGCTCTGAATGCTTATA-

CAAGACTCTTGGCAAAGAAGTATCCCCATATTGCCATAAACGCAGTTGGTC-

CAGGTTATACCAAAACAGACCTCAATAATAATTCCGGGATTCTCACAGTT-

GAAGAAGCTGCAGTAGGTCCTGTGAGGCTGGCTTTGATAGCCGAAACTA-
GAATTTCCGGCCTCTTCTTCAACAGAAATGAAGAGTCGACCTTTGATTAG-
GTCAACGTGATCCCTGATGAACTGGACTATTTTAGATTTTCAGAATGTGC-
TTGATTTTGTTGAAGTATTTATGGGATTTGTATGTATACTTTGATGTAT-
CATTGTATTAATAGAGCACATGTTGTGAT-
CAAAAAAAAAAAAAAAAAAAAAAAAAAA

INFORMATION FOR SEQ ID NO: 13B (SLG144)
SEQUENCE CHARACTERISTICS:
LENGTH: 284
TYPE: partial Peptide
STRANDNESS: Single
TOPOLOGY: Linear
OTHER INFORMATION: STRAWBERRY ALCOHOL DEHYDROGENASE
SEQUENCE DISCRIPTION FOR SEQ ID NO: 13B
ETTINFGSKKIAVVTGANKGIGLEISKQLAAKGVGVVLTARDVKRGTEAAENL-
KASGFSDVVFHQLDVTEPTTIGSLANFLETQFGKLDILVNNAGVVGSVYL-
TADYDPVQTYETARDCLKTNYYGLKQVTEALVPLLQKSEAARIVNVSSGL-
GQLRNIGNEKAKKELGDADNLNEEKVDKLVEEFLEDVKQDSIESKGWPLSI-
SAYIVSKAALNAYTRLLAKKYPHIAINAVGPGYTKTDLNNNSGILTVEEAAVG-
PVRLALIAETRISGLFFNRNEESTFD

INFORMATION FOR SEQ ID NO: 14B (Motif 1)

SEQUENCE CHARACTERISTICS:

LENGTH: 12

TYPE: amino acid

STRANDNESS: Single

TOPOLOGY: Linear

HYPOTHETICAL SEQUENCE: NO

SEQUENCE DISCRIPTION FOR SEQ ID NO: 14B

Trp Thr Asn Phe Phe Asn Pro Leu Asp Phe Gly Trp

W T N F F N P L D F G W

## Claims

1. An isolated DNA sequence encoding
(a) a polypeptide having an amino acid sequence as shown in SEQ ID NO: 1B or SEQ ID NO: 2B, or
(b) a polypeptide having at least 30% homology with the amino acid sequence SEQ ID NO: 1B or at least 40% homology with the amino acid sequence SEQ ID NO: 2B, or
(c) a fragment of polypeptide (a) or (b),
which polypeptide or fragment of polypeptide has alcohol acyl transferase activity and is involved in the biosynthetic pathway for aliphatic and /or aromatic ester production in fruit.

2. An isolated DNA sequence according to claim 1 encoding a polypeptide having at least 50% homology with any of the amino acid sequences SEQ ID NO: 1B or SEQ ID NO: 2B, or encoding a fragment thereof.

3. An isolated DNA sequence according to claim 1 or 2 encoding a polypeptide having at least 70% homology with any of the amino acid sequences SEQ ID NO: 1B or SEQ ID NO: 2B, or encoding a fragment thereof.

4. An isolated DNA sequence having a nucleic acid sequence
(a) as shown in SEQ ID NO: 1A or SEQ ID NO: 2A which encodes a polypeptide having alcohol acyl transferase activity and being involved in the biosynthetic pathway for aliphatic and/or aromatic ester production in fruit, or
(b) complementary to SEQ ID NO: 1A or SEQ ID NO: 2A, or
(c) which has at least 25% homology with any of the sequences of (a) or (b), or
(d) which is capable of hybridising under stringent conditions to any of the sequences (a) - (c).

5. An isolated DNA sequence according to claim 4 having a nucleic acid sequence
(c) which has at least 40% homology with any of the sequences of (a) or (b), or
(d) which is capable of hybridising under stringent conditions to said sequence (c).

6. An isolated DNA sequence according to claim 4 having a nucleic acid sequence
(c) which has at least 60% homology with any of the sequences of (a) or (b), or
(d) which is capable of hybridising under stringent conditions to said sequence (c).

7. A purified and isolated polypeptide,
(a) having an amino acid sequence as shown in SEQ ID NO: 1B or SEQ ID NO: 2B, or
(b) having at least 30% homology with the amino acid sequence SEQ ID NO: 1B or at least 40% homology with the amino acid sequence SEQ ID NO: 2B, or
(c) being a fragment of polypeptide (a) or (b), which polypeptide or fragment of polypeptide has alcohol acyl transferase activity and is involved in the biosynthetic pathway for aliphatic and/or aromatic ester production in fruit.

8. A purified and isolated polypeptide according to claim 7 having at least 50% homology with any of the amino acid sequences SEQ ID NO: 1B or SEQ ID NO: 2B, or a fragment thereof.

9. A purified and isolated polypeptide according to claims 7 or 8 having at least 70% homology with any of the amino acid sequences SEQ ID NO: 1B or SEQ ID NO: 2B, or a fragment thereof.

10. A recombinant expression vector comprising a coding sequence which is operably linked to a promoter sequence capable of directing expression of said coding sequence in a host cell for said vector, and a transcription termination sequence, in which the coding sequence is a DNA sequence as defined in any of claims 1 to 6.

11. A replicative cloning vector comprising an isolated DNA sequence as defined in any of claims 1 to 6 and a replicon operative in a host cell for said vector.

12. A method for regulating aliphatic and/or aromatic ester formation in fruit, comprising inserting into the genome of a fruit-producing plant one or more copies of one or more DNA sequences as defined in any of claims 1 to 6.

13. A method as claimed in claim 12 in which the plant is strawberry, citrus (lemon), banana, apple, pear, melon, tomato, sweet pepper, peach or mango.

14. A plant and propagating material thereof which contains within its genome a vector as defined in claim 10 or 11.

15. A genetically modified strawberry or lemon plant and propagating material derived therefrom which has a genome comprising an expression vector for over-expression or down-regulation of an endogenous strawberry or lemon plant gene counterpart of any of the DNA sequences as defined in claims 1 to 6.

16. A method for producing aromatic and/or aliphatic esters in microorganisms, plant cells or plants comprising inserting into the genome of the microorganism or plant one or more copies of DNA sequences as defined in any of claims 1 to 6, and feeding an alcohol and an acyl-CoA to the microorganism or plant (cell).

17. An isolated DNA sequence encoding
(a) a polypetide having an amino acid sequence as shown in SEQ ID NO: 3B, or
(b) a polypeptide having at least 80% homology with the amino acid sequence SEQ ID NO: 3B, or
(c) a fragment of polypeptide (a) or (b),
which polypeptide or fragment of polypeptide has aminotransferase activity and is involved in the biosynthetic pathway for aliphatic and/or aromatic ester production in fruit.

18. An isolated DNA sequence having a nucleic acid sequence
(a) as shown in SEQ ID NO: 3A which encodes a polypeptide having aminotransferase activity and being involved in the biosynthetic pathway for aliphatic and/or aromatic ester production in fruit, or
(b) complementary to SEQ ID NO: 3A, or
(c) which has at least 70 % homology with any of the sequences of (a) or (b), or
(d) which is capable of hybridising under stringent conditions to any of the sequences (a) - (c).

19. A purified and isolated polypeptide,
(a) having an amino acid sequence as shown in SEQ ID NO: 3B, or
(b) having at least 80% homology with the amino acid sequence SEQ ID NO: 3B, or
(c) being a fragment of polypeptide (a) or (b), which polypeptide or fragment of polypeptide has aminotransferase activity and is involved in the biosynthetic pathway for aliphatic and/or aromatic ester production in fruit.

20. An isolated DNA sequence encoding
(a) a polypeptide having an amino acid sequence as shown in SEQ ID NO: 4B, or
(b) a polypeptide having at least 90% homology with the amino acid sequence SEQ ID NO: 4B, or
(c) a fragment of polypeptide (a) or (b),
which polypeptide or fragment of polypeptide has thiolase activity and is involved in the biosynthetic pathway for aliphatic and/or aromatic ester production in fruit.

21. An isolated DNA sequence having a nucleic acid sequence
(a) as shown in SEQ ID NO: 4A which encodes a polypeptide having thiolase activity and being involved in the biosynthetic pathway for aliphatic and/or aromatic ester production in fruit, or
(b) complementary to SEQ ID NO: 4A, or
(c) which has at least 75 % homology with any of the sequences of (a) or (b), or
(d) which is capable of hybridising under stringent conditions to any of the sequences (a) - (c).

22. A purified and isolated polypeptide,
(a) having an amino acid sequence as shown in SEQ ID NO: 4B, or
(b) having at least 90% homology with the amino acid sequence SEQ ID NO: 4B, or
(c) being a fragment of polypeptide (a) or (b), which polypeptide or fragment of polypeptide has thiolase activity and is involved in the biosynthetic pathway for aliphatic and/or aromatic ester production in fruit.

23. An isolated DNA sequence encoding
(a) a polypetide having an amino acid sequence as shown in SEQ ID NO: 5B, or
(b) a polypeptide having at least 90% homology with the amino acid sequence SEQ ID NO: 5B, or
(c) a fragment of polypeptide (a) or (b),
which polypeptide or fragment of polypeptide has pyruvate decarboxylase activity and is involved in the biosynthetic pathway for aliphatic and/or aromatic ester production in fruit.

24. An isolated DNA sequence having a nucleic acid sequence
(a) as shown in SEQ ID NO: 5A which encodes a polypeptide having pyruvate decarboxylase activity and being involved in the biosynthetic pathway for aliphatic and/or aromatic ester production in fruit, or
(b) complementary to SEQ ID NO: 5A, or
(c) which has at least 75 % homology with any of the sequences of (a) or (b), or
(d) which is capable of hybridising under stringent conditions to any of the sequences (a) - (c).

25. A purified and isolated polypeptide,
(a) having an amino acid sequence as shown in SEQ ID NO: 5B, or
(b) having at least 90% homology with the amino acid sequence SEQ ID NO: 5B, or
(c) being a fragment of polypeptide (a) or (b), which polypeptide or fragment of polypeptide has pyruvate decarboxylase activity and is involved in the biosynthetic pathway for aliphatic and/or aromatic ester production in fruit.

26. An isolated DNA sequence encoding
(a) a polypetide having an amino acid sequence as shown in SEQ ID NO: 6B, or
(b) a polypeptide having at least 75% homology with the amino acid sequence SEQ ID NO: 6B, or
(c) a fragment of polypeptide (a) or (b),
which polypeptide or fragment of polypeptide has alcohol dehydrogenase activity and is involved in the biosynthetic pathway for aliphatic and/or aromatic ester production in fruit.

27. An isolated DNA sequence having a nucleic acid sequence
(a) as shown in SEQ ID NO: 6A which encodes a polypeptide having alcohol dehydrogenase activity and being involved in the biosynthetic pathway for aliphatic and/or aromatic ester production in fruit, or
(b) complementary to SEQ ID NO: 6A, or
(c) which has at least 65 % homology with any of the sequences of (a) or (b), or
(d) which is capable of hybridising under stringent conditions to any of the sequences (a) - (c).

28. A purified and isolated polypeptide,
(a) having an amino acid sequence as shown in SEQ ID NO: 6B, or
(b) having at least 75% homology with the amino acid sequence SEQ ID NO: 6B, or
(c) being a fragment of polypeptide (a) or (b), which polypeptide or fragment of polypeptide has alcohol dehydrogenase activity and is involved in the biosynthetic pathway for aliphatic and/or aromatic ester production in fruit.

29. An isolated DNA sequence encoding
(a) a polypeptide having an amino acid sequence selected from the group consisting of sequences SEQ ID NO: 7B, 8B, 9B and 10B, or
(b) a polypeptide having at least
i) 55% homology with the amino acid sequence of a 326 aa fragment from the C terminal end of the coding sequence of SEQ ID NO: 7B, or
ii) 75% homology with the amino acid sequence of a 278 aa fragment from the C terminal end of the coding sequence of of SEQ ID NO: 8B, or
iii) 65% homology with the amino acid sequence of a 284 aa fragment from the C terminal end of the coding sequence of SEQ ID NO: 9B, or
iv) 80% homology with the amino acid sequence of a 188 aa fragment from the C terminal end of the coding sequence of SEQ ID NO: 10B, or
(c) a fragment of polypeptide (a) or (b),
which polypeptide or fragment of polypeptide has alcohol dehydrogenase activity and is involved in the biosynthetic pathway for aliphatic and/or aromatic ester production in fruit.

30. An isolated DNA sequence having a nucleic acid sequence
(a) selected from the group consisting of sequences SEQ ID NO: 7A, 8A, 9A and 10A, which encodes a polypeptide having alcohol dehydrogenase activity and being involved in the biosynthetic pathway for aliphatic and/or aromatic ester production in fruit, or
(b) complementary to SEQ ID NO: 7A, 8A, 9A or 10A, or
(c) which has at least 55% homology with any of the sequences of (a) or (b), or
(d) which is capable of hybridising under stringent conditions to any of the sequences (a) - (c).

31. An isolated DNA sequence according to claim 30 having a nucleic acid sequence
(c) which has at least 65% homology with any of the sequences of (a) or (b), or
(d) which is capable of hybridising under stringent conditions to said sequence (c).

32. A purified and isolated polypeptide,
(a) having an amino acid sequence selected from the group consisting of sequences SEQ ID NO: 7B, 8B, 9B and 10B, or
(b) having at least
i) 55% homology with the amino acid sequence of a 326 aa fragment from the C terminal end of the coding sequence of of SEQ ID NO: 7B
ii) 75% homology with the amino acid sequence of a 278 aa fragment from the C terminal end of the coding sequence of SEQ ID NO: 8B, or
iii) 65% homology with the amino acid sequence of a 284 aa fragment from the C terminal end of the coding sequence of SEQ ID NO: 9B, or
iv) 30% homology with the amino acid sequence of a 188 aa fragment from the C terminal end of the coding sequence of SEQ ID NO: 10B, or
(c) being a fragment of polypeptide (a) or (b), which polypeptide or fragment of polypeptide has alcohol dehydrogenase activity and is involved in the biosynthetic pathway for aliphatic and/or aromatic ester production in fruit.

33. An isolated DNA sequence encoding
(a) a polypeptide having an amino acid sequence as shown in SEQ ID NO: 11B, or
(b) a polypeptide having at least 75% homology with the amino acid sequence of a 181 aa fragment from the C terminal end of the coding sequence of SEQ ID NO: 11B, or
(c) a fragment of polypeptide (a) or (b),
which polypeptide or fragment of polypeptide has alcohol dehydrogenase activity and is involved in the biosynthetic pathway for aliphatic and/or aromatic ester production in fruit.

34. An isolated DNA sequence having a nucleic acid sequence
(a) as shown in SEQ ID NO: 11A which encodes a polypeptide having alcohol dehydrogenase activity and being involved in the biosynthetic pathway for aliphatic and/or aromatic ester production in fruit, or
(b) complementary to SEQ ID NO: 11A, or
(c) which has at least 48 % homology with any of the sequences of (a) or (b), or
(d) which is capable of hybridising under stringent conditions to any of the sequences (a) - (c).

35. A purified and isolated polypeptide,
(a) having an amino acid sequence as shown in SEQ ID NO: 11B, or
(b) having at least 75% homology with the amino acid sequence of a 181 aa fragment from the 3' end of SEQ ID NO: 11B, or
(c) being a fragment of polypeptide (a) or (b), which polypeptide or fragment of polypeptide has alcohol dehydrogenase activity and is involved in the biosynthetic pathway for aliphatic and/or aromatic ester production in fruit.

36. An isolated DNA sequence encoding
(a) a polypeptide having an amino acid sequence selected from the group consisting of sequences SEQ ID NO: 12B and 13B, or
(b) a polypeptide having at least
i) 55% homology with the amino acid sequence of a 176 aa fragment from the C terminal end of the coding sequence of SEQ ID NO: 12B, or
ii) 35% homology with the amino acid sequence of a 284 aa fragment from the C terminal end of the coding sequence of SEQ ID NO: 13B, or
(c) a fragment of polypeptide (a) or (b),
which polypeptide or fragment of polypeptide has alcohol dehydrogenase activity and is involved in the biosynthetic pathway for aliphatic and/or aromatic ester production in fruit.

37. An isolated DNA sequence having a nucleic acid sequence
(a) selected from the group consisting of sequences SEQ ID NO: 12A and 13A, which encodes a polypeptide having alcohol dehydrogenase activity and being involved in the biosynthetic pathway for aliphatic and/or aromatic ester production in fruit, or
(b) complementary to SEQ ID NO: 12A or 13A, or
(c) which has at least 20% homology with any of the sequences of (a) or (b), or
(d) which is capable of hybridising under stringent conditions to any of the sequences (a) - (c).

38. An isolated DNA sequence according to claim 37 having a nucleic acid sequence
(c) which has at least 30% homology with any of the sequences of (a) or (b), or
(d) which is capable of hybridising under stringent conditions to said sequence (c).

39. A purified and isolated polypeptide,
(a) having an amino acid sequence selected from the group consisting of sequences SEQ ID NO: 12B and 13B, or
(b) having at least
i) 55% homology with the amino acid sequence of a 176 aa fragment from the C terminal end of the coding sequence of SEQ ID NO: 12B, or
ii) 35% homology with the amino acid sequence of a 284 aa fragment from the C terminal end of the coding sequence of SEQ ID NO: 13B, or
(c) being a fragment of polypeptide (a) or (b), which polypeptide or fragment of polypeptide has alcohol dehydrogenase activity and is involved in the biosynthetic pathway for aliphatic and/or aromatic ester production in fruit.

40. A method for regulating aliphatic and/or aromatic ester formation in fruit comprising inserting into the genome of a fruit-producing plant one or more copies of one or more DNA sequences as defined in any of claims 17, 18, 20, 21, 23, 24, 26, 27, 29, 30, 31, 33, 34, 36, 37 or 38.

41. A genetically modified strawberry or lemon plant and propagating material derived therefrom which has a genome comprising an expression vector for overexpression or downregulation of an endogenous strawberry or lemon plant gene counterpart of any of the DNA sequences as defined in claims 17, 18, 20, 21, 23, 24, 26, 27, 29, 30, 31, 33, 34, 36, 37 or 38.

42. A method for producing aromatic and/or aliphatic esters in microorganisms, plant cells or plants comprising inserting into the genome of the microorganism or plant one or more copies of DNA sequences as defined in any of claims 1 to 6, and one or more copies of DNA sequences as defined in any of claims 26, 27, 29, 30, 31, 33, 34, 36, 37 or 38, and feeding aldehydes and acyl-CoA to the microorganism or plant (cell).

43. A method for producing aromatic and/or aliphatic esters in microorganisms, plant cells or plants comprising inserting into the genome of the microorganism or plant one or more copies of DNA sequences as defined in any of claims 1 to 6, and one or more copies of DNA sequences as defined in any of claims 26, 27, 29, 30, 31, 33, 34, 36, 37 or 38, and one or more copies of DNA sequences as defined in any of claims 23 or 24, and feeding alpha-keto acids and acyl-CoA to the microorganism or plant (cell).

44. A method for producing aromatic and/or aliphatic esters in microorganisms, plant cells or plants comprising inserting into the genome of the microorganism or plant one or more copies of DNA sequences as defined in any of claims 1 to 6, and one or more copies of DNA sequences as defined in any of claims 26, 27, 29, 30, 31, 33, 34, 36, 37 or 38, and one or more copies of DNA sequences as defined in any of claims 23 or 24, and one or more copies of DNA sequences as defined in any of claims 17 or 18, and feeding amino acids and acyl-CoA to the microorganism or plant (cell).

45. A method for producing aromatic and/or aliphatic esters in microorganisms, plant cells or plants comprising inserting into the genome of the microorganism or plant one or more copies of DNA sequences as defined in any of claims 1 to 6, and one or more copies of DNA sequences as defined in any of claims 26, 27, 29, 30, 31, 33, 34, 36, 37 or 38, and one or more copies of DNA sequences as defined in any of claims 23 or 24, and one or more copies of DNA sequences as defined in any of claims 17 or 18, and one or more copies of DNA sequences as defined in any of claims 20 en 21, and feeding amino acids and fatty acids to the microorganism or plant (cell).

46. An isolated DNA sequence according to any of claims 1 to 6 obtainable by cloning using as a primer a nucleotide sequence encoding the amino acid sequence as set forth in SEQ ID NO: 14B.

47. An antibody capable of specifically binding one or more polypeptides as claimed in any of claims 7-9, 19, 22, 25, 28, 32, 35 and 39.

48. A diagnostic kit for screening fruit with specific reference to volatile aliphatic and/or aromatic ester compounds comprising
a) one or more polypeptides as claimed is any of claims 7-9, 19, 22, 25, 28, 32, 35 and 39, or
b) one or more DNA sequences as claimed in any of claims 1-6, 17, 18, 20, 21, 23, 24, 26, 27, 29, 30, 31, 33, 34, 36, 37 and 38, or
c) one or more antibodies as defined in claim 47.

49. A method for screening fruit
a) for fruit quality trait attributes, such as flavour, fragrance, aroma, scent, texture, shape, or
b) to distinguish between cultivars/varieties of fruits based on their volatile ester profiles, or
c) to monitor harvest time of fruit(s) for quality trait attributes, or
d) to monitor post-harvest fruit(s) for quality trait attributes, or
e) to monitor shelf-life of fruits, or
f) to monitor timing for application of pesticides based on volatile ester profiles, or
g) to monitor fruit resistance capacity based on their volatile ester profiles,
which comprises using the diagnostic kit as claimed in claim 48.


SEQUENCE LISTING

(1) GENERAL INFORMATION:

(i) APPLICANT:
(A) NAME: Centre for Plant Breeding and Reproduction
Research
(B) STREET: Droevendaalsesteeg 1
(C) CITY: Wageningen
(D) STATE: Gelderland
(E) COUNTRY: The Netherlands
(F) POSTAL CODE (ZIP): 6700 AA
(G) TELEPHONE: 31.317477000
(H) TELEFAX: 31.317418094

(ii) TITLE OF INVENTION: Fruit Flavour Related Genes And Use Thereof

(iii) NUMBER OF SEQUENCES: 27

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98204018.0

(2) INFORMATION FOR SEQ ID NO:1:

(i) SEQUENCE CHARACTERISTICS:
(A) LENGTH: 1632 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION:16..1371
(D) OTHER INFORMATION:/product= "Strawberry alcohol acyl
transferase"

(xi) SEQUENCE DESCRIPTION: SEQ ID NO:1:

ACCTACTTTG CCAAA ATG GAG AAA ATT GAG GTC AGT ATA AAT TCC AAA CAC 51
Met Glu Lys Ile Glu Val Ser Ile Asn Ser Lys His
1               5                   10

ACC ATC AAA CCA TCA ACT TCC TCT ACA CCA CTT CAG CCT TAC AAG CTT 99
Thr Ile Lys Pro Ser Thr Ser Ser Thr Pro Leu Gln Pro Tyr Lys Leu
        15                  20                  25

ACC CTC CTG GAC CAG CTC ACT CCT CCG GCG TAT GTC CCC ATC GTG TTC 147
Thr Leu Leu Asp Gln Leu Thr Pro Pro Ala Tyr Val Pro Ile Val Phe
    30                  35                  40

TTC TAC CCC ATT ACT GAC CAT GAC TTC AAT CTT CCT CAA ACC CTA GCT 195
Phe Tyr Pro Ile Thr Asp His Asp Phe Asn Leu Pro Gln Thr Leu Ala
45                  50                  55                  60

GAC TTA AGA CAA GCC CTT TCG GAG ACT CTC ACT TTG TAC TAT CCA CTC 243
Asp Leu Arg Gln Ala Leu Ser Glu Thr Leu Thr Leu Tyr Tyr Pro Leu
                65                  70                  75

TCT GGA AGG GTC AAA AAC AAC CTA TAC ATC GAT GAT TTT GAA GAA GGT 291
Ser Gly Arg Val Lys Asn Asn Leu Tyr Ile Asp Asp Phe Glu Glu Gly
            80                  85                  90

GTC CCA TAC CTT GAG GCT CGA GTG AAT TGT GAC ATG ACT GAT TTT CTA 339
Val Pro Tyr Leu Glu Ala Arg Val Asn Cys Asp Met Thr Asp Phe Leu
        95                  100                 105

AGG CTT CGG AAA ATC GAG TGC CTT AAT GAG TTT GTT CCA ATA AAA CCA 387
Arg Leu Arg Lys Ile Glu Cys Leu Asn Glu Phe Val Pro Ile Lys Pro
    110                 115                 120

TTT AGT ATG GAA GCA ATA TCT GAT GAG CGT TAC CCC TTG CTT GGA GTT 435
Phe Ser Met Glu Ala Ile Ser Asp Glu Arg Tyr Pro Leu Leu Gly Val
125                 130                 135                 140

CAA GTC AAC GTT TTC GAT TCT GGA ATA GCA ATC GGT GTC TCC GTC TCT 483
Gln Val Asn Val Phe Asp Ser Gly Ile Ala Ile Gly Val Ser Val Ser
                145                 150                 155

CAC AAG CTC ATC GAT GGA GGA ACG GCA GAC TGT TTT CTC AAG TCC TGG 531
His Lys Leu Ile Asp Gly Gly Thr Ala Asp Cys Phe Leu Lys Ser Trp
            160                 165                 170

GGT GCT GTT TTT CGA GGG TGT CGT GAA AAT ATC ATA CAT CCT AGT CTC 579
Gly Ala Val Phe Arg Gly Cys Arg Glu Asn Ile Ile His Pro Ser Leu
        175                 180                 185

TCT GAA GCA GCA TTG CTT TTC CCA CCG AGA GAT GAC TTG CCT GAA AAG 627
Ser Glu Ala Ala Leu Leu Phe Pro Pro Arg Asp Asp Leu Pro Glu Lys
    190                 195                 200

TAT GTC GAT CAG ATG GAA GCG TTA TGG TTT GCC GGA AAA AAA GTT GCT 675
Tyr Val Asp Gln Met Glu Ala Leu Trp Phe Ala Gly Lys Lys Val Ala
205                 210                 215                 220

ACA AGG AGA TTT GTA TTT GGT GTG AAA GCC ATA TCT TCA ATT CAA GAT 723
Thr Arg Arg Phe Val Phe Gly Val Lys Ala Ile Ser Ser Ile Gln Asp
                225                 230                 235

GAA GCG AAG AGC GAG TCC GTG CCC AAG CCA TCA CGA GTT CAT GCC GTC 771
Glu Ala Lys Ser Glu Ser Val Pro Lys Pro Ser Arg Val His Ala Val
            240                 245                 250

ACT GGT TTT CTC TGG AAA CAT CTA ATC GCT GCT TCT CGG GCA CTA ACA 819
Thr Gly Phe Leu Trp Lys His Leu Ile Ala Ala Ser Arg Ala Leu Thr
        255                 260                 265

TCA GGT ACT ACT TCA ACA AGA CTT TCT ATA GCG GCC CAG GCA GTG AAC 867
Ser Gly Thr Thr Ser Thr Arg Leu Ser Ile Ala Ala Gln Ala Val Asn
    270                 275                 280

TTA AGA ACA CGG ATG AAC ATG GAG ACA GTG TTG GAT AAT GCC ACT GGA 915
Leu Arg Thr Arg Met Asn Met Glu Thr Val Leu Asp Asn Ala Thr Gly
285                 290                 295                 300

AAC TTG TTC TGG TGG GCA CAG GCC ATA CTA GAG CTA AGT CAT ACA ACA 963
Asn Leu Phe Trp Trp Ala Gln Ala Ile Leu Glu Leu Ser His Thr Thr
                305                 310                 315

CCA GAG ATC AGT GAT CTT AAG CTG TGT GAC TTG GTT AAC TTG CTC AAT 1011
Pro Glu Ile Ser Asp Leu Lys Leu Cys Asp Leu Val Asn Leu Leu Asn
            320                 325                 330

GGA TCT GTC AAA CAA TGT AAC GGT GAT TAC TTT GAG ACT TTC AAG GGT 1059
Gly Ser Val Lys Gln Cys Asn Gly Asp Tyr Phe Glu Thr Phe Lys Gly
        335                 340                 345

AAA GAG GGA TAT GGA AGA ATG TGC GAG TAT CTA GAT TTT CAG AGG ACT 1107
Lys Glu Gly Tyr Gly Arg Met Cys Glu Tyr Leu Asp Phe Gln Arg Thr
    350                 355                 360

ATG AGT TCT ATG GAA CCA GCA CCG GAT ATT TAT TTA TTC TCG AGC TGG 1155
Met Ser Ser Met Glu Pro Ala Pro Asp Ile Tyr Leu Phe Ser Ser Trp
365                 370                 375                 380

ACT AAT TTT TTC AAC CCA CTT GAT TTT GGA TGG GGG AGG ACA TCA TGG 1203
Thr Asn Phe Phe Asn Pro Leu Asp Phe Gly Trp Gly Arg Thr Ser Trp
                385                 390                 395

ATT GGA GTT GCA GGA AAA ATT GAA TCT GCA AGT TGC AAG TTC ATA ATA 1251
Ile Gly Val Ala Gly Lys Ile Glu Ser Ala Ser Cys Lys Phe Ile Ile
            400                 405                 410

TTA GTT CCA ACA CAA TGC GGT TCT GGA ATT GAA GCG TGG GTG AAT CTA 1299
Leu Val Pro Thr Gln Cys Gly Ser Gly Ile Glu Ala Trp Val Asn Leu
        415                 420                 425

GAA GAA GAG AAA ATG GCT ATG CTA GAA CAA GAT CCC CAT TTT CTA GCG 1347
Glu Glu Glu Lys Met Ala Met Leu Glu Gln Asp Pro His Phe Leu Ala
    430                 435                 440

TTA GCA TCT CCA AAG ACC TTA ATT TAAAGATATT GATTAAGAAA GATTATGTGG 1401
Leu Ala Ser Pro Lys Thr Leu Ile
445                 450

CTCGTGCAAT GTTTCGATTT TGCAGTGAAT AAGGTTTAAA TTAGTTCACC AGCCAATCAA 1461

TAAAATGCAA GTATGATAGA CTTTGTCTAC GTATGTTATC CGAATGTGTT TCCATATGCT 1521

TGTAACCAAT ATAGCTCTTT ATTGTAACAA ATGCTCTATT AAGCTTCTAG CTATAAAGTT 1581

ATTTATCTAT TAAAAATAAA ACTATGGAAG TTTTACCAAA AAAAAAAAAA A 1632

(2) INFORMATION FOR SEQ ID NO:2:

(i) SEQUENCE CHARACTERISTICS:
(A) LENGTH: 452 amino acids
(B) TYPE: amino acid
(D) TOPOLOGY: linear

(ii) MOLECULE TYPE: protein
(xi) SEQUENCE DESCRIPTION: SEQ ID NO:2:

Met Glu Lys Ile Glu Val Ser Ile Asn Ser Lys His Thr Ile Lys Pro
1               5                   10                  15

Ser Thr Ser Ser Thr Pro Leu Gln Pro Tyr Lys Leu Thr Leu Leu Asp
            20                  25                  30

Gln Leu Thr Pro Pro Ala Tyr Val Pro Ile Val Phe Phe Tyr Pro Ile
        35                  40                  45

Thr Asp His Asp Phe Asn Leu Pro Gln Thr Leu Ala Asp Leu Arg Gln
    50                  55                  60

Ala Leu Ser Glu Thr Leu Thr Leu Tyr Tyr Pro Leu Ser Gly Arg Val
65                  70                  75                  80

Lys Asn Asn Leu Tyr Ile Asp Asp Phe Glu Glu Gly Val Pro Tyr Leu
                85                  90                  95

Glu Ala Arg Val Asn Cys Asp Met Thr Asp Phe Leu Arg Leu Arg Lys
            100                 105                 110

Ile Glu Cys Leu Asn Glu Phe Val Pro Ile Lys Pro Phe Ser Met Glu
        115                 120                 125

Ala Ile Ser Asp Glu Arg Tyr Pro Leu Leu Gly Val Gln Val Asn Val
    130                 135                 140

Phe Asp Ser Gly Ile Ala Ile Gly Val Ser Val Ser His Lys Leu Ile
145                 150                 155                 160

Asp Gly Gly Thr Ala Asp Cys Phe Leu Lys Ser Trp Gly Ala Val Phe
                165                 170                 175

Arg Gly Cys Arg Glu Asn Ile Ile His Pro Ser Leu Ser Glu Ala Ala
            180                 185                 190

Leu Leu Phe Pro Pro Arg Asp Asp Leu Pro Glu Lys Tyr Val Asp Gln
        195                 200                 205

Met Glu Ala Leu Trp Phe Ala Gly Lys Lys Val Ala Thr Arg Arg Phe
    210                 215                 220

Val Phe Gly Val Lys Ala Ile Ser Ser Ile Gln Asp Glu Ala Lys Ser
225                 230                 235                 240

Glu Ser Val Pro Lys Pro Ser Arg Val His Ala Val Thr Gly Phe Leu
                245                 250                 255

Trp Lys His Leu Ile Ala Ala Ser Arg Ala Leu Thr Ser Gly Thr Thr
            260                 265                 270

Ser Thr Arg Leu Ser Ile Ala Ala Gln Ala Val Asn Leu Arg Thr Arg
        275                 280                 285

Met Asn Met Glu Thr Val Leu Asp Asn Ala Thr Gly Asn Leu Phe Trp
    290                 295                 300

Trp Ala Gln Ala Ile Leu Glu Leu Ser His Thr Thr Pro Glu Ile Ser
305                 310                 315                 320

Asp Leu Lys Leu Cys Asp Leu Val Asn Leu Leu Asn Gly Ser Val Lys
                325                 330                 335

Gln Cys Asn Gly Asp Tyr Phe Glu Thr Phe Lys Gly Lys Glu Gly Tyr
            340                 345                 350

Gly Arg Met Cys Glu Tyr Leu Asp Phe Gln Arg Thr Met Ser Ser Met
        355                 360                 365

Glu Pro Ala Pro Asp Ile Tyr Leu Phe Ser Ser Trp Thr Asn Phe Phe
    370                 375                 380

Asn Pro Leu Asp Phe Gly Trp Gly Arg Thr Ser Trp Ile Gly Val Ala
385                 390                 395                 400

Gly Lys Ile Glu Ser Ala Ser Cys Lys Phe Ile Ile Leu Val Pro Thr
                405                 410                 415

Gln Cys Gly Ser Gly Ile Glu Ala Trp Val Asn Leu Glu Glu Glu Lys
            420                 425                 430

Met Ala Met Leu Glu Gln Asp Pro His Phe Leu Ala Leu Ala Ser Pro
        435                 440                 445

Lys Thr Leu Ile
    450

(2) INFORMATION FOR SEQ ID NO:3:

     (i) SEQUENCE CHARACTERISTICS:
          (A) LENGTH: 1613 base pairs
          (B) TYPE: nucleic acid
          (C) STRANDEDNESS: single
          (D) TOPOLOGY: linear

    (ii) MOLECULE TYPE: cDNA

    (ix) FEATURE:
          (A) NAME/KEY: CDS
          (B) LOCATION:125..1426
          (D) OTHER INFORMATION:/product= "Citrus limon alcohol
               acyl transferase"

    (xi) SEQUENCE DESCRIPTION: SEQ ID NO:3:

CTTATTTAAA AGTTCATCAA CAAATTGTTC TACCACTTAC CATTTCTCAT AGCTCTGCAA      60

GTTCGGATTT GACTCTTTCT CTTTTCCTCA TTCCGGCCGG TGTTGATAGT TACATTTTGG     120

CACA ATG AAA ATT CAC GTT AAG GAG TCA ACA ATT ATA CGC CCT GCT CAA      169
     Met Lys Ile His Val Lys Glu Ser Thr Ile Ile Arg Pro Ala Gln
       1               5                  10                  15

GAA ACA CCC AAG CAT CGC CTA CAA ATA TCC GAC CTA GAC ATG ATT GTG      217
Glu Thr Pro Lys His Arg Leu Gln Ile Ser Asp Leu Asp Met Ile Val
                 20                  25                  30

CCA TCC AAT TAC GTT CCC AGT GTG TAT TTC TAT CGG CGG TCC AGT GAC      265
Pro Ser Asn Tyr Val Pro Ser Val Tyr Phe Tyr Arg Arg Ser Ser Asp
             35                  40                  45

TGC ACC GAT TTT TTT GAA GTT GGT TTG CTG AAG AAG GCT CTG AGC GAA      313
Cys Thr Asp Phe Phe Glu Val Gly Leu Leu Lys Lys Ala Leu Ser Glu
         50                  55                  60

GTT CTT GTG CCG TTT TAC CCC GTT GCC GGA AGG TTG CAG AAG GAT GAA      361
Val Leu Val Pro Phe Tyr Pro Val Ala Gly Arg Leu Gln Lys Asp Glu
     65                  70                  75

AAT CGC AAA ATT GAG ATT CTA TGT AAC GGA GAG GGA GTT TTG TTT CTG      409
Asn Arg Lys Ile Glu Ile Leu Cys Asn Gly Glu Gly Val Leu Phe Leu
 80                  85                  90                  95

GAG GCC GAA ACA AGT TGT GGT ATT GAT GAT TTC GGT GAC TTC TCA CAA      457
Glu Ala Glu Thr Ser Cys Gly Ile Asp Asp Phe Gly Asp Phe Ser Gln
                100                 105                 110

GGC TCG AAA CTC CTG ACG CTT GTT CCA ACT GTT GGT GAT ACA AAG GAT      505
Gly Ser Lys Leu Leu Thr Leu Val Pro Thr Val Gly Asp Thr Lys Asp
            115                 120                 125

ATA TCC TCC CAT CCA CTC TTG ATG GCA CAG GTA ACT TAT TTC AAA TGT      553
Ile Ser Ser His Pro Leu Leu Met Ala Gln Val Thr Tyr Phe Lys Cys
        130                 135                 140

GGA GGC GTT TGT GTT GGA ACT AGA GTG AAT CAT ACA CTG GTA GAT GGA     601
Gly Gly Val Cys Val Gly Thr Arg Val Asn His Thr Leu Val Asp Gly
    145                 150                 155

GCT TCA GCG TAC CAT ATC ATC AAC TCA TGG GCG GAG ACG ACG CGT GGC     649
Ala Ser Ala Tyr His Ile Ile Asn Ser Trp Ala Glu Thr Thr Arg Gly
160                 165                 170                 175

GTT CCT ATT AGC ACT CAA CCA TTC TAT GAT CGG ACC ATA CTG AGT GTT     697
Val Pro Ile Ser Thr Gln Pro Phe Tyr Asp Arg Thr Ile Leu Ser Val
                180                 185                 190

GGG GTT CCA ACT TCT CCC AAA TTC CAT CAC ATT GAA TAT GAC CCG CCT     745
Gly Val Pro Thr Ser Pro Lys Phe His His Ile Glu Tyr Asp Pro Pro
            195                 200                 205

CCT TCC ATG AAC GCT CCT CCT ACC CAA AAT CCT GAA ATC ATT TCT ACC     793
Pro Ser Met Asn Ala Pro Pro Thr Gln Asn Pro Glu Ile Ile Ser Thr
        210                 215                 220

GCA ATC CTT AAC CTA TCA CTT GAT CAA ATC CAC ACC CTC AAA GAG AAA     841
Ala Ile Leu Asn Leu Ser Leu Asp Gln Ile His Thr Leu Lys Glu Lys
    225                 230                 235

TCT AAG ACA GAT CAT GAA CCC AAC GTC AAG TAT AGT AGG ATG GCG ATC     889
Ser Lys Thr Asp His Glu Pro Asn Val Lys Tyr Ser Arg Met Ala Ile
240                 245                 250                 255

CTA GCA GCA CAT ATC TGG CGT AGC ATG TGT AAA GCG CGC GGA TTA TCT     937
Leu Ala Ala His Ile Trp Arg Ser Met Cys Lys Ala Arg Gly Leu Ser
                260                 265                 270

GAT GAT CAA GTT AGC AAG TTA CAC TTT CCT ACA GAC GGA CGA CAG AGA     985
Asp Asp Gln Val Ser Lys Leu His Phe Pro Thr Asp Gly Arg Gln Arg
            275                 280                 285

TTG AAT CCA CCA CTC CCG CCT GGA TAT TTT GGA AAT GTA ATT TTC ACC    1033
Leu Asn Pro Pro Leu Pro Pro Gly Tyr Phe Gly Asn Val Ile Phe Thr
        290                 295                 300

ACG TCG TTG ACG GCT TCA TCG GGT GAT ATC CTA AGT GAA CCA TTG AAT    1081
Thr Ser Leu Thr Ala Ser Ser Gly Asp Ile Leu Ser Glu Pro Leu Asn
    305                 310                 315

CAT ACT GTT GAA AGA ATT CAA AAA GCA TTA AAG CGG ATG GAC GAT GAG    1129
His Thr Val Glu Arg Ile Gln Lys Ala Leu Lys Arg Met Asp Asp Glu
320                 325                 330                 335

TAT TTG AAA TCA GCA CTT GCT TAC CTA AAG CAA CAG CCT GAT TTA AAT    1177
Tyr Leu Lys Ser Ala Leu Ala Tyr Leu Lys Gln Gln Pro Asp Leu Asn
                340                 345                 350

GCT CTA CGG AAA GGA GGC CAC ATT TAC AAG TGC CCT AAC CTC AAT ATC    1225
Ala Leu Arg Lys Gly Gly His Ile Tyr Lys Cys Pro Asn Leu Asn Ile
            355                 360                 365

GTC AAT TTG GCA AAT ATG CCA ATG TAT GTT GCG AAT TTT GGA TGG GGC    1273
Val Asn Leu Ala Asn Met Pro Met Tyr Val Ala Asn Phe Gly Trp Gly
        370                 375                 380

CAG CCG ATA TTT GCG AGG ATC GTT AAC ACA TAT TAT GAA GGG ATA GCA    1321
Gln Pro Ile Phe Ala Arg Ile Val Asn Thr Tyr Tyr Glu Gly Ile Ala
    385                 390                 395

CAT ATT TAT CCA AGT CCG AGC AAT GAT GGG ACC TTG TCA GTG GTT ATA    1369
His Ile Tyr Pro Ser Pro Ser Asn Asp Gly Thr Leu Ser Val Val Ile
400                 405                 410                 415

AAC TCG GTA GCC GAT CAC ATG CAG CTG TTC AAG AAG TTC TTT TAC GAG 1417
Asn Ser Val Ala Asp His Met Gln Leu Phe Lys Lys Phe Phe Tyr Glu
                420                 425                 430

ATC TTT GAT TAAGGTATGA AAGACCTAGG TATTTTATAT TTTCTAGAAA 1466
Ile Phe Asp

TGTCACTTTT TTTTTTTTTT TTTTTTGGGG GCGCAAATGT TGTCTTACTT GGAATTTTAT 1526

ATATTTTAAT CCATGTTTTT ATGGAAGGCA GTGGTGTTGC AAAAAAAAAA AAAAAAAAAA 1586

AAAAAAAAAA AAAAAAAAAA AAAAAAA 1613

(2) INFORMATION FOR SEQ ID NO:4:

(i) SEQUENCE CHARACTERISTICS:
(A) LENGTH: 434 amino acids
(B) TYPE: amino acid
(D) TOPOLOGY: linear

(ii) MOLECULE TYPE: protein
(xi) SEQUENCE DESCRIPTION: SEQ ID NO:4:

Met Lys Ile His Val Lys Glu Ser Thr Ile Ile Arg Pro Ala Gln Glu
1               5                   10                  15

Thr Pro Lys His Arg Leu Gln Ile Ser Asp Leu Asp Met Ile Val Pro
            20                  25                  30

Ser Asn Tyr Val Pro Ser Val Tyr Phe Tyr Arg Arg Ser Ser Asp Cys
        35                  40                  45

Thr Asp Phe Phe Glu Val Gly Leu Leu Lys Lys Ala Leu Ser Glu Val
    50                  55                  60

Leu Val Pro Phe Tyr Pro Val Ala Gly Arg Leu Gln Lys Asp Glu Asn
65                  70                  75                  80

Arg Lys Ile Glu Ile Leu Cys Asn Gly Glu Gly Val Leu Phe Leu Glu
                85                  90                  95

Ala Glu Thr Ser Cys Gly Ile Asp Asp Phe Gly Asp Phe Ser Gln Gly
            100                 105                 110

Ser Lys Leu Leu Thr Leu Val Pro Thr Val Gly Asp Thr Lys Asp Ile
        115                 120                 125

Ser Ser His Pro Leu Leu Met Ala Gln Val Thr Tyr Phe Lys Cys Gly
    130                 135                 140

Gly Val Cys Val Gly Thr Arg Val Asn His Thr Leu Val Asp Gly Ala
145                 150                 155                 160

Ser Ala Tyr His Ile Ile Asn Ser Trp Ala Glu Thr Thr Arg Gly Val
                165                 170                 175

Pro Ile Ser Thr Gln Pro Phe Tyr Asp Arg Thr Ile Leu Ser Val Gly
            180                 185                 190

Val Pro Thr Ser Pro Lys Phe His His Ile Glu Tyr Asp Pro Pro Pro
        195                 200                 205

Ser Met Asn Ala Pro Pro Thr Gln Asn Pro Glu Ile Ile Ser Thr Ala
    210                 215                 220

Ile Leu Asn Leu Ser Leu Asp Gln Ile His Thr Leu Lys Glu Lys Ser
225                 230                 235                 240

Lys Thr Asp His Glu Pro Asn Val Lys Tyr Ser Arg Met Ala Ile Leu
                245                 250                 255

Ala Ala His Ile Trp Arg Ser Met Cys Lys Ala Arg Gly Leu Ser Asp
            260                 265                 270

Asp Gln Val Ser Lys Leu His Phe Pro Thr Asp Gly Arg Gln Arg Leu
        275                 280                 285

Asn Pro Pro Leu Pro Pro Gly Tyr Phe Gly Asn Val Ile Phe Thr Thr
    290                 295                 300

Ser Leu Thr Ala Ser Ser Gly Asp Ile Leu Ser Glu Pro Leu Asn His
305                 310                 315                 320

Thr Val Glu Arg Ile Gln Lys Ala Leu Lys Arg Met Asp Asp Glu Tyr
                325                 330                 335

Leu Lys Ser Ala Leu Ala Tyr Leu Lys Gln Gln Pro Asp Leu Asn Ala
            340                 345                 350

Leu Arg Lys Gly Gly His Ile Tyr Lys Cys Pro Asn Leu Asn Ile Val
        355                 360                 365

Asn Leu Ala Asn Met Pro Met Tyr Val Ala Asn Phe Gly Trp Gly Gln
    370                 375                 380

Pro Ile Phe Ala Arg Ile Val Asn Thr Tyr Tyr Glu Gly Ile Ala His
385                 390                 395                 400

Ile Tyr Pro Ser Pro Ser Asn Asp Gly Thr Leu Ser Val Val Ile Asn
                405                 410                 415

Ser Val Ala Asp His Met Gln Leu Phe Lys Lys Phe Phe Tyr Glu Ile
            420                 425                 430

Phe Asp

(2) INFORMATION FOR SEQ ID NO:5:

(i) SEQUENCE CHARACTERISTICS:
(A) LENGTH: 1586 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION:78..1268
(D) OTHER INFORMATION:/product= "Strawberry
aminotransferase"

(xi) SEQUENCE DESCRIPTION: SEQ ID NO:5:

AAACCGTCGG CGTCTGTAAA TGCGTCGCCG CTCCGGAGAA GACAGAGTAC AAGACTCAGG      60

TGAATCGCAA TGCCAAC ATG GCC AAG CTT CAA GCC GGT TAT CTT TTT CCA      110
                   Met Ala Lys Leu Gln Ala Gly Tyr Leu Phe Pro
                     1               5                  10

GAG ATT GCG AGG AGG AGG AAT GCG CAC TTG CAG AAG CAC CCT GAT GCG 158
Glu Ile Ala Arg Arg Arg Asn Ala His Leu Gln Lys His Pro Asp Ala
            15                  20                  25

AAG ATA ATT CCA CTT GGA ATT GGT GAT ACT ACC GAG CCA ATT CCA GAA 206
Lys Ile Ile Pro Leu Gly Ile Gly Asp Thr Thr Glu Pro Ile Pro Glu
        30                  35                  40

TAT ATA ACC TCT GCA ATG GCA AAG AGA GCA CTT GCC ATG TCC ACC CTA 254
Tyr Ile Thr Ser Ala Met Ala Lys Arg Ala Leu Ala Met Ser Thr Leu
    45                  50                  55

GAG GGT TAC AGT GGT TAT GGA CCT GAA CAA GGT GAA AAG CCA CTG AGA 302
Glu Gly Tyr Ser Gly Tyr Gly Pro Glu Gln Gly Glu Lys Pro Leu Arg
60                  65                  70                  75

GTT GCA ATT GCT AAA ACG TTT TAT GGC GAC CTT GGC ATA GAG GAA GAT 350
Val Ala Ile Ala Lys Thr Phe Tyr Gly Asp Leu Gly Ile Glu Glu Asp
                80                  85                  90

GAC ATA TTT GTT TCT GAT GGG GCA AAA TGT GAC ATA TCC CGC CTT CAG 398
Asp Ile Phe Val Ser Asp Gly Ala Lys Cys Asp Ile Ser Arg Leu Gln
            95                  100                 105

GTT CTT TTT GGG GCG GAT AAA ACA ATA GCA GTG CAA GAT CCA TCG TAT 446
Val Leu Phe Gly Ala Asp Lys Thr Ile Ala Val Gln Asp Pro Ser Tyr
        110                 115                 120

CCG GCT TAT GTA GAC TCA AGT GTT ATT ATG GGC CAG ACA GGA CAG TAT 494
Pro Ala Tyr Val Asp Ser Ser Val Ile Met Gly Gln Thr Gly Gln Tyr
    125                 130                 135

CAG AAA TCT GTT CAG AAG TTT GGA AAC ATC GAG TAC ATG AGG TGT ACT 542
Gln Lys Ser Val Gln Lys Phe Gly Asn Ile Glu Tyr Met Arg Cys Thr
140                 145                 150                 155

CCC GAT AAT GGA TTT TTT CCT GAT CTG TCC TCT ACT AAG CGA ACA GAT 590
Pro Asp Asn Gly Phe Phe Pro Asp Leu Ser Ser Thr Lys Arg Thr Asp
                160                 165                 170

ATC ATA TTT TTC TGT TCA CCA AAC AAT CCT ACT GGT TCT GCT GCA ACA 638
Ile Ile Phe Phe Cys Ser Pro Asn Asn Pro Thr Gly Ser Ala Ala Thr
            175                 180                 185

AGG GAG CAA CTG ACA CAA CTT GTA AAG TTT GCC AAG GAT AAT GGT TCA 686
Arg Glu Gln Leu Thr Gln Leu Val Lys Phe Ala Lys Asp Asn Gly Ser
        190                 195                 200

ATC ATA GTC TAT GAT TCT GCA TAT GCC ATG TAT ATG TCA GAT GAT AAT 734
Ile Ile Val Tyr Asp Ser Ala Tyr Ala Met Tyr Met Ser Asp Asp Asn
    205                 210                 215

CCA CGC TCC ATC TTT GAA ATC CCT GGA GCT AAA GAT GTT GCA CTT GAG 782
Pro Arg Ser Ile Phe Glu Ile Pro Gly Ala Lys Asp Val Ala Leu Glu
220                 225                 230                 235

ACA TCA TCA TTT AGT AAG TAT GCC GGA TTC ACT GGA GTT CGT TTG GGG 830
Thr Ser Ser Phe Ser Lys Tyr Ala Gly Phe Thr Gly Val Arg Leu Gly
                240                 245                 250

TGG ACT GTG GTT CCA AAG CAG TTG CAG TAT TCA GAT GGT TTT CAA GTT 878
Trp Thr Val Val Pro Lys Gln Leu Gln Tyr Ser Asp Gly Phe Gln Val
            255                 260                 265

GCC AAG GAT TTC AAC CGC ATT GTT TGT ACT TGC TTC AAT GGT GCA TCC 926
Ala Lys Asp Phe Asn Arg Ile Val Cys Thr Cys Phe Asn Gly Ala Ser
        270                 275                 280

ACT ATT ATC CAA GCT GGT GGT CTG GCT TGC CTT CAA CCA AAG GGT GTT 974
Thr Ile Ile Gln Ala Gly Gly Leu Ala Cys Leu Gln Pro Lys Gly Val
    285                 290                 295

AAG GCT ATG CAC GGT GTG ATA AAT TTC TAC AAA GAA AAT ACT AAG ATC 1022
Lys Ala Met His Gly Val Ile Asn Phe Tyr Lys Glu Asn Thr Lys Ile
300                 305                 310                 315

ATA ATG GAG ACG TTT AAC TCT CTT GGC TTT AAC GTG TAT GGA GGG ACA 1070
Ile Met Glu Thr Phe Asn Ser Leu Gly Phe Asn Val Tyr Gly Gly Thr
                320                 325                 330

AAC GCT CCA TAT GTG TGG GTC CAC TTC CCT GGA CAA AGC TCC TGG GAT 1118
Asn Ala Pro Tyr Val Trp Val His Phe Pro Gly Gln Ser Ser Trp Asp
            335                 340                 345

GTG TTT GCT GAG ATC CTT GAG AAG ACT CAT GTG GTA ACC ACA CCT GGA 1166
Val Phe Ala Glu Ile Leu Glu Lys Thr His Val Val Thr Thr Pro Gly
        350                 355                 360

AGT GGC TTT GGA CCT GGT GGT GAA GGT TTC ATC AGG GTA AGT GCC TTT 1214
Ser Gly Phe Gly Pro Gly Gly Glu Gly Phe Ile Arg Val Ser Ala Phe
    365                 370                 375

GGA CAC AGG AAA AAT ATA TTA GAA GCA TGT AAA AGA TTC AAG CAA TTA 1262
Gly His Arg Lys Asn Ile Leu Glu Ala Cys Lys Arg Phe Lys Gln Leu
380                 385                 390                 395

TAC AAG TGAGGACTGC GGATCTGAAT TGTAGACCAG TTTCTACTGC ATGCTAGTTG 1318
Tyr Lys

AACCTATTTG CCTCCCATTT CCGTTCTATG CTAAATATTT TAGCACGTTC CAATTCCGTA 1378

TTCAGTTTGT CGGCTTTAGT TTATGAATTA TGGAGATTTT AGCTATTGTA AAAATGATTC 1438

GATCAGCCTT GTTTTCATGT GTTACACTTA ATTGTTGTAA CATTTGTGAG GATCAGAAGC 1498

TTTGATTCTG TTTGCTAGAA TAGTATAATT TTACCTAAAT AAAGTGGTTG ATCTTTCTTG 1558

GCCTGCAAAA AAAAAAAAAA AAAAAAAA 1586

(2) INFORMATION FOR SEQ ID NO:6:

(i) SEQUENCE CHARACTERISTICS:
(A) LENGTH: 397 amino acids
(B) TYPE: amino acid
(D) TOPOLOGY: linear

(ii) MOLECULE TYPE: protein
(xi) SEQUENCE DESCRIPTION: SEQ ID NO:6:

Met Ala Lys Leu Gln Ala Gly Tyr Leu Phe Pro Glu Ile Ala Arg Arg
1               5                   10                  15

Arg Asn Ala His Leu Gln Lys His Pro Asp Ala Lys Ile Ile Pro Leu
            20                  25                  30

Gly Ile Gly Asp Thr Thr Glu Pro Ile Pro Glu Tyr Ile Thr Ser Ala
        35                  40                  45

Met Ala Lys Arg Ala Leu Ala Met Ser Thr Leu Glu Gly Tyr Ser Gly
    50                  55                  60

Tyr Gly Pro Glu Gln Gly Glu Lys Pro Leu Arg Val Ala Ile Ala Lys
65                  70                  75                  80

Thr Phe Tyr Gly Asp Leu Gly Ile Glu Glu Asp Asp Ile Phe Val Ser
                85                  90                  95

Asp Gly Ala Lys Cys Asp Ile Ser Arg Leu Gln Val Leu Phe Gly Ala
            100                 105                 110

Asp Lys Thr Ile Ala Val Gln Asp Pro Ser Tyr Pro Ala Tyr Val Asp
        115                 120                 125

Ser Ser Val Ile Met Gly Gln Thr Gly Gln Tyr Gln Lys Ser Val Gln
    130                 135                 140

Lys Phe Gly Asn Ile Glu Tyr Met Arg Cys Thr Pro Asp Asn Gly Phe
145                 150                 155                 160

Phe Pro Asp Leu Ser Ser Thr Lys Arg Thr Asp Ile Ile Phe Phe Cys
                165                 170                 175

Ser Pro Asn Asn Pro Thr Gly Ser Ala Ala Thr Arg Glu Gln Leu Thr
            180                 185                 190

Gln Leu Val Lys Phe Ala Lys Asp Asn Gly Ser Ile Ile Val Tyr Asp
        195                 200                 205

Ser Ala Tyr Ala Met Tyr Met Ser Asp Asp Asn Pro Arg Ser Ile Phe
    210                 215                 220

Glu Ile Pro Gly Ala Lys Asp Val Ala Leu Glu Thr Ser Ser Phe Ser
225                 230                 235                 240

Lys Tyr Ala Gly Phe Thr Gly Val Arg Leu Gly Trp Thr Val Val Pro
                245                 250                 255

Lys Gln Leu Gln Tyr Ser Asp Gly Phe Gln Val Ala Lys Asp Phe Asn
            260                 265                 270

Arg Ile Val Cys Thr Cys Phe Asn Gly Ala Ser Thr Ile Ile Gln Ala
        275                 280                 285

Gly Gly Leu Ala Cys Leu Gln Pro Lys Gly Val Lys Ala Met His Gly
    290                 295                 300

Val Ile Asn Phe Tyr Lys Glu Asn Thr Lys Ile Ile Met Glu Thr Phe
305                 310                 315                 320

Asn Ser Leu Gly Phe Asn Val Tyr Gly Gly Thr Asn Ala Pro Tyr Val
                325                 330                 335

Trp Val His Phe Pro Gly Gln Ser Ser Trp Asp Val Phe Ala Glu Ile
            340                 345                 350

Leu Glu Lys Thr His Val Val Thr Thr Pro Gly Ser Gly Phe Gly Pro
        355                 360                 365

Gly Gly Glu Gly Phe Ile Arg Val Ser Ala Phe Gly His Arg Lys Asn
    370                 375                 380

Ile Leu Glu Ala Cys Lys Arg Phe Lys Gln Leu Tyr Lys
385                 390                 395

(2) INFORMATION FOR SEQ ID NO:7:

     (i) SEQUENCE CHARACTERISTICS:
          (A) LENGTH: 1775 base pairs
          (B) TYPE: nucleic acid
          (C) STRANDEDNESS: single
          (D) TOPOLOGY: linear

(ii) MOLECULE TYPE: cDNA

(ix) FEATURE:
(A) NAME/KEY: CDS
(B) LOCATION:37..1410
(D) OTHER INFORMATION:/product= "Strawberry thiolase"

(xi) SEQUENCE DESCRIPTION: SEQ ID NO:7:

CGCTCCTTTG ATTTCCTTGT TTCAATTATC AAGAGT ATG GAG AAA GCG ATC AAC 54
Met Glu Lys Ala Ile Asn
1               5

AGG CAG AAG GTT CTC CTC GAC CAT CTC CGA CCT TCT TCT TCT TCC GAC 102
Arg Gln Lys Val Leu Leu Asp His Leu Arg Pro Ser Ser Ser Ser Asp
            10                  15                  20

GAC TCT TCT CTC TCC GCG TCG GTA TGT GCG GCT GGG GAT AGC GCT GCG 150
Asp Ser Ser Leu Ser Ala Ser Val Cys Ala Ala Gly Asp Ser Ala Ala
        25                  30                  35

TAT GCT AGG AAT CAT GTC TTT GGG GAC GAT GTC GTC ATC GTT GCA GCT 198
Tyr Ala Arg Asn His Val Phe Gly Asp Asp Val Val Ile Val Ala Ala
    40                  45                  50

TTT CGC ACT CCA CTC TGC AAG GCT AAG CGT GGC GGC TTC AAG TAT ACT 246
Phe Arg Thr Pro Leu Cys Lys Ala Lys Arg Gly Gly Phe Lys Tyr Thr
55                  60                  65                  70

TAT GCT GAT GAT CTC CTC GCA CCT GTC CTC AAG GCC GTG GTT GAG AAA 294
Tyr Ala Asp Asp Leu Leu Ala Pro Val Leu Lys Ala Val Val Glu Lys
                75                  80                  85

ACC AAT CTC AAT CCC AAG GAA GTC GGG GAT ATT GTT GTC GGT ACC GTC 342
Thr Asn Leu Asn Pro Lys Glu Val Gly Asp Ile Val Val Gly Thr Val
            90                  95                  100

TTG GCC CCA GGA TCT CAG AGA GCT AGC GAA TGC AGG ATG GCT GCT TTC 390
Leu Ala Pro Gly Ser Gln Arg Ala Ser Glu Cys Arg Met Ala Ala Phe
        105                 110                 115

TAT GCT GGC TTC CCT GAG ACT GTG CCG GTT AGA ACT GTG AAC AGA CAA 438
Tyr Ala Gly Phe Pro Glu Thr Val Pro Val Arg Thr Val Asn Arg Gln
    120                 125                 130

TGT TCG TCT GGC CTC CAA GCA GTT GCT GAT GTT GCT GCT GCC ATT AGA 486
Cys Ser Ser Gly Leu Gln Ala Val Ala Asp Val Ala Ala Ala Ile Arg
135                 140                 145                 150

GCA GGG TTT TAT GAT ATT GGC ATT GGT GCT GGT TTG GAA TCC ATG ACT 534
Ala Gly Phe Tyr Asp Ile Gly Ile Gly Ala Gly Leu Glu Ser Met Thr
                155                 160                 165

GCA AAC CCA ATG GCA TGG GAA GGG GAT GTT AAT CCT AAA GTA AAG ATC 582
Ala Asn Pro Met Ala Trp Glu Gly Asp Val Asn Pro Lys Val Lys Ile
            170                 175                 180

TTT GAA CAA GCC CAG AAT TGC CTT CTT CCT ATG GGA GTC ACC TCA GAA 630
Phe Glu Gln Ala Gln Asn Cys Leu Leu Pro Met Gly Val Thr Ser Glu
        185                 190                 195

AAT GTT GCT CAT CGT TTT GGT GTT TCA AGA CAG GAG CAA GAT CAG GCT 678
Asn Val Ala His Arg Phe Gly Val Ser Arg Gln Glu Gln Asp Gln Ala
    200                 205                 210

GCA GTT GAC TCT CAT AGA AAG GCA GCT GCT GCT GCT GCT GCT GGT AGA     726
Ala Val Asp Ser His Arg Lys Ala Ala Ala Ala Ala Ala Ala Gly Arg
215                 220                 225                 230

TTT AAA GAT GAA ATC ATC CCT GTG GCA ACC AAG ATT GTT GAT CCA AAA     774
Phe Lys Asp Glu Ile Ile Pro Val Ala Thr Lys Ile Val Asp Pro Lys
                235                 240                 245

TCT GGT GAT GAG AAA CCT GTT ACA ATC TCT GTT GAT GAT GGG ATT CGA     822
Ser Gly Asp Glu Lys Pro Val Thr Ile Ser Val Asp Asp Gly Ile Arg
            250                 255                 260

AAC ACA ACA TTG GCG GAC CTA GCA AAG CTG AAG CCT GTG TTT AAG AAA     870
Asn Thr Thr Leu Ala Asp Leu Ala Lys Leu Lys Pro Val Phe Lys Lys
        265                 270                 275

GAT GGG ACC ACC ACT GCT GGT AAT TCT AGT CAA GTT AGT GAT GGT GCT     918
Asp Gly Thr Thr Thr Ala Gly Asn Ser Ser Gln Val Ser Asp Gly Ala
    280                 285                 290

GGA GCT GTT CTC TTG ATG AAG AGA AGT GTT GCC GAC CAA AAA GGA TTG     966
Gly Ala Val Leu Leu Met Lys Arg Ser Val Ala Asp Gln Lys Gly Leu
295                 300                 305                 310

CCG ATT CTT GGT GTA TTC AGG AAT TTT GTT GCT GTT GGT GTG GAT CCT     1014
Pro Ile Leu Gly Val Phe Arg Asn Phe Val Ala Val Gly Val Asp Pro
                315                 320                 325

GCC ATC ATG GGT GTT GGC CCA GCT GCT GCA ATT CCA GTT GCA GTT AAG     1062
Ala Ile Met Gly Val Gly Pro Ala Ala Ala Ile Pro Val Ala Val Lys
            330                 335                 340

GCA GCT GGT TTA GAG CTT GAT GAT ATT GAC CTT TTT GAG ATA AAT GAG     1110
Ala Ala Gly Leu Glu Leu Asp Asp Ile Asp Leu Phe Glu Ile Asn Glu
        345                 350                 355

GCT TTT GCA TCC CAA TTT GTG TAT TGC CGT AAC AAG CTG GGA CTT GAT     1158
Ala Phe Ala Ser Gln Phe Val Tyr Cys Arg Asn Lys Leu Gly Leu Asp
    360                 365                 370

CCA GAA AAA ATC AAT GTT AAC GGA GGT GCA ATG GCC ATC GGC CAT CCA     1206
Pro Glu Lys Ile Asn Val Asn Gly Gly Ala Met Ala Ile Gly His Pro
375                 380                 385                 390

CTT GGT GCA ACA GGT GCC CGG TGT GTT GCC ACT CTT TTG CAT GAG ATG     1254
Leu Gly Ala Thr Gly Ala Arg Cys Val Ala Thr Leu Leu His Glu Met
                395                 400                 405

AAG CGT CGT GGT AAA GAC TGC CGC TAT GGA GTG ATC TCA ATG TGC ATA     1302
Lys Arg Arg Gly Lys Asp Cys Arg Tyr Gly Val Ile Ser Met Cys Ile
            410                 415                 420

GGC ACA GGG ATG GGT GCA GCC GCT GTT TTT GAA AGA GGA GAC CGG ACC     1350
Gly Thr Gly Met Gly Ala Ala Ala Val Phe Glu Arg Gly Asp Arg Thr
        425                 430                 435

GAT GAA CTC TGC AAT GCT CGC AAG GTT GAA TCA CTC AAC TTC TTA TCC     1398
Asp Glu Leu Cys Asn Ala Arg Lys Val Glu Ser Leu Asn Phe Leu Ser
    440                 445                 450

AAG GAT GTT CGG TAGTAGAGAA TGGTTAGTGA CAGGAGCTAT TCCAATCAAT     1450
Lys Asp Val Arg
455

AATGTTTGGT GGAGTCTGAA AATCATAGTA AAGCACTGGA ATAACGTTGC TAAGTTTTTC     1510

GTTGGGTACT ACCTTGTTTA TTGGGATGGA ATACACATGT AGTTGGTTTG TTCTCCCAGA     1570

CCTCCCACTT GTTGGCATAT TCATTTTTGT CCAACCTAAA AAGTTCCATT TTATAGGACT 1630

TCATCTCAAT AACATTGGGT TTGCGCCACT AAAGCAGTGC CTAAAACTGT AATTGGGTAA 1690

TTTTGGTATA CCTGTTTGCT ACTTTTCTTT TCTAAGTTAA TCAAGCCCTG CCCACCTCAT 1750

ATAAAAAAAA AAAAAAAAAA AAAAA 1775

(2) INFORMATION FOR SEQ ID NO:8:

(i) SEQUENCE CHARACTERISTICS:
(A) LENGTH: 458 amino acids
(B) TYPE: amino acid
(D) TOPOLOGY: linear

(ii) MOLECULE TYPE: protein
(xi) SEQUENCE DESCRIPTION: SEQ ID NO:8:

Met Glu Lys Ala Ile Asn Arg Gln Lys Val Leu Leu Asp His Leu Arg
1               5                   10                  15

Pro Ser Ser Ser Ser Asp Asp Ser Ser Leu Ser Ala Ser Val Cys Ala
            20                  25                  30

Ala Gly Asp Ser Ala Ala Tyr Ala Arg Asn His Val Phe Gly Asp Asp
        35                  40                  45

Val Val Ile Val Ala Ala Phe Arg Thr Pro Leu Cys Lys Ala Lys Arg
    50                  55                  60

Gly Gly Phe Lys Tyr Thr Tyr Ala Asp Asp Leu Leu Ala Pro Val Leu
65                  70                  75                  80

Lys Ala Val Val Glu Lys Thr Asn Leu Asn Pro Lys Glu Val Gly Asp
                85                  90                  95

Ile Val Val Gly Thr Val Leu Ala Pro Gly Ser Gln Arg Ala Ser Glu
            100                 105                 110

Cys Arg Met Ala Ala Phe Tyr Ala Gly Phe Pro Glu Thr Val Pro Val
        115                 120                 125

Arg Thr Val Asn Arg Gln Cys Ser Ser Gly Leu Gln Ala Val Ala Asp
    130                 135                 140

Val Ala Ala Ala Ile Arg Ala Gly Phe Tyr Asp Ile Gly Ile Gly Ala
145                 150                 155                 160

Gly Leu Glu Ser Met Thr Ala Asn Pro Met Ala Trp Glu Gly Asp Val
                165                 170                 175

Asn Pro Lys Val Lys Ile Phe Glu Gln Ala Gln Asn Cys Leu Leu Pro
            180                 185                 190

Met Gly Val Thr Ser Glu Asn Val Ala His Arg Phe Gly Val Ser Arg
        195                 200                 205

Gln Glu Gln Asp Gln Ala Ala Val Asp Ser His Arg Lys Ala Ala Ala
    210                 215                 220

Ala Ala Ala Ala Gly Arg Phe Lys Asp Glu Ile Ile Pro Val Ala Thr
225                 230                 235                 240

Lys Ile Val Asp Pro Lys Ser Gly Asp Glu Lys Pro Val Thr Ile Ser
                245                 250                 255

Val Asp Asp Gly Ile Arg Asn Thr Thr Leu Ala Asp Leu Ala Lys Leu
            260                 265                 270

Lys Pro Val Phe Lys Lys Asp Gly Thr Thr Thr Ala Gly Asn Ser Ser
        275                 280                 285

Gln Val Ser Asp Gly Ala Gly Ala Val Leu Leu Met Lys Arg Ser Val
    290                 295                 300

Ala Asp Gln Lys Gly Leu Pro Ile Leu Gly Val Phe Arg Asn Phe Val
305                 310                 315                 320

Ala Val Gly Val Asp Pro Ala Ile Met Gly Val Gly Pro Ala Ala Ala
                325                 330                 335

Ile Pro Val Ala Val Lys Ala Ala Gly Leu Glu Leu Asp Asp Ile Asp
            340                 345                 350

Leu Phe Glu Ile Asn Glu Ala Phe Ala Ser Gln Phe Val Tyr Cys Arg
        355                 360                 365

Asn Lys Leu Gly Leu Asp Pro Glu Lys Ile Asn Val Asn Gly Gly Ala
    370                 375                 380

Met Ala Ile Gly His Pro Leu Gly Ala Thr Gly Ala Arg Cys Val Ala
385                 390                 395                 400

Thr Leu Leu His Glu Met Lys Arg Arg Gly Lys Asp Cys Arg Tyr Gly
                405                 410                 415

Val Ile Ser Met Cys Ile Gly Thr Gly Met Gly Ala Ala Ala Val Phe
            420                 425                 430

Glu Arg Gly Asp Arg Thr Asp Glu Leu Cys Asn Ala Arg Lys Val Glu
        435                 440                 445

Ser Leu Asn Phe Leu Ser Lys Asp Val Arg
    450                 455

(2) INFORMATION FOR SEQ ID NO:9:

     (i) SEQUENCE CHARACTERISTICS:
          (A) LENGTH: 2141 base pairs
          (B) TYPE: nucleic acid
          (C) STRANDEDNESS: single
          (D) TOPOLOGY: linear

    (ii) MOLECULE TYPE: cDNA


    (ix) FEATURE:
          (A) NAME/KEY: CDS
          (B) LOCATION:78..1892
          (D) OTHER INFORMATION:/product= "Strawberry pyruvate
                decarboxylase"


    (xi) SEQUENCE DESCRIPTION: SEQ ID NO:9:

ATTTTCACTC AGAGTCTCAA TCTTTCATCA CAAAAATTCC CATTTGATCA CAAAAAAGTT      60

TCAACCTTTA AACCTCC ATG GAC ACC AAG ATT GGC TCC ATC GAC GTC TGC        110
                   Met Asp Thr Lys Ile Gly Ser Ile Asp Val Cys
                     1               5                  10

AAA ACC GAG AAC CAC GAC GTC GGT TGT TTA CCA AAC AGC GCC ACC TCC 158
Lys Thr Glu Asn His Asp Val Gly Cys Leu Pro Asn Ser Ala Thr Ser
            15                  20                  25

ACC GTT CAA AAC TCA GTC CCT TCC ACC TCC CTC AGC TCC GCC GAC GCC 206
Thr Val Gln Asn Ser Val Pro Ser Thr Ser Leu Ser Ser Ala Asp Ala
        30                  35                  40

ACC CTC GGC CGC CAC CTG GCA CGC CGC CTC GTT CAA ATC GGC GTC ACC 254
Thr Leu Gly Arg His Leu Ala Arg Arg Leu Val Gln Ile Gly Val Thr
    45                  50                  55

GAC GTC TTC ACC GTC CCC GGC GAC TTC AAC TTG ACC CTT CTT GAC CAC 302
Asp Val Phe Thr Val Pro Gly Asp Phe Asn Leu Thr Leu Leu Asp His
60                  65                  70                  75

CTC ATC GCC GAG CCC GGC CTC ACC AAC ATT GGC TGC TGC AAC GAG CTC 350
Leu Ile Ala Glu Pro Gly Leu Thr Asn Ile Gly Cys Cys Asn Glu Leu
                80                  85                  90

AAC GCC GGG TAC GCC GCC GAC GGC TAC GCG CGG TCG CGT GGC GTC GGC 398
Asn Ala Gly Tyr Ala Ala Asp Gly Tyr Ala Arg Ser Arg Gly Val Gly
            95                  100                 105

GCG TGC GTG GTG ACT TTC ACT GTT GGT GGA CTG AGT GTG CTG AAC GCG 446
Ala Cys Val Val Thr Phe Thr Val Gly Gly Leu Ser Val Leu Asn Ala
        110                 115                 120

ATC GCC GGC GCG TAT AGT GAG AAT TTG CCG GTG ATT TGT ATT GTT GGT 494
Ile Ala Gly Ala Tyr Ser Glu Asn Leu Pro Val Ile Cys Ile Val Gly
    125                 130                 135

GGG CCC AAC TCT AAC GAT TAT GGG ACT AAC CGG ATT CTT CAC CAT ACT 542
Gly Pro Asn Ser Asn Asp Tyr Gly Thr Asn Arg Ile Leu His His Thr
140                 145                 150                 155

ATT GGG TTG CCG GAC TTC AGT CAA GAG CTC CGG TGC TTT CAG ACC GTG 590
Ile Gly Leu Pro Asp Phe Ser Gln Glu Leu Arg Cys Phe Gln Thr Val
                160                 165                 170

ACT TGC TTT CAG GCT GTG GTG AAT AAT CTG GAG GAT GCA CAT GAG ATG 638
Thr Cys Phe Gln Ala Val Val Asn Asn Leu Glu Asp Ala His Glu Met
            175                 180                 185

ATT GAT ACT GCA ATT TCG ACT GCG TTG AAA GAA AGC AAG CCT GTG TAT 686
Ile Asp Thr Ala Ile Ser Thr Ala Leu Lys Glu Ser Lys Pro Val Tyr
        190                 195                 200

ATC AGC ATT GGC TGC AAC TTG GCT GGG ATT CCT CAT CCT ACT TTC AGC 734
Ile Ser Ile Gly Cys Asn Leu Ala Gly Ile Pro His Pro Thr Phe Ser
    205                 210                 215

CGT GAA CCT GTT CCA TTT TCA TTG TCT CCA AAA TTG AGC AAT AAG TGG 782
Arg Glu Pro Val Pro Phe Ser Leu Ser Pro Lys Leu Ser Asn Lys Trp
220                 225                 230                 235

GGA TTA GAG GCT GCA GTG GAG GCT GCT GCA GAG TTC TTG AAC AAG GCA 830
Gly Leu Glu Ala Ala Val Glu Ala Ala Ala Glu Phe Leu Asn Lys Ala
                240                 245                 250

GTG AAG CCA GTT ATG GTG GGC GGG CCC AAA CTG CGC TCT GCA CAT GCT 878
Val Lys Pro Val Met Val Gly Gly Pro Lys Leu Arg Ser Ala His Ala
            255                 260                 265

GGT GAT GCC TTT GTT GAA CTG GCT GAT GCT TCT GGA TTT GCT CTG GCT 926
Gly Asp Ala Phe Val Glu Leu Ala Asp Ala Ser Gly Phe Ala Leu Ala
        270                 275                 280

GTG ATG CCA TCA GCA AAG GGG CAA GTG CCA GAG CAC CAC CCC CAT TTC 974
Val Met Pro Ser Ala Lys Gly Gln Val Pro Glu His His Pro His Phe
    285                 290                 295

ATC GGA ACG TAC TGG GGT GCT GTG AGC ACT GCC TTT TGT GCT GAG ATT 1022
Ile Gly Thr Tyr Trp Gly Ala Val Ser Thr Ala Phe Cys Ala Glu Ile
300                 305                 310                 315

GTG GAG TCT GCA GAT GCA TAC TTG TTT GCT GGG CCG ATT TTC AAT GAC 1070
Val Glu Ser Ala Asp Ala Tyr Leu Phe Ala Gly Pro Ile Phe Asn Asp
                320                 325                 330

TAC AGC TCA GTT GGG TAC TCG CTC CTT CTC AAG AAA GAG AAG GCG ATC 1118
Tyr Ser Ser Val Gly Tyr Ser Leu Leu Leu Lys Lys Glu Lys Ala Ile
            335                 340                 345

ATT GTG CAG CCA GAT CGT GTG ACG ATA GGG AAT GGC CCT ACA TTT GGT 1166
Ile Val Gln Pro Asp Arg Val Thr Ile Gly Asn Gly Pro Thr Phe Gly
        350                 355                 360

TGT GTT CTC ATG AAG GAT TTC CTC TTA GGC CTA GCA AAG AAG CTG AAG 1214
Cys Val Leu Met Lys Asp Phe Leu Leu Gly Leu Ala Lys Lys Leu Lys
    365                 370                 375

CAT AAC AAC ACT GCT CAT GAG AAC TAC CGC AGG ATC TTT GTG CCT GAT 1262
His Asn Asn Thr Ala His Glu Asn Tyr Arg Arg Ile Phe Val Pro Asp
380                 385                 390                 395

GGC CAC CCT CTG AAG GCT GCA CCC AAA GAA CCT TTG AGG GTT AAT GTT 1310
Gly His Pro Leu Lys Ala Ala Pro Lys Glu Pro Leu Arg Val Asn Val
                400                 405                 410

CTG TTC AAA CAC ATT CAG AAT ATG CTG TCA GCT GAA ACC GCT GTG ATT 1358
Leu Phe Lys His Ile Gln Asn Met Leu Ser Ala Glu Thr Ala Val Ile
            415                 420                 425

GCT GAG ACA GGG GAC TCA TGG TTT AAC TGT CAG AAG CTG AAA TTG CCA 1406
Ala Glu Thr Gly Asp Ser Trp Phe Asn Cys Gln Lys Leu Lys Leu Pro
        430                 435                 440

CCC GGC TGC GGG TAT GAG TTC CAA ATG CAA TAT GGA TCA ATT GGT TGG 1454
Pro Gly Cys Gly Tyr Glu Phe Gln Met Gln Tyr Gly Ser Ile Gly Trp
    445                 450                 455

TCA GTT GGA GCA ACT CTT GGG TAT GCT CAG GCT GTA CCT GAG AAG CGA 1502
Ser Val Gly Ala Thr Leu Gly Tyr Ala Gln Ala Val Pro Glu Lys Arg
460                 465                 470                 475

GTG ATT TCT TTC ATT GGT GAT GGG AGC TTC CAG GTG ACT GCT CAA GAT 1550
Val Ile Ser Phe Ile Gly Asp Gly Ser Phe Gln Val Thr Ala Gln Asp
                480                 485                 490

GTG TCC ACA ATG ATT CGA AAT GGA CAG AGA ACC ATT ATT TTC CTG ATA 1598
Val Ser Thr Met Ile Arg Asn Gly Gln Arg Thr Ile Ile Phe Leu Ile
            495                 500                 505

AAC AAT GGT GGA TAC ACC ATT GAA GTG GAA ATC CAT GAT GGA CCA TAC 1646
Asn Asn Gly Gly Tyr Thr Ile Glu Val Glu Ile His Asp Gly Pro Tyr
        510                 515                 520

AAT GTG ATC AAG AAC TGG AAC TAC ACT GGA CTG GTT GAT GCA ATC CAC 1694
Asn Val Ile Lys Asn Trp Asn Tyr Thr Gly Leu Val Asp Ala Ile His
    525                 530                 535

AAT GGG GAA GGC AAG TGC TGG ACA ACC AAG GTG CGT TGC GAA GAG GAG 1742
Asn Gly Glu Gly Lys Cys Trp Thr Thr Lys Val Arg Cys Glu Glu Glu
540                 545                 550                 555

CTG ATT GAA GCA ATA GAG ACT GCA AAT GGA CCC AAG AAG GAT AGC TTC 1790
Leu Ile Glu Ala Ile Glu Thr Ala Asn Gly Pro Lys Lys Asp Ser Phe
                560                 565                 570

TGC TTC ATT GAG GTG ATT GTT CAC AAG GAT GAT ACC AGC AAA GAG TTG 1838
Cys Phe Ile Glu Val Ile Val His Lys Asp Asp Thr Ser Lys Glu Leu
            575                 580                 585

CTT GAG TGG GGG TCT AGG GTT TCT GCT GCC AAC AGC CGC CCA CCT AAT 1886
Leu Glu Trp Gly Ser Arg Val Ser Ala Ala Asn Ser Arg Pro Pro Asn
        590                 595                 600

CCT CAG TAAAACTCTC CTGTGTCATA TGAAGGCCTT CATTCACATT CACAGATTTA 1942
Pro Gln
    605

GATCAAGCCA AGCTCTTGTG CAAATTTTCC TTATGTTTTT CCTGTCAACT GGAGAATGGT 2002

GTCTGTCAAG TTTTTTTTAC ACTACAGTGA TTTCTGGTTT GTCTGTATAT TTCCTTCTGA 2062

ATATTAGTAT CTTCTGATTT TTCAATTGAT CAAATTCTGT GATCCTAAAT GGTTTGTGGA 2122

AAAAAAAAAA AAAAAAAAA 2141

(2) INFORMATION FOR SEQ ID NO:10:

(i) SEQUENCE CHARACTERISTICS:
(A) LENGTH: 605 amino acids
(B) TYPE: amino acid
(D) TOPOLOGY: linear

(ii) MOLECULE TYPE: protein
(xi) SEQUENCE DESCRIPTION: SEQ ID NO:10:

Met Asp Thr Lys Ile Gly Ser Ile Asp Val Cys Lys Thr Glu Asn His
1               5                   10                  15

Asp Val Gly Cys Leu Pro Asn Ser Ala Thr Ser Thr Val Gln Asn Ser
            20                  25                  30

Val Pro Ser Thr Ser Leu Ser Ser Ala Asp Ala Thr Leu Gly Arg His
        35                  40                  45

Leu Ala Arg Arg Leu Val Gln Ile Gly Val Thr Asp Val Phe Thr Val
    50                  55                  60

Pro Gly Asp Phe Asn Leu Thr Leu Leu Asp His Leu Ile Ala Glu Pro
65                  70                  75                  80

Gly Leu Thr Asn Ile Gly Cys Cys Asn Glu Leu Asn Ala Gly Tyr Ala
                85                  90                  95

Ala Asp Gly Tyr Ala Arg Ser Arg Gly Val Gly Ala Cys Val Val Thr
            100                 105                 110

Phe Thr Val Gly Gly Leu Ser Val Leu Asn Ala Ile Ala Gly Ala Tyr
        115                 120                 125

Ser Glu Asn Leu Pro Val Ile Cys Ile Val Gly Gly Pro Asn Ser Asn
    130                 135                 140

Asp Tyr Gly Thr Asn Arg Ile Leu His His Thr Ile Gly Leu Pro Asp
145                 150                 155                 160

Phe Ser Gln Glu Leu Arg Cys Phe Gln Thr Val Thr Cys Phe Gln Ala
                165                 170                 175

Val Val Asn Asn Leu Glu Asp Ala His Glu Met Ile Asp Thr Ala Ile
            180                 185                 190

Ser Thr Ala Leu Lys Glu Ser Lys Pro Val Tyr Ile Ser Ile Gly Cys
        195                 200                 205

Asn Leu Ala Gly Ile Pro His Pro Thr Phe Ser Arg Glu Pro Val Pro
    210                 215                 220

Phe Ser Leu Ser Pro Lys Leu Ser Asn Lys Trp Gly Leu Glu Ala Ala
225                 230                 235                 240

Val Glu Ala Ala Ala Glu Phe Leu Asn Lys Ala Val Lys Pro Val Met
                245                 250                 255

Val Gly Gly Pro Lys Leu Arg Ser Ala His Ala Gly Asp Ala Phe Val
            260                 265                 270

Glu Leu Ala Asp Ala Ser Gly Phe Ala Leu Ala Val Met Pro Ser Ala
        275                 280                 285

Lys Gly Gln Val Pro Glu His His Pro His Phe Ile Gly Thr Tyr Trp
    290                 295                 300

Gly Ala Val Ser Thr Ala Phe Cys Ala Glu Ile Val Glu Ser Ala Asp
305                 310                 315                 320

Ala Tyr Leu Phe Ala Gly Pro Ile Phe Asn Asp Tyr Ser Ser Val Gly
                325                 330                 335

Tyr Ser Leu Leu Leu Lys Lys Glu Lys Ala Ile Ile Val Gln Pro Asp
            340                 345                 350

Arg Val Thr Ile Gly Asn Gly Pro Thr Phe Gly Cys Val Leu Met Lys
        355                 360                 365

Asp Phe Leu Leu Gly Leu Ala Lys Lys Leu Lys His Asn Asn Thr Ala
    370                 375                 380

His Glu Asn Tyr Arg Arg Ile Phe Val Pro Asp Gly His Pro Leu Lys
385                 390                 395                 400

Ala Ala Pro Lys Glu Pro Leu Arg Val Asn Val Leu Phe Lys His Ile
                405                 410                 415

Gln Asn Met Leu Ser Ala Glu Thr Ala Val Ile Ala Glu Thr Gly Asp
            420                 425                 430

Ser Trp Phe Asn Cys Gln Lys Leu Lys Leu Pro Pro Gly Cys Gly Tyr
        435                 440                 445

Glu Phe Gln Met Gln Tyr Gly Ser Ile Gly Trp Ser Val Gly Ala Thr
    450                 455                 460

Leu Gly Tyr Ala Gln Ala Val Pro Glu Lys Arg Val Ile Ser Phe Ile
465                 470                 475                 480

Gly Asp Gly Ser Phe Gln Val Thr Ala Gln Asp Val Ser Thr Met Ile
                485                 490                 495

Arg Asn Gly Gln Arg Thr Ile Ile Phe Leu Ile Asn Asn Gly Gly Tyr
            500                 505                 510

Thr Ile Glu Val Glu Ile His Asp Gly Pro Tyr Asn Val Ile Lys Asn
        515                 520                 525

Trp Asn Tyr Thr Gly Leu Val Asp Ala Ile His Asn Gly Glu Gly Lys
    530                 535                 540

Cys Trp Thr Thr Lys Val Arg Cys Glu Glu Glu Leu Ile Glu Ala Ile
545                 550                 555                 560

Glu Thr Ala Asn Gly Pro Lys Lys Asp Ser Phe Cys Phe Ile Glu Val
                565                 570                 575

Ile Val His Lys Asp Asp Thr Ser Lys Glu Leu Leu Glu Trp Gly Ser
            580                 585                 590

Arg Val Ser Ala Ala Asn Ser Arg Pro Pro Asn Pro Gln
        595                 600                 605

(2) INFORMATION FOR SEQ ID NO:11:

(i) SEQUENCE CHARACTERISTICS:
(A) LENGTH: 1415 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION:56..1054
(D) OTHER INFORMATION:/product= "Strawberry alcohol
dehydrogenase"

(xi) SEQUENCE DESCRIPTION: SEQ ID NO:11:

TAATCTAGCT TCTGCACCAA AACTATCAGA TAATTAAGAA TCTGCCACAG AGAAA ATG 58
Met
1

GTG ATG TCT ATC GAG CAG GAA CAC CCC AAG AAG GCA TCT GGA TGG GCT 106
Val Met Ser Ile Glu Gln Glu His Pro Lys Lys Ala Ser Gly Trp Ala
            5                   10                  15

GCA AGA GAT TCA TCT GGT GTT CTC TCT CCC TTC AGT TTC TCC AGA AGG 154
Ala Arg Asp Ser Ser Gly Val Leu Ser Pro Phe Ser Phe Ser Arg Arg
        20                  25                  30

GAA ACC GGA GAG AAA GAC GTG ACG TTC AAA GTG ATG TAC TGT GGG ATT 202
Glu Thr Gly Glu Lys Asp Val Thr Phe Lys Val Met Tyr Cys Gly Ile
    35                  40                  45

TGC CAT TCG GAC CTT CAC ATG GTC AAG AAT GAA TGG GGC TTC TCT ACC 250
Cys His Ser Asp Leu His Met Val Lys Asn Glu Trp Gly Phe Ser Thr
50                  55                  60                  65

TAT CCT CTG GTT CCA GGG CAT GAG ATT GTT GGT GAA GTG ACG GAA GTA 298
Tyr Pro Leu Val Pro Gly His Glu Ile Val Gly Glu Val Thr Glu Val
                70                  75                  80

GGA AGC AAT GTA CAA AAA TTC AAA GTT GGA GAC AGA GTC GGT GTT GGA 346
Gly Ser Asn Val Gln Lys Phe Lys Val Gly Asp Arg Val Gly Val Gly
            85                  90                  95

TGC ATT GTG GGA TCT TGC CGA TCT TGT GAA AAT TGT ACC GAC CAC CTT 394
Cys Ile Val Gly Ser Cys Arg Ser Cys Glu Asn Cys Thr Asp His Leu
        100                 105                 110

GAG AAC TAC TGC CCC AAA CAG ATA CTC ACT TAC GGT GCC AAG TAC TAC 442
Glu Asn Tyr Cys Pro Lys Gln Ile Leu Thr Tyr Gly Ala Lys Tyr Tyr
    115                 120                 125

GAC GGA ACC ACC ACC TAT GGC GGT TAC TCT GAC ATT ATG GTG GCC GAT 490
Asp Gly Thr Thr Thr Tyr Gly Gly Tyr Ser Asp Ile Met Val Ala Asp
130                 135                 140                 145

GAA CAC TTC ATA GTA CGC ATC CCA GAC AAC TTG CCT CTT GAT GGT GCT 538
Glu His Phe Ile Val Arg Ile Pro Asp Asn Leu Pro Leu Asp Gly Ala
                150                 155                 160

GCG CCG CTC CTA TGT GCC GGG ATT ACA ACC TAC AGC CCC CTG AGA TAT 586
Ala Pro Leu Leu Cys Ala Gly Ile Thr Thr Tyr Ser Pro Leu Arg Tyr
            165                 170                 175

TTC GGA CTT GAC AAG CCC GGC ATG CAT GTA GGT GTG GTC GGC CTA GGC 634
Phe Gly Leu Asp Lys Pro Gly Met His Val Gly Val Val Gly Leu Gly
        180                 185                 190

GGT TTA GGC CAC GTC GCC GTG AAG TTT GCC AAG GCT ATG GGA GTG AAG 682
Gly Leu Gly His Val Ala Val Lys Phe Ala Lys Ala Met Gly Val Lys
    195                 200                 205

GTT ACA GTG ATT AGT ACA TCC CCT AAG AAA GAG GAG GAA GCT CGT AAA 730
Val Thr Val Ile Ser Thr Ser Pro Lys Lys Glu Glu Glu Ala Arg Lys
210                 215                 220                 225

CAC CTA GGA GCT GAC TCG TTT TTG GTT AGC CGT GAC CAA GAT CAA ATG 778
His Leu Gly Ala Asp Ser Phe Leu Val Ser Arg Asp Gln Asp Gln Met
                230                 235                 240

CAG GCT GCC ATT GGT ACC ATG GAT GGG ATC ATT GAC ACG GTT TCT GCA 826
Gln Ala Ala Ile Gly Thr Met Asp Gly Ile Ile Asp Thr Val Ser Ala
            245                 250                 255

CAA CAT CCT CTC CTG CCT TTG ATT GGT TTG TTG AAG TCT CAT GGA AAG 874
Gln His Pro Leu Leu Pro Leu Ile Gly Leu Leu Lys Ser His Gly Lys
        260                 265                 270

CTT GTT ATG GTT GGT GCA CCA GAG AAG CCT CTT GAA CTG CCA GTT TTT 922
Leu Val Met Val Gly Ala Pro Glu Lys Pro Leu Glu Leu Pro Val Phe
    275                 280                 285

CCT TTA CTC ATG GGA AGA AAG ATG GTA GCT GGT AGC GGC ATT GGG GGT 970
Pro Leu Leu Met Gly Arg Lys Met Val Ala Gly Ser Gly Ile Gly Gly
290                 295                 300                 305

ATG AAG GAG ACA CAA GAG ATG ATA GAT TTT GCA GCC AAG CAC AAC ATT 1018
Met Lys Glu Thr Gln Glu Met Ile Asp Phe Ala Ala Lys His Asn Ile
                310                 315                 320

ACA GCA GAC ATC GAA GTC ATA CCA ATC GAC TAC TTG TAACACTGCT 1064
Thr Ala Asp Ile Glu Val Ile Pro Ile Asp Tyr Leu
            325                 330

ATGGAGCGTC TAGTCAAAGC AGATGTCAGA TACCGTTTTG TCATCGACAT TGGAAACACA 1124

CTGAAGGCTA GCTCTTAAAT TCTGCAATCC AGACTGGATC AATGAAGAAA CAAGAACAGA 1184

AACGGAGACT GATTTAGTGT CATACTCGGT GTTGGTTTTC CTTGTAGCAT TTTTTGTTGT 1244

CTGCTACATG AATAATGATC ACATGAACAA CTGCCTTCTG TGATGATTTG ATAATAAAAG 1304

AATACATGAA CAATGATACT GCCTTCTTTT GTAATGTTTT TTACTATATA ATCATTTCAA 1364

ATTATTTTGC TATATCTCTA AAAAAAAAAA AAAAAAAAAA AAAAAAAAAA A 1415

(2) INFORMATION FOR SEQ ID NO:12:

(i) SEQUENCE CHARACTERISTICS:
(A) LENGTH: 333 amino acids
(B) TYPE: amino acid
(D) TOPOLOGY: linear

(ii) MOLECULE TYPE: protein
(xi) SEQUENCE DESCRIPTION: SEQ ID NO:12:

Met Val Met Ser Ile Glu Gln Glu His Pro Lys Lys Ala Ser Gly Trp
1               5                   10                  15

Ala Ala Arg Asp Ser Ser Gly Val Leu Ser Pro Phe Ser Phe Ser Arg
            20                  25                  30

Arg Glu Thr Gly Glu Lys Asp Val Thr Phe Lys Val Met Tyr Cys Gly
        35                  40                  45

Ile Cys His Ser Asp Leu His Met Val Lys Asn Glu Trp Gly Phe Ser
    50                  55                  60

Thr Tyr Pro Leu Val Pro Gly His Glu Ile Val Gly Glu Val Thr Glu
65                  70                  75                  80

Val Gly Ser Asn Val Gln Lys Phe Lys Val Gly Asp Arg Val Gly Val
                85                  90                  95

Gly Cys Ile Val Gly Ser Cys Arg Ser Cys Glu Asn Cys Thr Asp His
            100                 105                 110

Leu Glu Asn Tyr Cys Pro Lys Gln Ile Leu Thr Tyr Gly Ala Lys Tyr
        115                 120                 125

Tyr Asp Gly Thr Thr Thr Tyr Gly Gly Tyr Ser Asp Ile Met Val Ala
    130                 135                 140

Asp Glu His Phe Ile Val Arg Ile Pro Asp Asn Leu Pro Leu Asp Gly
145                 150                 155                 160

Ala Ala Pro Leu Leu Cys Ala Gly Ile Thr Thr Tyr Ser Pro Leu Arg
                165                 170                 175

Tyr Phe Gly Leu Asp Lys Pro Gly Met His Val Gly Val Val Gly Leu
            180                 185                 190

Gly Gly Leu Gly His Val Ala Val Lys Phe Ala Lys Ala Met Gly Val
        195                 200                 205

Lys Val Thr Val Ile Ser Thr Ser Pro Lys Lys Glu Glu Glu Ala Arg
    210                 215                 220

Lys His Leu Gly Ala Asp Ser Phe Leu Val Ser Arg Asp Gln Asp Gln
225                 230                 235                 240

Met Gln Ala Ala Ile Gly Thr Met Asp Gly Ile Ile Asp Thr Val Ser
                245                 250                 255

Ala Gln His Pro Leu Leu Pro Leu Ile Gly Leu Leu Lys Ser His Gly
            260                 265                 270

Lys Leu Val Met Val Gly Ala Pro Glu Lys Pro Leu Glu Leu Pro Val
        275                 280                 285

Phe Pro Leu Leu Met Gly Arg Lys Met Val Ala Gly Ser Gly Ile Gly
    290                 295                 300

Gly Met Lys Glu Thr Gln Glu Met Ile Asp Phe Ala Ala Lys His Asn
305                 310                 315                 320

Ile Thr Ala Asp Ile Glu Val Ile Pro Ile Asp Tyr Leu
                325                 330

(2) INFORMATION FOR SEQ ID NO:13:

(i) SEQUENCE CHARACTERISTICS:
(A) LENGTH: 1227 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "partial cDNA"

(ix) FEATURE:
(A) NAME/KEY: CDS
(B) LOCATION:2..979
(D) OTHER INFORMATION:/product= "Strawberry alcohol
dehydrogenase"

(xi) SEQUENCE DESCRIPTION: SEQ ID NO:13:

G GAA ACA GGA GCA ACG GAC GTA AGA TTC AAA GTG TTG TAC TGT GGA      46
  Glu Thr Gly Ala Thr Asp Val Arg Phe Lys Val Leu Tyr Cys Gly
    1               5                  10                  15

GTA TGC CAT TCG GAC ATA CAC ATG GCC AAA AAT GAT TGG GGG ACT TCT      94
Val Cys His Ser Asp Ile His Met Ala Lys Asn Asp Trp Gly Thr Ser
                20                  25                  30

ACC TAT CCT ATT GTA CCT GGG CAT GAA CTT GTT GGT GTA GTA ACA GAA     142
Thr Tyr Pro Ile Val Pro Gly His Glu Leu Val Gly Val Val Thr Glu
            35                  40                  45

GTA GGA TGC AAA GTA AAG AAA TTC AAA AGT TGG AGA CAA GGT CGG TGT     190
Val Gly Cys Lys Val Lys Lys Phe Lys Ser Trp Arg Gln Gly Arg Cys
        50                  55                  60

TGG TTG CAT GGT CGA CTC AGA CCA ACT TGC GAA AAT TGT ATC CAT CAC     238
Trp Leu His Gly Arg Leu Arg Pro Thr Cys Glu Asn Cys Ile His His
    65                  70                  75

CTA GAA AAT TAC TGT CCG AAT CTG ATA CAA ACC TAC GGT TCT AAA TAC     286
Leu Glu Asn Tyr Cys Pro Asn Leu Ile Gln Thr Tyr Gly Ser Lys Tyr
80                  85                  90                  95

TAC GAC GGA ACC ATG ACA TAC GGA GGT TAC TCG AAC AAC ATG GTG ACT     334
Tyr Asp Gly Thr Met Thr Tyr Gly Gly Tyr Ser Asn Asn Met Val Thr
                100                 105                 110

GAT GAG CAC TTC ATT GTT CGG ATC CCG GAC AAC TTA CCT CTT GAT GGC     382
Asp Glu His Phe Ile Val Arg Ile Pro Asp Asn Leu Pro Leu Asp Gly
            115                 120                 125

GCT GCT CCG CTT CTA TGT GCC GGG ATT ACA ACT TAC AGC CCA TGG AGA     430
Ala Ala Pro Leu Leu Cys Ala Gly Ile Thr Thr Tyr Ser Pro Trp Arg
        130                 135                 140

TAT TAT GGA CTT GAC AAA CCC GGT ATG CAT CTT GGT GTT GAA TGG CCT     478
Tyr Tyr Gly Leu Asp Lys Pro Gly Met His Leu Gly Val Glu Trp Pro
    145                 150                 155

AGG CGG TTT AGG TCA CGT CCG CCG TTA AAT TTG CCA GGG CTT TGG GGC 526
Arg Arg Phe Arg Ser Arg Pro Pro Leu Asn Leu Pro Gly Leu Trp Gly
160                 165                 170                 175

TCA AGG TTA CAG TCA TTA GTA CCT CCC CTA ATT AAA GAA GGA GGC AGC 574
Ser Arg Leu Gln Ser Leu Val Pro Pro Leu Ile Lys Glu Gly Gly Ser
                180                 185                 190

TAT GGA ACA TCT CCC GCG CTG ATG CAT TCC CTG CTT AGA ACT GAC CAA 622
Tyr Gly Thr Ser Pro Ala Leu Met His Ser Leu Leu Arg Thr Asp Gln
            195                 200                 205

GAT CAG ATG GAG GCT GCC ATG AGC ACA ATG GAT GGT ATC ATT GAC ACA 670
Asp Gln Met Glu Ala Ala Met Ser Thr Met Asp Gly Ile Ile Asp Thr
        210                 215                 220

GTT CCT GCA GTT CGA CCT CTA GAG CCT TTG ATT TCA TTG TTG AAG ACT 718
Val Pro Ala Val Arg Pro Leu Glu Pro Leu Ile Ser Leu Leu Lys Thr
    225                 230                 235

AAT GGA AAA GTT GTT ACC GTT GGT ATA GCA GTG CAG CCA CTC GAT CTC 766
Asn Gly Lys Val Val Thr Val Gly Ile Ala Val Gln Pro Leu Asp Leu
240                 245                 250                 255

CCA GTT TTC CCT TTG ATA ATA GGA AGG AAG ATG GTA GCT GGT AGT GCC 814
Pro Val Phe Pro Leu Ile Ile Gly Arg Lys Met Val Ala Gly Ser Ala
                260                 265                 270

ATT GGA GGT ATG AAA GAG ACG CAA GAG ATG ATT GAT TTT GCT GCT GAA 862
Ile Gly Gly Met Lys Glu Thr Gln Glu Met Ile Asp Phe Ala Ala Glu
            275                 280                 285

CAT AAC ATA ACA GCT GAC ATC GAG GTC ATC CCG ATT GAT TAC CTG AAC 910
His Asn Ile Thr Ala Asp Ile Glu Val Ile Pro Ile Asp Tyr Leu Asn
        290                 295                 300

ACC GCA ATG GAA CGC GTT GTC AAA AAA GAT GTC AGG TTT CGA TTT GTC 958
Thr Ala Met Glu Arg Val Val Lys Lys Asp Val Arg Phe Arg Phe Val
    305                 310                 315

ATC GAC GTT GAG AAC ACA TTG TAAGTCCGCC TAAGTTTTTC ATTCAATTCT 1009
Ile Asp Val Glu Asn Thr Leu
320                 325

GTTAATAAGA CTATGCATTA ATATATGACT GACTCTCCAT AGGATGGAGT TATCAGTCTT 1069

CAAATTTCTA GACATATTTT GTGATCAAAT AAATGGAATG GCTTTGTTTT CCTTTTCCAC 1129

TAAGATTAGA TTTCAGTTGT ATTGTTTTTA AAGAGATTGA TGTTTTTATT AATTGTAACA 1189

GTGTTATCAG TCTAATCATT AAAAAAAAAA AAAAAAAA 1227

(2) INFORMATION FOR SEQ ID NO:14:

(i) SEQUENCE CHARACTERISTICS:
(A) LENGTH: 326 amino acids
(B) TYPE: amino acid
(D) TOPOLOGY: linear

(ii) MOLECULE TYPE: protein
(xi) SEQUENCE DESCRIPTION: SEQ ID NO:14:

Glu Thr Gly Ala Thr Asp Val Arg Phe Lys Val Leu Tyr Cys Gly Val
1               5                   10                  15

Cys His Ser Asp Ile His Met Ala Lys Asn Asp Trp Gly Thr Ser Thr
            20                  25                  30

Tyr Pro Ile Val Pro Gly His Glu Leu Val Gly Val Val Thr Glu Val
        35                  40                  45

Gly Cys Lys Val Lys Lys Phe Lys Ser Trp Arg Gln Gly Arg Cys Trp
    50                  55                  60

Leu His Gly Arg Leu Arg Pro Thr Cys Glu Asn Cys Ile His His Leu
65                  70                  75                  80

Glu Asn Tyr Cys Pro Asn Leu Ile Gln Thr Tyr Gly Ser Lys Tyr Tyr
                85                  90                  95

Asp Gly Thr Met Thr Tyr Gly Gly Tyr Ser Asn Asn Met Val Thr Asp
            100                 105                 110

Glu His Phe Ile Val Arg Ile Pro Asp Asn Leu Pro Leu Asp Gly Ala
        115                 120                 125

Ala Pro Leu Leu Cys Ala Gly Ile Thr Thr Tyr Ser Pro Trp Arg Tyr
    130                 135                 140

Tyr Gly Leu Asp Lys Pro Gly Met His Leu Gly Val Glu Trp Pro Arg
145                 150                 155                 160

Arg Phe Arg Ser Arg Pro Pro Leu Asn Leu Pro Gly Leu Trp Gly Ser
                165                 170                 175

Arg Leu Gln Ser Leu Val Pro Pro Leu Ile Lys Glu Gly Gly Ser Tyr
            180                 185                 190

Gly Thr Ser Pro Ala Leu Met His Ser Leu Leu Arg Thr Asp Gln Asp
        195                 200                 205

Gln Met Glu Ala Ala Met Ser Thr Met Asp Gly Ile Ile Asp Thr Val
    210                 215                 220

Pro Ala Val Arg Pro Leu Glu Pro Leu Ile Ser Leu Leu Lys Thr Asn
225                 230                 235                 240

Gly Lys Val Val Thr Val Gly Ile Ala Val Gln Pro Leu Asp Leu Pro
                245                 250                 255

Val Phe Pro Leu Ile Ile Gly Arg Lys Met Val Ala Gly Ser Ala Ile
            260                 265                 270

Gly Gly Met Lys Glu Thr Gln Glu Met Ile Asp Phe Ala Ala Glu His
        275                 280                 285

Asn Ile Thr Ala Asp Ile Glu Val Ile Pro Ile Asp Tyr Leu Asn Thr
    290                 295                 300

Ala Met Glu Arg Val Val Lys Lys Asp Val Arg Phe Arg Phe Val Ile
305                 310                 315                 320

Asp Val Glu Asn Thr Leu
                325

(2) INFORMATION FOR SEQ ID NO:15:

     (i) SEQUENCE CHARACTERISTICS:
          (A) LENGTH: 1063 base pairs
          (B) TYPE: nucleic acid
          (C) STRANDEDNESS: single
          (D) TOPOLOGY: linear

(ii) MOLECULE TYPE: other nucleic acid
(A) DESCRIPTION: /desc = "partial cDNA"

(ix) FEATURE:
(A) NAME/KEY: CDS
(B) LOCATION:3..836
(D) OTHER INFORMATION:/product= "Strawberry alcohol
dehydrogenase"

(xi) SEQUENCE DESCRIPTION: SEQ ID NO:15:

GC AAA GTG CAA AAA TTT AAA GTT GGA GAC AAA GTT GGT GTT GGG TGC 47
Lys Val Gln Lys Phe Lys Val Gly Asp Lys Val Gly Val Gly Cys
1               5                   10                  15

TTG GTA GGC TCA TGC AAA ACT TGC GAC AGC TGT GCT AAC GAT TTG GAG 95
Leu Val Gly Ser Cys Lys Thr Cys Asp Ser Cys Ala Asn Asp Leu Glu
                20                  25                  30

AAC TAC TGC CCC AAA CAG ATA CAG ACT TAC GGC GCC AAG TAC CTT GAC 143
Asn Tyr Cys Pro Lys Gln Ile Gln Thr Tyr Gly Ala Lys Tyr Leu Asp
            35                  40                  45

GGA ACA ACC ACA TAC GGC GGT TAC TCT GAC ATC ATG GTG GCG GAT GAG 191
Gly Thr Thr Thr Tyr Gly Gly Tyr Ser Asp Ile Met Val Ala Asp Glu
        50                  55                  60

GCC TTT GTA ATC CGT ATT CCG GAC AAC CTG CCT CTT GAG GGT GCT GCT 239
Ala Phe Val Ile Arg Ile Pro Asp Asn Leu Pro Leu Glu Gly Ala Ala
    65                  70                  75

CCT CTC CTA TGT GCC GGA ATC ACA ACT TAC AGT CCC CTG AGG TAT TTC 287
Pro Leu Leu Cys Ala Gly Ile Thr Thr Tyr Ser Pro Leu Arg Tyr Phe
80                  85                  90                  95

GGA CTT GAC AAA CCC GGC ATG CAT GTC GGG GTG GTT GGC CTT GGC GGT 335
Gly Leu Asp Lys Pro Gly Met His Val Gly Val Val Gly Leu Gly Gly
                100                 105                 110

TTA GGC CAT GTC GCG GTG AAG TTT GCC AAG GCT TTG GGG GTT AAT GTC 383
Leu Gly His Val Ala Val Lys Phe Ala Lys Ala Leu Gly Val Asn Val
            115                 120                 125

ACA GTG ATC AGT ACC TCC GCT AAT AAG AAA GAT GAA GCT ATT AAA CAC 431
Thr Val Ile Ser Thr Ser Ala Asn Lys Lys Asp Glu Ala Ile Lys His
        130                 135                 140

CTT GGT GCT GAT TCT TTC TTG GTC AGT CGT GAC CAA GAT CAG ATG CAG 479
Leu Gly Ala Asp Ser Phe Leu Val Ser Arg Asp Gln Asp Gln Met Gln
    145                 150                 155

GCT GCC ATG GGA ACA TTG GAC GGT ATC ATC GAC ACA GTT TCC GCA GTC 527
Ala Ala Met Gly Thr Leu Asp Gly Ile Ile Asp Thr Val Ser Ala Val
160                 165                 170                 175

CAC CCC CTC CCA CCT TTG ATT AGT TTA TTG AAG GCT AAT GGA AAG CTT 575
His Pro Leu Pro Pro Leu Ile Ser Leu Leu Lys Ala Asn Gly Lys Leu
                180                 185                 190

GTT ATG GTT GGA GCA CCA GAG AAG CCA CTT GAG CTA CCA GTT TTT TCT 623
Val Met Val Gly Ala Pro Glu Lys Pro Leu Glu Leu Pro Val Phe Ser
            195                 200                 205

TTA ATA ATG GGA AGG AAG ACT TTA GCC GGT AGT AAT ATC GGA GGT ATC 671
Leu Ile Met Gly Arg Lys Thr Leu Ala Gly Ser Asn Ile Gly Gly Ile
        210                 215                 220

AAG GAG ACA CAA GAG ATG ATA GAT TTG GCA GCC AAA CAC AAC ATA ACG 719
Lys Glu Thr Gln Glu Met Ile Asp Leu Ala Ala Lys His Asn Ile Thr
    225                 230                 235

GCC GAC ATC GAG ATT ATC CCC ATC GAC TAT TTG AAC ACT GCT ATG GAG 767
Ala Asp Ile Glu Ile Ile Pro Ile Asp Tyr Leu Asn Thr Ala Met Glu
240                 245                 250                 255

CGT CTT GCT AAA GGG GAT GTT AGA TAC CGT TTT GTC ATC GAC ATC GGA 815
Arg Leu Ala Lys Gly Asp Val Arg Tyr Arg Phe Val Ile Asp Ile Gly
                260                 265                 270

AAC ACA TTG AAG CCG GCC ATT TAAATTTGCA TTTCGATCAG AAACTGAATC 866
Asn Thr Leu Lys Pro Ala Ile
            275

AAGCGAGGTC GAGAGGCCTA CGTAACAATG CAAACATGTG CTAGCTTGTT CTTGGAGTAG 926

TCTTTAGCTT TTCTCTGATG TATTCCATCT GTTTTGTTCA TGTCCCATCT TATTATGAGA 986

AAAATGTGGG TACCGTGGAT ATTGAATAAA TGAAGAGCTA CTGGAACGAT GGTTTCACAA 1046

AAAAAAAAAA AAAAAAA 1063

(2) INFORMATION FOR SEQ ID NO:16:

(i) SEQUENCE CHARACTERISTICS:
(A) LENGTH: 278 amino acids
(B) TYPE: amino acid
(D) TOPOLOGY: linear

(ii) MOLECULE TYPE: protein
(xi) SEQUENCE DESCRIPTION: SEQ ID NO:16:

Lys Val Gln Lys Phe Lys Val Gly Asp Lys Val Gly Val Gly Cys Leu
1               5                   10                  15

Val Gly Ser Cys Lys Thr Cys Asp Ser Cys Ala Asn Asp Leu Glu Asn
            20                  25                  30

Tyr Cys Pro Lys Gln Ile Gln Thr Tyr Gly Ala Lys Tyr Leu Asp Gly
        35                  40                  45

Thr Thr Thr Tyr Gly Gly Tyr Ser Asp Ile Met Val Ala Asp Glu Ala
    50                  55                  60

Phe Val Ile Arg Ile Pro Asp Asn Leu Pro Leu Glu Gly Ala Ala Pro
65                  70                  75                  80

Leu Leu Cys Ala Gly Ile Thr Thr Tyr Ser Pro Leu Arg Tyr Phe Gly
                85                  90                  95

Leu Asp Lys Pro Gly Met His Val Gly Val Val Gly Leu Gly Gly Leu
            100                 105                 110

Gly His Val Ala Val Lys Phe Ala Lys Ala Leu Gly Val Asn Val Thr
        115                 120                 125

Val Ile Ser Thr Ser Ala Asn Lys Lys Asp Glu Ala Ile Lys His Leu
    130                 135                 140

Gly Ala Asp Ser Phe Leu Val Ser Arg Asp Gln Asp Gln Met Gln Ala
145                 150                 155                 160

Ala Met Gly Thr Leu Asp Gly Ile Ile Asp Thr Val Ser Ala Val His
                165                 170                 175

Pro Leu Pro Pro Leu Ile Ser Leu Leu Lys Ala Asn Gly Lys Leu Val
            180                 185                 190

Met Val Gly Ala Pro Glu Lys Pro Leu Glu Leu Pro Val Phe Ser Leu
        195                 200                 205

Ile Met Gly Arg Lys Thr Leu Ala Gly Ser Asn Ile Gly Gly Ile Lys
    210                 215                 220

Glu Thr Gln Glu Met Ile Asp Leu Ala Ala Lys His Asn Ile Thr Ala
225                 230                 235                 240

Asp Ile Glu Ile Ile Pro Ile Asp Tyr Leu Asn Thr Ala Met Glu Arg
                245                 250                 255

Leu Ala Lys Gly Asp Val Arg Tyr Arg Phe Val Ile Asp Ile Gly Asn
            260                 265                 270

Thr Leu Lys Pro Ala Ile
        275

(2) INFORMATION FOR SEQ ID NO:17:

     (i) SEQUENCE CHARACTERISTICS:
          (A) LENGTH: 1228 base pairs
          (B) TYPE: nucleic acid
          (C) STRANDEDNESS: single
          (D) TOPOLOGY: linear

    (ii) MOLECULE TYPE: other nucleic acid
          (A) DESCRIPTION:  /desc = "partial cDNA"


    (ix) FEATURE:
          (A) NAME/KEY: CDS
          (B) LOCATION:1..849
          (D) OTHER INFORMATION:/product= "Strawberry alcohol
               dehydrogenase"


    (xi) SEQUENCE DESCRIPTION: SEQ ID NO:17:

GCA AGA GAT TCA TCT GGT GTC CTC TCT CCC TTC AAT TTC TCC AGA AGG        48
Ala Arg Asp Ser Ser Gly Val Leu Ser Pro Phe Asn Phe Ser Arg Arg
  1               5                  10                  15

GAA ACC GGA GAG AAA GAC GTT ATG TTC AAA GTG TTG TAC TGT GGA ATT        96
Glu Thr Gly Glu Lys Asp Val Met Phe Lys Val Leu Tyr Cys Gly Ile
             20                  25                  30

TGC CAT TCG GAC CTT CAC ATG GTC AAG AAT GAA TGG GGC TTC TCT ACC       144
Cys His Ser Asp Leu His Met Val Lys Asn Glu Trp Gly Phe Ser Thr
         35                  40                  45

TAT CCT TTG GTC CCG GGG CAT GAG ATT GTT GGT GAA GTT ACG GAA GTA       192
Tyr Pro Leu Val Pro Gly His Glu Ile Val Gly Glu Val Thr Glu Val
     50                  55                  60

GGG AGC AAA GTA CAA AAA TTT AAA GTT GGA GAC AGA GTC GGT GTT GGA       240
Gly Ser Lys Val Gln Lys Phe Lys Val Gly Asp Arg Val Gly Val Gly
 65                  70                  75                  80

TGC GTT GTG GGA TCT TGC CGA TCT TGT GAA AAT TGT ACC GAC CAC CTT 288
Cys Val Val Gly Ser Cys Arg Ser Cys Glu Asn Cys Thr Asp His Leu
                85                  90                  95

GAG AAC TAC TGC CCC AAA CAG ATA CTC ACT TAC GGT GCC AAG TAC TAC 336
Glu Asn Tyr Cys Pro Lys Gln Ile Leu Thr Tyr Gly Ala Lys Tyr Tyr
            100                 105                 110

GAC GGA ACC ACC ACC TAT GGC GGT TAC TCT GAC ATT ATG GTG GCC GAC 384
Asp Gly Thr Thr Thr Tyr Gly Gly Tyr Ser Asp Ile Met Val Ala Asp
        115                 120                 125

GAA CAC TTC ATA GTA CGC ATC CCA GAC AAC TTG CCT CTT GAT GGC GCT 432
Glu His Phe Ile Val Arg Ile Pro Asp Asn Leu Pro Leu Asp Gly Ala
    130                 135                 140

GCG CCG CTC CTA TGT GCC GGG ATT ACA ACC TAC AGC CCC CTG AGA TAT 480
Ala Pro Leu Leu Cys Ala Gly Ile Thr Thr Tyr Ser Pro Leu Arg Tyr
145                 150                 155                 160

TTC GGA CTT GAC AAG CCC GGC ATG CAT GTA GGT GTG GTC GGC CTA GGC 528
Phe Gly Leu Asp Lys Pro Gly Met His Val Gly Val Val Gly Leu Gly
                165                 170                 175

GGT TTA GGC CAC GTC GCC GTG AAG TTT GCC AAG GCT ATG GGA GTG AAG 576
Gly Leu Gly His Val Ala Val Lys Phe Ala Lys Ala Met Gly Val Lys
            180                 185                 190

GTT ACA GTG ATC AGT ACG TCC CCT AAG AAA GAG GAG GAA GCT CTT AAA 624
Val Thr Val Ile Ser Thr Ser Pro Lys Lys Glu Glu Glu Ala Leu Lys
        195                 200                 205

CAC CTA GGA GCT GAC TCG TTT TTC GTT AGC CGT GAC CAA GAT CAA ATG 672
His Leu Gly Ala Asp Ser Phe Phe Val Ser Arg Asp Gln Asp Gln Met
    210                 215                 220

CAG GCT GCC ATT GGT ACC ATG GAT GGG ATC ATT GAC ACA GTT TCT GCA 720
Gln Ala Ala Ile Gly Thr Met Asp Gly Ile Ile Asp Thr Val Ser Ala
225                 230                 235                 240

CAA CAT CCT CTC CTG CCT TTG ATT GGT TTG TTG AAG TCT CAT GGA AAG 768
Gln His Pro Leu Leu Pro Leu Ile Gly Leu Leu Lys Ser His Gly Lys
                245                 250                 255

CTT GTT ATG GTT GGT GCA CCA GAG AAG CCT CTT GAA CTT CCA GTT TTT 816
Leu Val Met Val Gly Ala Pro Glu Lys Pro Leu Glu Leu Pro Val Phe
            260                 265                 270

CCT TTA CTC ATG GGA AGA AAG ATG GGT AGC TGG TAACCGGCAT TTGGGGGTAT 869
Pro Leu Leu Met Gly Arg Lys Met Gly Ser Trp
        275                 280

GAAGGAGACA CAAGAGATGA TAGATTTTGC TGCCAGGCAC AACATAACAG CAGACATCGA 929

AGTCATACAA TCGACTACTT AAACACTGCT ATGGAGCGTT TAGTCAAAGC AGATGTCAGA 989

TACCGTTTTG TCATCGACAT TGGAAACACA CTGAAGGCTA GCACTTAAAT TCTGCAATCC 1049

AGACTGTATC AATGAAGAAA CAAGAACAGA AACTGAGATT GATTTGGTGT CATACTCCGC 1109

CTATGGTTTT CCTTACAGCA TTTTTTGTTG TTTGCTACAT GAATAACGAT CACATGAACT 1169

GTGATGATTT GATAATAAAA GAATACATAA ACAAAAAAAA CAAAAAAAAA AAAAAAAAA 1228

(2) INFORMATION FOR SEQ ID NO:18:

(i) SEQUENCE CHARACTERISTICS:
(A) LENGTH: 283 amino acids
(B) TYPE: amino acid
(D) TOPOLOGY: linear

(ii) MOLECULE TYPE: protein
(xi) SEQUENCE DESCRIPTION: SEQ ID NO:18:

Ala Arg Asp Ser Ser Gly Val Leu Ser Pro Phe Asn Phe Ser Arg Arg
1               5                   10                  15

Glu Thr Gly Glu Lys Asp Val Met Phe Lys Val Leu Tyr Cys Gly Ile
            20                  25                  30

Cys His Ser Asp Leu His Met Val Lys Asn Glu Trp Gly Phe Ser Thr
        35                  40                  45

Tyr Pro Leu Val Pro Gly His Glu Ile Val Gly Glu Val Thr Glu Val
    50                  55                  60

Gly Ser Lys Val Gln Lys Phe Lys Val Gly Asp Arg Val Gly Val Gly
65                  70                  75                  80

Cys Val Val Gly Ser Cys Arg Ser Cys Glu Asn Cys Thr Asp His Leu
                85                  90                  95

Glu Asn Tyr Cys Pro Lys Gln Ile Leu Thr Tyr Gly Ala Lys Tyr Tyr
            100                 105                 110

Asp Gly Thr Thr Thr Tyr Gly Gly Tyr Ser Asp Ile Met Val Ala Asp
        115                 120                 125

Glu His Phe Ile Val Arg Ile Pro Asp Asn Leu Pro Leu Asp Gly Ala
    130                 135                 140

Ala Pro Leu Leu Cys Ala Gly Ile Thr Thr Tyr Ser Pro Leu Arg Tyr
145                 150                 155                 160

Phe Gly Leu Asp Lys Pro Gly Met His Val Gly Val Val Gly Leu Gly
                165                 170                 175

Gly Leu Gly His Val Ala Val Lys Phe Ala Lys Ala Met Gly Val Lys
            180                 185                 190

Val Thr Val Ile Ser Thr Ser Pro Lys Lys Glu Glu Glu Ala Leu Lys
        195                 200                 205

His Leu Gly Ala Asp Ser Phe Phe Val Ser Arg Asp Gln Asp Gln Met
    210                 215                 220

Gln Ala Ala Ile Gly Thr Met Asp Gly Ile Ile Asp Thr Val Ser Ala
225                 230                 235                 240

Gln His Pro Leu Leu Pro Leu Ile Gly Leu Leu Lys Ser His Gly Lys
                245                 250                 255

Leu Val Met Val Gly Ala Pro Glu Lys Pro Leu Glu Leu Pro Val Phe
            260                 265                 270

Pro Leu Leu Met Gly Arg Lys Met Gly Ser Trp
        275                 280

(2) INFORMATION FOR SEQ ID NO:19:

(i) SEQUENCE CHARACTERISTICS:

(A) LENGTH: 852 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "partial cDNA"

(ix) FEATURE:
(A) NAME/KEY: CDS
(B) LOCATION:3..566
(D) OTHER INFORMATION:/product= "Strawberry alcohol
dehydrogenase"

(xi) SEQUENCE DESCRIPTION: SEQ ID NO:19:

GT CCC CTG AGG TAT TTC GGA CTT GAC AAA CCC GGC ATG CAT GTC GGG 47
Pro Leu Arg Tyr Phe Gly Leu Asp Lys Pro Gly Met His Val Gly
1               5                   10                  15

GTG GTT GGC CTT GGC GGT TTA GGC CAT GTC GCG GTG AAG TTT GCC AAG 95
Val Val Gly Leu Gly Gly Leu Gly His Val Ala Val Lys Phe Ala Lys
                20                  25                  30

GCT TTG GGG GTT GAG GTC ACA GTG ATC AGT ACC TCC GCT AAT AAG AAA 143
Ala Leu Gly Val Glu Val Thr Val Ile Ser Thr Ser Ala Asn Lys Lys
            35                  40                  45

GAT GAA GCT ATT AAA CAC CTT GGT GCT GAT TCT TTC TTG GTC AGT CGT 191
Asp Glu Ala Ile Lys His Leu Gly Ala Asp Ser Phe Leu Val Ser Arg
        50                  55                  60

GAC CAA GAT CAG ATG CAG GCT GCC ATG GGA ACA TTG GAC GGT ATC ATC 239
Asp Gln Asp Gln Met Gln Ala Ala Met Gly Thr Leu Asp Gly Ile Ile
    65                  70                  75

GAC ACA GTT TCT GCA GTC CAC CCC CTC CCA CCT TTG ATT AGT TTA TTG 287
Asp Thr Val Ser Ala Val His Pro Leu Pro Pro Leu Ile Ser Leu Leu
80                  85                  90                  95

AAG GCT AAT GGA AAG CTT GTT ATG GTT GGA GCA CCA GAG AAG CCA CTT 335
Lys Ala Asn Gly Lys Leu Val Met Val Gly Ala Pro Glu Lys Pro Leu
                100                 105                 110

GAG CTA CCA GTT TTT TCT TTA ATA ATG GGA AGG AAG ACT TTA GCC GGT 383
Glu Leu Pro Val Phe Ser Leu Ile Met Gly Arg Lys Thr Leu Ala Gly
            115                 120                 125

AGT AAT ATC GGA GGT ATC AAG GAG ACA CAA GAG ATG ATA GAT TTG GCA 431
Ser Asn Ile Gly Gly Ile Lys Glu Thr Gln Glu Met Ile Asp Leu Ala
        130                 135                 140

GCT AAA CAC AAC ATA ACG GCC GAC ATC GAG GTC ATC CCC ATC GAT TAT 479
Ala Lys His Asn Ile Thr Ala Asp Ile Glu Val Ile Pro Ile Asp Tyr
    145                 150                 155

TTG AAC ACT GCA ATG GAG CGT CTT GCT AAA GGG GAT GTT AGA TAC CGG 527
Leu Asn Thr Ala Met Glu Arg Leu Ala Lys Gly Asp Val Arg Tyr Arg
160                 165                 170                 175

TTT GTC ATC GAC ATC GGA AAC ACA TTG AAG CCG GCC ACT TAAATTTGCA 576
Phe Val Ile Asp Ile Gly Asn Thr Leu Lys Pro Ala Thr
                180                 185

TTTCGATCAG AAACTGAATC AAGCGATGTC GAGAGGCCTA CGTAACAATG TAAACATGTG 636

CTAGCTTGTT CTTGTAGTAG TCTTTAGCAT TTCTCTGATG TACTCCTTCT GTTTTGTTCA 696

TGTTCCATCT TATAATAAGA TTCTTATTAT GAAAAAAATA TGGTACCGTG GATATTGAAT 756

AAATGAAGAA CTACTGGAAC AATGGTTTCA CAAATTATTT GTGGTGCTAA AAAAAAAAAA 816

AAAAAAAAAA AAAAAAAAAA AAAAAAAAAA AAAAAA 852

(2) INFORMATION FOR SEQ ID NO:20:

(i) SEQUENCE CHARACTERISTICS:
(A) LENGTH: 188 amino acids
(B) TYPE: amino acid
(D) TOPOLOGY: linear

(ii) MOLECULE TYPE: protein
(xi) SEQUENCE DESCRIPTION: SEQ ID NO:20:

Pro Leu Arg Tyr Phe Gly Leu Asp Lys Pro Gly Met His Val Gly Val
1               5                   10                  15

Val Gly Leu Gly Gly Leu Gly His Val Ala Val Lys Phe Ala Lys Ala
            20                  25                  30

Leu Gly Val Glu Val Thr Val Ile Ser Thr Ser Ala Asn Lys Lys Asp
        35                  40                  45

Glu Ala Ile Lys His Leu Gly Ala Asp Ser Phe Leu Val Ser Arg Asp
    50                  55                  60

Gln Asp Gln Met Gln Ala Ala Met Gly Thr Leu Asp Gly Ile Ile Asp
65                  70                  75                  80

Thr Val Ser Ala Val His Pro Leu Pro Pro Leu Ile Ser Leu Leu Lys
                85                  90                  95

Ala Asn Gly Lys Leu Val Met Val Gly Ala Pro Glu Lys Pro Leu Glu
            100                 105                 110

Leu Pro Val Phe Ser Leu Ile Met Gly Arg Lys Thr Leu Ala Gly Ser
        115                 120                 125

Asn Ile Gly Gly Ile Lys Glu Thr Gln Glu Met Ile Asp Leu Ala Ala
    130                 135                 140

Lys His Asn Ile Thr Ala Asp Ile Glu Val Ile Pro Ile Asp Tyr Leu
145                 150                 155                 160

Asn Thr Ala Met Glu Arg Leu Ala Lys Gly Asp Val Arg Tyr Arg Phe
                165                 170                 175

Val Ile Asp Ile Gly Asn Thr Leu Lys Pro Ala Thr
            180                 185

(2) INFORMATION FOR SEQ ID NO:21:

(i) SEQUENCE CHARACTERISTICS:
(A) LENGTH: 663 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "partial cDNA"

(ix) FEATURE:
(A) NAME/KEY: CDS
(B) LOCATION:3..545
(D) OTHER INFORMATION:/product= "Strawberry alcohol
dehydrogenase"

(xi) SEQUENCE DESCRIPTION: SEQ ID NO:21:

AG TTT GGT CTT GAT GTG GGT GGA TTA AGG GGA GGG ATA TTG GGA CTT 47
Phe Gly Leu Asp Val Gly Gly Leu Arg Gly Gly Ile Leu Gly Leu
1               5                   10                  15

GGA GGT GTT GGA CAC ATG GGG GTG AAG ATA GCA AAG GCT ATG GGA CAC 95
Gly Gly Val Gly His Met Gly Val Lys Ile Ala Lys Ala Met Gly His
                20                  25                  30

CAT ATC ACC GTG ATA AGC TCT TCT GAT AAG AAG AAA AAA GAG GCC TTG 143
His Ile Thr Val Ile Ser Ser Ser Asp Lys Lys Lys Lys Glu Ala Leu
            35                  40                  45

GAG CAT ATT GGT GCT GAT GAG TAC TTG GTG AGC TCT GAT GCC ACC CAA 191
Glu His Ile Gly Ala Asp Glu Tyr Leu Val Ser Ser Asp Ala Thr Gln
        50                  55                  60

ATG CAA GAG GCT ATG GAC TCA CTG GAT TAC ATT ATT GAC ACC ATT CCA 239
Met Gln Glu Ala Met Asp Ser Leu Asp Tyr Ile Ile Asp Thr Ile Pro
    65                  70                  75

GTG TTC CAC CCT CTT GAG CCT TAC CTC TCT TTG TTG AAG CTT GAT GGG 287
Val Phe His Pro Leu Glu Pro Tyr Leu Ser Leu Leu Lys Leu Asp Gly
80                  85                  90                  95

AAG TTG ATC TTG ATG GGT GTT ATC AAC ACC CCA TTG CAA TTT GTC TCT 335
Lys Leu Ile Leu Met Gly Val Ile Asn Thr Pro Leu Gln Phe Val Ser
                100                 105                 110

CCA TTG GTC ATG CTT GGG GAG GAA GAC GAT CAC CGG GAG CTT TGT GGG 383
Pro Leu Val Met Leu Gly Glu Glu Asp Asp His Arg Glu Leu Cys Gly
            115                 120                 125

GAG CAT GAA GGA GAT GGA GGA GAT GCT CGA GTT CTG CAA AGA GAA AGA 431
Glu His Glu Gly Asp Gly Gly Asp Ala Arg Val Leu Gln Arg Glu Arg
        130                 135                 140

GCT GAA ACG ATG ATT GAA GTG GTG AAG ATG GAC TAC ATC AAC GAA GCT 479
Ala Glu Thr Met Ile Glu Val Val Lys Met Asp Tyr Ile Asn Glu Ala
    145                 150                 155

TTC GAA AGG TTG GAG AAG AAC GAC GTT AGG TAC AGG TTC GTT GTG GAT 527
Phe Glu Arg Leu Glu Lys Asn Asp Val Arg Tyr Arg Phe Val Val Asp
160                 165                 170                 175

TGT TGC CGG CAG CAA TCT TGATCAATAA GAAAGAAAGA AGGCATCATC 575
Cys Cys Arg Gln Gln Ser
                180

GAGTGTTGTC CTATTTTTAT CGAGTACTCT GTCTCATCTT ATCTTAAACA ATATAAATAA 635

ACAAAGAAAA AAAAAAAAAA AAAAAAAA 663

(2) INFORMATION FOR SEQ ID NO:22:

(i) SEQUENCE CHARACTERISTICS:
(A) LENGTH: 181 amino acids
(B) TYPE: amino acid

(D) TOPOLOGY: linear

(ii) MOLECULE TYPE: protein
(xi) SEQUENCE DESCRIPTION: SEQ ID NO:22:

Phe Gly Leu Asp Val Gly Gly Leu Arg Gly Gly Ile Leu Gly Leu Gly
1               5                   10                  15

Gly Val Gly His Met Gly Val Lys Ile Ala Lys Ala Met Gly His His
            20                  25                  30

Ile Thr Val Ile Ser Ser Ser Asp Lys Lys Lys Lys Glu Ala Leu Glu
        35                  40                  45

His Ile Gly Ala Asp Glu Tyr Leu Val Ser Ser Asp Ala Thr Gln Met
    50                  55                  60

Gln Glu Ala Met Asp Ser Leu Asp Tyr Ile Ile Asp Thr Ile Pro Val
65                  70                  75                  80

Phe His Pro Leu Glu Pro Tyr Leu Ser Leu Leu Lys Leu Asp Gly Lys
                85                  90                  95

Leu Ile Leu Met Gly Val Ile Asn Thr Pro Leu Gln Phe Val Ser Pro
            100                 105                 110

Leu Val Met Leu Gly Glu Glu Asp Asp His Arg Glu Leu Cys Gly Glu
        115                 120                 125

His Glu Gly Asp Gly Gly Asp Ala Arg Val Leu Gln Arg Glu Arg Ala
    130                 135                 140

Glu Thr Met Ile Glu Val Val Lys Met Asp Tyr Ile Asn Glu Ala Phe
145                 150                 155                 160

Glu Arg Leu Glu Lys Asn Asp Val Arg Tyr Arg Phe Val Val Asp Cys
                165                 170                 175

Cys Arg Gln Gln Ser
            180

(2) INFORMATION FOR SEQ ID NO:23:

(i) SEQUENCE CHARACTERISTICS:
(A) LENGTH: 694 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "partial cDNA"

(ix) FEATURE:
(A) NAME/KEY: CDS
(B) LOCATION:1..528
(D) OTHER INFORMATION:/product= "Strawberry alcohol
dehydrogenase"

(xi) SEQUENCE DESCRIPTION: SEQ ID NO:23:

GTG CAT TGC TAT GCC TAT GAA GGC AAG ATG CAA GAA CAT CTG CAA TTA          48
Val His Cys Tyr Ala Tyr Glu Gly Lys Met Gln Glu His Leu Gln Leu
1               5                   10                  15

TGT GAA GAC GAG TTT AAA AAG ATA ATG AAG ATA AAT TTC ATG TCT GCA     96
Cys Glu Asp Glu Phe Lys Lys Ile Met Lys Ile Asn Phe Met Ser Ala
            20                  25                  30

TGG TTT CTG GTA AAT GCC GTT GGC AGA AGA ATG CGA GAT CAT AAA TCA     144
Trp Phe Leu Val Asn Ala Val Gly Arg Arg Met Arg Asp His Lys Ser
        35                  40                  45

GGA GGT TCC ATC ATA TTG TTG ACC TCG ATT GTT GGA GCT GAA AGA GGG     192
Gly Gly Ser Ile Ile Leu Leu Thr Ser Ile Val Gly Ala Glu Arg Gly
    50                  55                  60

CTT TAT ACA GGA GCT GTT GCC TAT GGT GCA TGT TCG GCA GCA CTG CAG     240
Leu Tyr Thr Gly Ala Val Ala Tyr Gly Ala Cys Ser Ala Ala Leu Gln
65                  70                  75                  80

CAG TTA GTA AGG TCG TCG GCA TTG GAG ATT GGA AAA TAC CAG ATC AGG     288
Gln Leu Val Arg Ser Ser Ala Leu Glu Ile Gly Lys Tyr Gln Ile Arg
                85                  90                  95

GTT AAT GCA ATC GCA CGT GGT TTG CAT TTG GAA GAT GAG TTT CCT AAG     336
Val Asn Ala Ile Ala Arg Gly Leu His Leu Glu Asp Glu Phe Pro Lys
            100                 105                 110

TCT GTG GGA ATA GAG AGA GCA AAG AAG CTG GTG AAT GAT GCA GTT CCG     384
Ser Val Gly Ile Glu Arg Ala Lys Lys Leu Val Asn Asp Ala Val Pro
        115                 120                 125

CTG GAG AGA TGG CTT GAT GTT AAA AAT GAT GTG GCT TCA AGT GTC ATA     432
Leu Glu Arg Trp Leu Asp Val Lys Asn Asp Val Ala Ser Ser Val Ile
    130                 135                 140

TAT TTG GTC AGT GAT GGT TCA AGG TAC ATG ACG GGC ACA ACT ATA TTT     480
Tyr Leu Val Ser Asp Gly Ser Arg Tyr Met Thr Gly Thr Thr Ile Phe
145                 150                 155                 160

GTT GAT GGG GCA CAG TCT CTC GTG AGG CCT CGA ATG CGT TCT TAT ATG     528
Val Asp Gly Ala Gln Ser Leu Val Arg Pro Arg Met Arg Ser Tyr Met
                165                 170                 175

TGATTCTTGC TCCTATTATA TCCTCCTAGC CATTATTAGC TACTTAGGTT TGTTCATACT     588

TCATAGGTGA ACTCATTAGC TATTCTTACA TTTGTTCCTT ATGAATAAAG AAGTCAAGAT     648

TCAAAAAAAA AAAAAAAAAA AAAAAAAAAA AAAAAAAAAA AAAAAA     694

(2) INFORMATION FOR SEQ ID NO:24:

(i) SEQUENCE CHARACTERISTICS:
(A) LENGTH: 176 amino acids
(B) TYPE: amino acid
(D) TOPOLOGY: linear

(ii) MOLECULE TYPE: protein
(xi) SEQUENCE DESCRIPTION: SEQ ID NO:24:

Val His Cys Tyr Ala Tyr Glu Gly Lys Met Gln Glu His Leu Gln Leu
1               5                   10                  15

Cys Glu Asp Glu Phe Lys Lys Ile Met Lys Ile Asn Phe Met Ser Ala
            20                  25                  30

Trp Phe Leu Val Asn Ala Val Gly Arg Arg Met Arg Asp His Lys Ser
        35                  40                  45

Gly Gly Ser Ile Ile Leu Leu Thr Ser Ile Val Gly Ala Glu Arg Gly
    50                  55                  60

Leu Tyr Thr Gly Ala Val Ala Tyr Gly Ala Cys Ser Ala Ala Leu Gln
65                  70                  75                  80

Gln Leu Val Arg Ser Ser Ala Leu Glu Ile Gly Lys Tyr Gln Ile Arg
                85                  90                  95

Val Asn Ala Ile Ala Arg Gly Leu His Leu Glu Asp Glu Phe Pro Lys
            100                 105                 110

Ser Val Gly Ile Glu Arg Ala Lys Lys Leu Val Asn Asp Ala Val Pro
        115                 120                 125

Leu Glu Arg Trp Leu Asp Val Lys Asn Asp Val Ala Ser Ser Val Ile
    130                 135                 140

Tyr Leu Val Ser Asp Gly Ser Arg Tyr Met Thr Gly Thr Thr Ile Phe
145                 150                 155                 160

Val Asp Gly Ala Gln Ser Leu Val Arg Pro Arg Met Arg Ser Tyr Met
                165                 170                 175

(2) INFORMATION FOR SEQ ID NO:25:

(i) SEQUENCE CHARACTERISTICS:
(A) LENGTH: 1010 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "partial cDNA"

(ix) FEATURE:
(A) NAME/KEY: CDS
(B) LOCATION:2..850
(D) OTHER INFORMATION:/product= "Strawberry alcohol
dehydrogenase"

(xi) SEQUENCE DESCRIPTION: SEQ ID NO:25:

G GAA ACT ACC ATC AAT TTT GGG TCT AAG AAG ATT GCA GTT GTT ACT      46
  Glu Thr Thr Ile Asn Phe Gly Ser Lys Lys Ile Ala Val Val Thr
    1               5                  10                  15

GGA GCC AAC AAA GGG ATT GGA CTT GAG ATT AGC AAG CAA TTA GCT GCT      94
Gly Ala Asn Lys Gly Ile Gly Leu Glu Ile Ser Lys Gln Leu Ala Ala
                20                  25                  30

AAA GGA GTT GGG GTG GTA TTA ACA GCA AGA GAT GTG AAG AGA GGA ACA     142
Lys Gly Val Gly Val Val Leu Thr Ala Arg Asp Val Lys Arg Gly Thr
            35                  40                  45

GAA GCT GCT GAA AAT CTT AAG GCT TCT GGG TTC TCT GAT GTG GTA TTT     190
Glu Ala Ala Glu Asn Leu Lys Ala Ser Gly Phe Ser Asp Val Val Phe
        50                  55                  60

CAT CAG CTA GAT GTA ACA GAG CCG ACT ACT ATT GGT TCT TTG GCA AAC     238
His Gln Leu Asp Val Thr Glu Pro Thr Thr Ile Gly Ser Leu Ala Asn
    65                  70                  75

TTT CTT GAA ACG CAA TTT GGA AAG CTT GAC ATA TTG GTT AAC AAT GCA 286
Phe Leu Glu Thr Gln Phe Gly Lys Leu Asp Ile Leu Val Asn Asn Ala
80                  85                  90                  95

GGA GTC GTT GGA TCT GTA TAC CTC ACA GCC GAC TAT GAT CCA GTG CAA 334
Gly Val Val Gly Ser Val Tyr Leu Thr Ala Asp Tyr Asp Pro Val Gln
                100                 105                 110

ACA TAC GAG ACA GCG AGG GAT TGT TTG AAA ACA AAC TAT TAT GGG CTC 382
Thr Tyr Glu Thr Ala Arg Asp Cys Leu Lys Thr Asn Tyr Tyr Gly Leu
            115                 120                 125

AAG CAA GTC ACA GAA GCA CTT GTT CCG CTG CTT CAA AAA TCT GAA GCT 430
Lys Gln Val Thr Glu Ala Leu Val Pro Leu Leu Gln Lys Ser Glu Ala
        130                 135                 140

GCA AGG ATA GTC AAT GTC TCT TCC GGA TTA GGA CAG CTA AGA AAT ATT 478
Ala Arg Ile Val Asn Val Ser Ser Gly Leu Gly Gln Leu Arg Asn Ile
    145                 150                 155

GGA AAT GAG AAG GCC AAG AAG GAG CTA GGA GAT GCA GAT AAC CTC AAC 526
Gly Asn Glu Lys Ala Lys Lys Glu Leu Gly Asp Ala Asp Asn Leu Asn
160                 165                 170                 175

GAG GAG AAA GTG GAC AAG CTA GTT GAG GAA TTT CTG GAG GAT GTG AAA 574
Glu Glu Lys Val Asp Lys Leu Val Glu Glu Phe Leu Glu Asp Val Lys
                180                 185                 190

CAG GAT TCG ATA GAA TCC AAA GGC TGG CCT CTA AGT ATA TCT GCC TAC 622
Gln Asp Ser Ile Glu Ser Lys Gly Trp Pro Leu Ser Ile Ser Ala Tyr
            195                 200                 205

ATT GTC TCA AAA GCA GCT CTG AAT GCT TAT ACA AGA CTC TTG GCA AAG 670
Ile Val Ser Lys Ala Ala Leu Asn Ala Tyr Thr Arg Leu Leu Ala Lys
        210                 215                 220

AAG TAT CCC CAT ATT GCC ATA AAC GCA GTT GGT CCA GGT TAT ACC AAA 718
Lys Tyr Pro His Ile Ala Ile Asn Ala Val Gly Pro Gly Tyr Thr Lys
    225                 230                 235

ACA GAC CTC AAT AAT AAT TCC GGG ATT CTC ACA GTT GAA GAA GCT GCA 766
Thr Asp Leu Asn Asn Asn Ser Gly Ile Leu Thr Val Glu Glu Ala Ala
240                 245                 250                 255

GTA GGT CCT GTG AGG CTG GCT TTG ATA GCC GAA ACT AGA ATT TCC GGC 814
Val Gly Pro Val Arg Leu Ala Leu Ile Ala Glu Thr Arg Ile Ser Gly
                260                 265                 270

CTC TTC TTC AAC AGA AAT GAA GAG TCG ACC TTT GAT TAGGTCAACG 860
Leu Phe Phe Asn Arg Asn Glu Glu Ser Thr Phe Asp
            275                 280

TGATCCCTGA TGAACTGGAC TATTTTAGAT TTTCAGAATG TGCTTGATTT TGTTGAAGTA 920

TTTATGGGAT TTGTATGTAT ACTTTGATGT ATCATTGTAT TAATAGAGCA CATGTTGTGA 980

TCAAAAAAAA AAAAAAAAAA AAAAAAAAAA 1010

(2) INFORMATION FOR SEQ ID NO:26:

(i) SEQUENCE CHARACTERISTICS:
(A) LENGTH: 283 amino acids
(B) TYPE: amino acid
(D) TOPOLOGY: linear

(ii) MOLECULE TYPE: protein

(xi) SEQUENCE DESCRIPTION: SEQ ID NO:26:

Glu Thr Thr Ile Asn Phe Gly Ser Lys Lys Ile Ala Val Val Thr Gly
1               5                   10                  15

Ala Asn Lys Gly Ile Gly Leu Glu Ile Ser Lys Gln Leu Ala Ala Lys
            20                  25                  30

Gly Val Gly Val Val Leu Thr Ala Arg Asp Val Lys Arg Gly Thr Glu
        35                  40                  45

Ala Ala Glu Asn Leu Lys Ala Ser Gly Phe Ser Asp Val Val Phe His
    50                  55                  60

Gln Leu Asp Val Thr Glu Pro Thr Thr Ile Gly Ser Leu Ala Asn Phe
65                  70                  75                  80

Leu Glu Thr Gln Phe Gly Lys Leu Asp Ile Leu Val Asn Asn Ala Gly
                85                  90                  95

Val Val Gly Ser Val Tyr Leu Thr Ala Asp Tyr Asp Pro Val Gln Thr
            100                 105                 110

Tyr Glu Thr Ala Arg Asp Cys Leu Lys Thr Asn Tyr Tyr Gly Leu Lys
        115                 120                 125

Gln Val Thr Glu Ala Leu Val Pro Leu Leu Gln Lys Ser Glu Ala Ala
    130                 135                 140

Arg Ile Val Asn Val Ser Ser Gly Leu Gly Gln Leu Arg Asn Ile Gly
145                 150                 155                 160

Asn Glu Lys Ala Lys Lys Glu Leu Gly Asp Ala Asp Asn Leu Asn Glu
                165                 170                 175

Glu Lys Val Asp Lys Leu Val Glu Glu Phe Leu Glu Asp Val Lys Gln
            180                 185                 190

Asp Ser Ile Glu Ser Lys Gly Trp Pro Leu Ser Ile Ser Ala Tyr Ile
        195                 200                 205

Val Ser Lys Ala Ala Leu Asn Ala Tyr Thr Arg Leu Leu Ala Lys Lys
    210                 215                 220

Tyr Pro His Ile Ala Ile Asn Ala Val Gly Pro Gly Tyr Thr Lys Thr
225                 230                 235                 240

Asp Leu Asn Asn Asn Ser Gly Ile Leu Thr Val Glu Glu Ala Ala Val
                245                 250                 255

Gly Pro Val Arg Leu Ala Leu Ile Ala Glu Thr Arg Ile Ser Gly Leu
            260                 265                 270

Phe Phe Asn Arg Asn Glu Glu Ser Thr Phe Asp
        275                 280

(2) INFORMATION FOR SEQ ID NO:27:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: peptide

(iii) HYPOTHETICAL: NO

(xi) SEQUENCE DESCRIPTION: SEQ ID NO:27:

Trp Thr Asn Phe Phe Asn Pro Leu Asp Phe Gly Trp
1               5                   10